# EUROPEAN PATENT SPECIFICATION

(11) **EP 2 714 903 B1**
(45) Date of publication and mention of the grant of the patent: **21.03.2018**
(21) Application number: 12792440.5
(22) Date of filing: 31.05.2012
(51) Int. Cl.: C12Q 1/68, C12N 15/09, A61K 31/713, A61K 48/00, A61P 35/00, C12N 15/113, C12Q 1/02, G01N 33/15, G01N 33/50, G01N 33/574

(54) **SUV39H2 AS A TARGET GENE FOR CANCER THERAPY**
SUV39H2 ALS ZIELGEN FÜR KREBSTHERAPIE
SUV39H2 À TITRE DE GÈNE CIBLE EN THÉRAPIE ANTICANCÉREUSE

(30) Priority: 03.06.2011 US 201161493235 P
(43) Date of publication of application: 09.04.2014
(73) Proprietor: OncoTherapy Science, Inc., Kawasaki-shi Kanagawa 213-0012 (JP)
(72) Inventor: HAMAMOTO, Ryuji, Tokyo 108-8639 (JP); NAKAMURA, Yusuke, Tokyo 108-8639 (JP); TSUNODA, Takuya, Kawasaki-shi Kanagawa 213-0012 (JP)
(74) Representative: Dennemeyer & Associates S.A.
(86) International application number: PCT/JP2012/003569
(87) International publication number: WO 2012/164936

(56) References cited:
- WO-A1-01/94620
- WO-A1-2005/116204
- WO-A1-2009/126537
- WO-A1-2009/126970
- WO-A1-2010/007093
- WO-A1-2010/007093
- WO-A2-2007/013671
- JP-A- 2003 277 288
- JP-A- 2006 162 446
- US-A1- 2002 039 776
- US-A1- 2003 157 532
- US-A1- 2008 234 138
- OZDAG, H. ET AL.: 'Differential expression of selected histone modifier genes in human solid cancers' BMC GENOMICS vol. 7, no. 90, 2006, XP021014692

## Description

### Technical Field

The present invention relates to methods of detecting and diagnosing a predisposition to developing cancer, particularly lung cancer, cervical cancer, bladder cancer, esophageal cancer, osteosarcoma, prostate cancer and soft tissue tumor. The present invention also relates to methods of screening for a candidate substance for either or both of treating and preventing a cancer linked to the over-expression of the SUV39H2 gene, examples of which include lung cancer, cervical cancer, bladder cancer, esophageal cancer, osteosarcoma, prostate cancer and soft tissue tumor. Moreover, the present invention relates to double-stranded molecules that inhibit or reduce expression of the SUV39H2 gene and therapeutic uses thereof.

### Background Art

The nucleosome, the basic unit of DNA packaging in eukaryotes that consists of a 147-bp DNA wound in sequence around a histone protein core, is a fundamental unit of chromatin structures [NPL1]. All four core histones (H3, H4, H2A and H2B) possess unstructured N-terminal tails and these N-termini of histones are particularly subjected to a diverse array of post-translational modifications: acetylation, methylation, phosphorylation, ubiquitination, SUMOylation and ADP-ribosylation [NPL2]. These histone modifications cause dynamic changes to the chromatin structure and thereby impinge on transcriptional regulation, DNA replication, DNA repair, and alternative splicing [NPL3,4]. Among these epigenetic marks on histones, the methylation process is particularly crucial for transcriptional regulation [NPL5]. Five lysine residues (H3K4, H3K9, H3K27, H3K36 and H4K20) are located in the N-terminal tails and are representative lysines that can become mono-, di-, or trimethylated. Whereas H3K9, H3K27 and H4K20 methylation mainly represses transcription, methylation marks on H3K4 and H3K36 are associated with the induction of active transcription [NPL6] .For instance, methylation of histone H3 at lysine 9 (H3K9) is one of the most abundant and stable histone modifications, and is involved in both gene repression and heterochromatin formation. H3K9 can be mono-, di- or trimethylated on H3K9, whereas silent euchromatin regions are enriched for mono-and dimethylated H3K9 [NPL17]. In mammals, heterochromatic regions are highly trimethylated on H3K9, whereas silent euchromatic regions are enriched for mono- and dimethylated H3K9 [NPL17]. H3K9 methylation has been linked to de novo gene silencing and DNA methylation, and it is inherited after mitosis in a manner coupled to DNA methylation.

It has previously been reported that some histone methyltransferases and demethylases are deeply involved in human carcinogenesis [NPL7,8,9,10,11]. For instance, SMYD3, PRMT1, PRMT6, SUV420H1 and SUV420H2 have been shown to stimulate the proliferation of cells through its enzymatic activity [PTL1, NPL8,9,12,13,14,18].

SUV39H2, also known as KMT1B [NPL15], is a SET-domain containing histone methyltransferase and is known to methylate the H3K9 lysine residue. Suv39h2, the murine homologue of human SUV39H2, has been isolated and characterized as the second murine Suv39h gene, and demonstrated to share 59% identity with Suv39h1 [NPL16]. The expression of Suv39h2 is restricted to adult testis, and immunolocalization of endogenous Suv39h2 protein reveals enriched distributions at heterochromatin during the first meiotic prophase and in the early stages of sperminogenesis. During mid-pachytene, Suv39h2 specifically accumulates within the chromatin of the silenced sex chromosomes present in the XY body. In addition, the histone methyltransferase activity of Suv39h2 appears to play an important role in regulating higher-order chromatin dynamics during male meiosis [NPL16].

However, to date, there has been no suggestion or demonstration of the significance of SUV39H2 deregulation in human carcinogenesis.

### Citation List

### Patent Literature

[PTL 1] WO2005/071102

### Non-Patent Literature

[NPL 1] Strahl BD et al. Nature 2000; 403: 41-45
[NPL 2] Kouzarides T et al. Cell 2007; 128: 693-705
[NPL 3] Huertas D et al. Epigenetics 2009; 4: 31-42
[NPL 4] Luco RF et al. Science 2010; 327: 996-1000
[NPL 5] Kouzarides T et al. Curr Opin Genet Dev 2002; 12: 198-209
[NPL 6] Peterson CL et al. Curr Biol 2004; 14: R546-551
[NPL 7] Cho HS et al. Cancer Res 2010
[NPL 8] Hamamoto R et al. Nat Cell Biol 2004;6:731-40
[NPL 9] Hamamoto R et al. Cancer Sci 2006;97:113-8
[NPL 10] Yoshimatsu M et al. Int J Cancer 2011; 128: 562-573
[NPL 11] Hayami S et al. Int J Cancer 2011; 128: 574-586
[NPL 12] Kunizaki M et al. Cancer Res 2007;67:10759-65
[NPL 13] Silva FP et al. Oncogene 2008;27:2686-92
[NPL 14] Tsuge M et al. Nat Genet 2005;37:1104-7
[NPL 15] Allis CD et al. Cell 2007; 131: 633-636
[NPL 16] O'Carroll D et al. Mol Cell Biol 2000; 20: 9423-9433
[NPL 17] Martin C et al. Nat Rev Mol Cell Biol 2005;6:838-49
[NPL 18] Schneider R et al. Trends Biochem Sci 2002;27:396-402.

### Summary of Invention

The present invention is defined by the appended claim. Also disclosed therein are novel compositions and methods for detecting, diagnosing, treating and/or preventing cancer, particularly lung cancer, cervical cancer, bladder cancer, esophageal cancer, osteosarcoma, prostate cancer and soft tissue tumor.

Central to the present invention is the discovery that the SUV39H2 gene is over-expressed in cancer cells and the knock-down of SUV39H2 by double-stranded molecules against the SUV39H2 gene lead to significant suppression of cancer cell growth. Such double-stranded molecules can be composed of a sense strand and an antisense strand, wherein the sense strand includes a nucleotide sequence corresponding to a target sequence selected from the group consisting of SEQ ID NOs: 19 and 20 and the antisense strand includes a sequence that is complementary to the sense strand, further wherein the sense and the antisense strands of the molecule hybridize to each other to form a double-stranded molecule.

Therefore, it is an object of the present description is to provide isolated double-stranded molecules, when introduced into a cell, inhibits the expression of an SUV39H2 gene as well as cell proliferation, the molecule including a sense strand and an antisense strand complementary thereto, the strands hybridized to each other to form the double-stranded molecule. These double-stranded molecules may be utilized in an isolated state or encoded in vectors and expressed from the vectors. Accordingly, also disclosed therein are such double-stranded molecules as well as vectors and host cells expressing them.

Also disclosed therein are methods for inhibiting the growth of SUV39H2-expressing cells and treating an SUV39H2-associated disease such as cancer, examples of which include lung cancer, cervical cancer, bladder cancer, esophageal cancer, osteosarcoma, prostate cancer and soft tissue tumor, by administering the double-stranded molecules or vectors of the present invention to a subject in need thereof. Such methods encompass administering to a subject a composition containing one or more of the double-stranded molecules or vectors of the present invention.

Also disclosed therein are compositions for either or both of the treatment and prevention of an SUV39H2-associated disease such as cancer, examples of which include lung cancer, cervical cancer, bladder cancer, esophageal cancer, osteosarcoma, prostate cancer and soft tissue tumor, such compositions including at least one of the double-stranded molecules or vectors of the present invention.

Also disclosed therein is a method of detecting or diagnosing cancer in a subject using as an index the expression level of the SUV39H2 gene. More particularly, an increase in the expression level of the test gene (i.e., SUV39H2) in a subject-derived biological sample as compared to a normal control level of the test gene indicates the presence of cancer in the subject or that the subject suffers from cancer, examples of which include lung cancer, cervical cancer, bladder cancer, esophageal cancer, osteosarcoma, prostate cancer and soft tissue tumor.

Also disclosed therein is method of screening for a candidate substance useful for either or both of prevention and treatment of an SUV39H2-associated disease such as cancer, examples of which include lung cancer, cervical cancer, bladder cancer, esophageal cancer, osteosarcoma, prostate cancer and soft tissue tumor. A useful candidate substance can be selected using as an index the binding activity to the SUV39H2 polypeptide, the inhibitory activity against a biological activity of the SUV39H2 polypeptide or the suppressing activity against the expression level of the SUV39H2 gene. The biological activities of the SUV39H2 polypeptide that are of particular interest to the screening method of the present invention include cell-proliferation promoting activity and methyltransferase activity (e.g., histone methyltransferase activity).

Also disclosed therein is a kit for detecting or diagnosing cancer that comprises a reagent for detecting an SUV39H2 mRNA, an SUV39H2 protein or a biological activity of an SUV39H2 protein.

A series of specific objectives is set forth below. It will be understood by those skilled in the art that one or more aspects of this invention can meet certain objectives, while one or more other aspects can meet certain other objectives. Each objective may not apply equally, in all its respects, to every aspect of this invention. As such, the following objects can be viewed in the alternative with respect to any one aspect of this invention.

More specifically, the present description provides the following [1] to [29]:
[1]A method of detecting or diagnosing cancer in a subject or determining a predisposition for developing cancer, said method comprising determining an expression level of an SUV39H2 gene in a subject-derived biological sample, wherein an increase of said level compared to a normal control level of said gene indicates that said subject suffers from or is at risk of developing cancer, wherein the expression level is determined by a method selected from the group consisting of:
   (a) detecting the mRNA of the SUV39H2 gene;
   (b) detecting the protein encoded by the SUV39H2 gene; and
   (c) detecting the biological activity of the protein encoded by the SUV39H2 gene;
[2]The method of [1], wherein said increase is at least 10% greater than said normal control level;
[3]The method of [1], wherein the subject-derived biological sample comprises a biopsy specimen, saliva, sputum, blood, serum, plasma, pleural effusion or urine;
[4]A kit for detecting or diagnosing cancer or determining a predisposition for developing cancer, which comprises a reagent selected from the group consisting of:
   (a) a reagent for detecting the mRNA of the SUV39H2 gene;
   (b) a reagent for detecting the protein encoded by the SUV39H2 gene; and
   (c) a reagent for detecting the biological activity of the protein encoded by the SUV39H2 gene;
[5]The kit of [4], wherein the reagent is a probe to a transcript of the SUV39H2 gene;
[6]The kit of [4], wherein the reagent is an antibody against the protein encoded by the SUV39H2 gene;
[7]The method of any one of [1] to [3], or the kit of any one of [4] to [6], wherein the biological activity to be detected is cell-proliferation promoting activity or methyltransferase activity;
[8]The method of any one of [1] to [3], or the kit of any one of [4] to [6], wherein the cancer is selected from the group consisting of lung cancer, cervical cancer, bladder cancer, esophageal cancer, osteosarcoma, prostate cancer and soft tissue tumor;
[9]A method of screening for a candidate substance for either or both of the treatment and prevention of cancer or the inhibition of cancer cell growth, said method comprising the steps of:
   (a) contacting a test substance with an SUV39H2 polypeptide or functional equivalent thereof;
   (b) detecting the binding activity between the polypeptide or functional equivalent thereof and the test substance; and
   (c) selecting the test substance that binds to the polypeptide or functional equivalent thereof;
[10]A method of screening for a candidate substance for either or both of the treatment and prevention of cancer or the inhibition of cancer cell growth, said method comprising the steps of:
   (a) contacting a test substance with a cell expressing the SUV39H2 gene; and
   (b) selecting the test substance that reduces the expression level of the SUV39H2 gene in comparison with the expression level in the absence of the test substance;
[11]A method of screening for a candidate substance for either or both of the treatment and prevention of cancer or the inhibition of cancer cell growth, said method comprising the steps of:
   (a) contacting a test substance with an SUV39H2 polypeptide or functional equivalent thereof;
   (b) detecting a biological activity of the polypeptide or functional equivalent thereof of step (a); and
   (c) selecting the test substance that suppresses the biological activity of the polypeptide or functional equivalent thereof in comparison with the biological activity detected in the absence of the test substance;
[12]The method of [11], wherein the biological activity is cell proliferation promoting activity or methyltransferase activity;
[13]A method of screening for a candidate substance for either or both of the treatment and prevention of cancer or the inhibition of cancer cell growth, said method comprising the steps of:
   a) contacting a test substance with a cell into which a vector comprising the transcriptional regulatory region of the SUV39H2 gene and a reporter gene that is expressed under the control of the transcriptional regulatory region has been introduced,
   b) measuring the expression or activity level of said reporter gene; and
   c) selecting the test substance that reduces the expression or activity level of said reporter gene, as compared to a level in the absence of the test substance;
[14]A method of screening for a candidate substance for either or both of the treatment and prevention of cancer or the inhibition of cancer cell growth, the method including the steps of:
   (a) contacting a test substance with a cell expressing the SUV39H2 gene and a downstream gene selected from the genes shown in Table 7 and Table 8;
   (b) detecting the expression level of the downstream gene; and
   (c) selecting the test substance that alters the expression level of the downstream gene in comparison with the expression level detected in the absence of the test substance;
[15]The method of [14], wherein the downstream gene of the SUV39H2 gene is selected from the genes shown in Table 7 and the step (c) is the step of selecting the test substance that increases the expression level of the downstream gene in comparison with the expression level detected in the absence of the test substance;
[16]The method of [14], wherein the downstream gene of the SUV39H2 gene is selected from the genes shown in Table 8 and the step (c) is the step of selecting the test substance that reduces the expression level of the downstream gene in comparison with the expression level detected in the absence of the test substance;
[17]The method of any one of [9] to [16], the cancer is selected from the group consisting of lung cancer, cervical cancer, bladder cancer, esophageal cancer, osteosarcoma, prostate cancer and soft tissue tumor;
[18]A double-stranded molecule comprising a sense strand and an antisense strand, wherein the sense strand comprises a nucleotide sequence corresponding to a target sequence of SEQ ID NO: 19 or 20, and wherein the antisense strand comprises a nucleotide sequence which is complementary to said target sequence, wherein said sense strand and said antisense strand hybridize to each other to form a double-stranded molecule, and wherein said double-stranded molecule, when introduced into a cell expressing the SUV39H2 gene, inhibits the expression of the SUV39H2 gene;
[19]The double-stranded molecule of [18], wherein the double-stranded molecule is between about 19 and about 25 nucleotides in length;
[20]The double-stranded molecule of [18], wherein said double-stranded molecule is a single polynucleotide molecule comprising the sense strand and the antisense strand linked via a single-stranded nucleotide sequence;
[21]The double-stranded molecule of [20], wherein said double-stranded molecule has the general formula

   5'-[A]-[B]-[A']-3' or 5'-[A']-[B]-[A]-3',

   wherein [A] is a sense strand comprising a nucleotide sequence corresponding to a target sequence of SEQ ID NO: 19 or 20; [B] is a nucleotide sequence consisting of about 3 to about 23 nucleotides; and [A'] is an antisense strand comprising a nucleotide sequence complementary to the target sequence;
[22]A vector encoding the double-stranded molecule of any one of [18] to [21];
[23]A vector comprising a sense strand nucleic acid and an antisense strand nucleic acid, wherein said sense strand nucleic acid comprises a nucleotide sequence of SEQ ID NO: 19 or 20, and said antisense strand nucleic acid is composed of a nucleotide sequence complementary to the sense strand, wherein the transcripts of said sense strand and said antisense strand hybridize to each other to form a double-stranded molecule, and wherein said vector inhibits expression of the SUV39H2 gene;
[24]A method of either or both of the treatment and prevention of cancer in a subject, wherein said method comprises administering to said subject a pharmaceutically effective amount of a double-stranded molecule against an SUV39H2 gene or a vector encoding said double-stranded molecule, wherein the double-stranded molecule inhibits cell proliferation as well as the expression of the SUV39H2 gene when introduced into a cell expressing the SUV39H2 gene;
[25]The method of [24], wherein the double-stranded molecule is that of any one of [18] to [21];
[26]The method of [24], wherein the vector is that of [22] or [23];
[27]A composition for either or both of the treatment and prevention of cancer,
   wherein said composition comprises a pharmaceutically effective amount of a double-stranded molecule against an SUV39H2 gene or a vector encoding said double-stranded molecule, and a pharmaceutically acceptable carrier, wherein the double-stranded molecule inhibits cell proliferation as well as the expression of the SUV39H2 gene when introduced into a cell expressing the SUV39H2 gene;
[28]The composition of [27], wherein the double-stranded molecule is that of any one of [18] to [21]; and
[29]The composition of [27], wherein the vector is that of [22] or [23].

### Brief Description of Drawings

Various aspects and applications of the present invention will become apparent to the skilled artisan upon consideration of the brief description of the figures and the detailed description of the present invention and its preferred embodiments which follows:

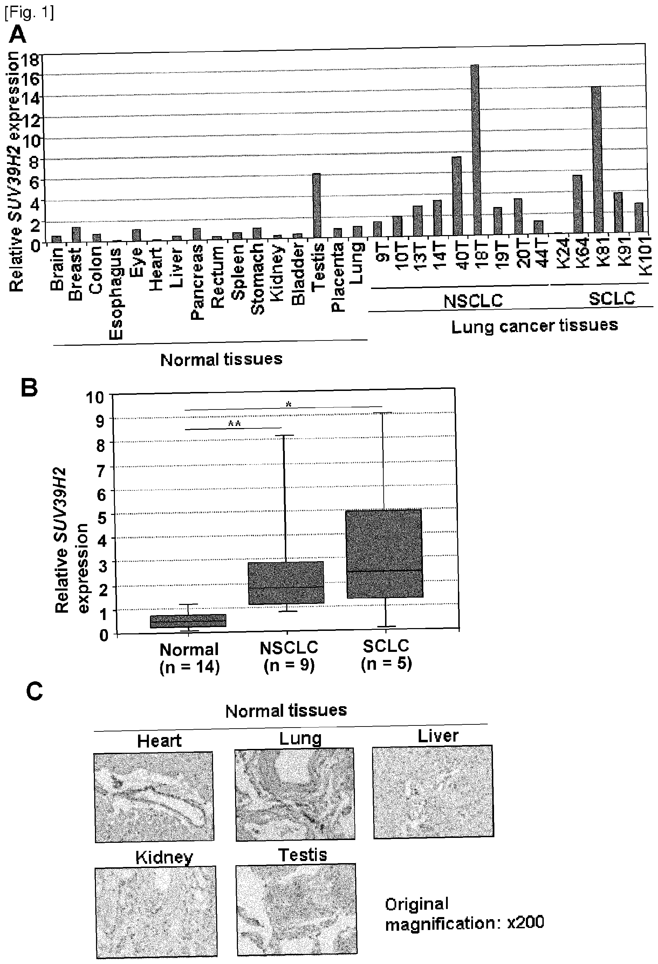

[fig. 1] Figure 1 demonstrates the over-expression of SUV39H2 in clinical lung cancers. Part A depicts the mRNA levels of SUV39H2 in 14 lung cancer cases (NSCLC: 9 cases; SCLC: 5 cases) and 16 normal vital organs. Part B depicts the quantitative real-time-PCR measurements using 14 lung cancer samples and 14 normal tissues, and the result is shown by box-whisker plot. For statistical analysis, Kruskal-Wallis (*P < 0.05) and Student's t-test (**P < 0.05) were performed. Part C depicts the immunohistochemical staining of SUV39H2 in normal adult human tissues. All tissue samples were purchased from BioChain. Original magnification: x200.

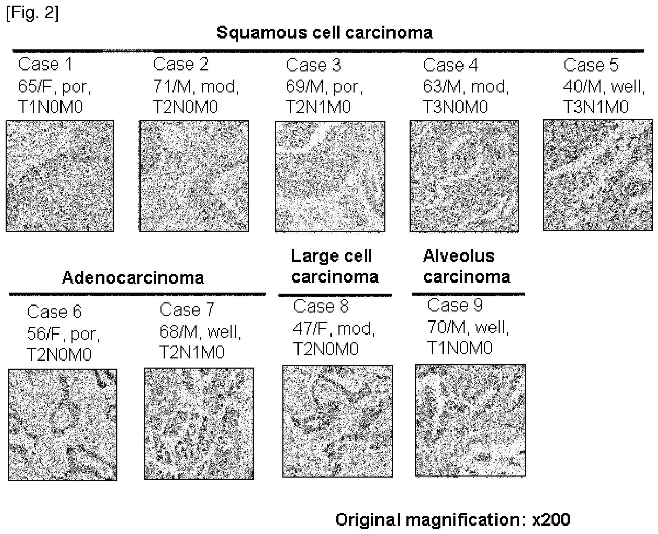

[fig.2]Figure 2 demonstrates the elevated expression of SUV39H2 in lung cancer tissues. Tissue microarray images of lung tumors stained by standard immunohistochemistry for SUV39H2 protein are shown. Immunohistochemistry analysis of SUV39H2 protein expression in lung cancer tissues shows that SUV39H2 is abundantly expressed in the nucleus. Clinical information for each section is represented above histological pictures. All tissue samples were purchased from BioChain. Original magnification: x200.

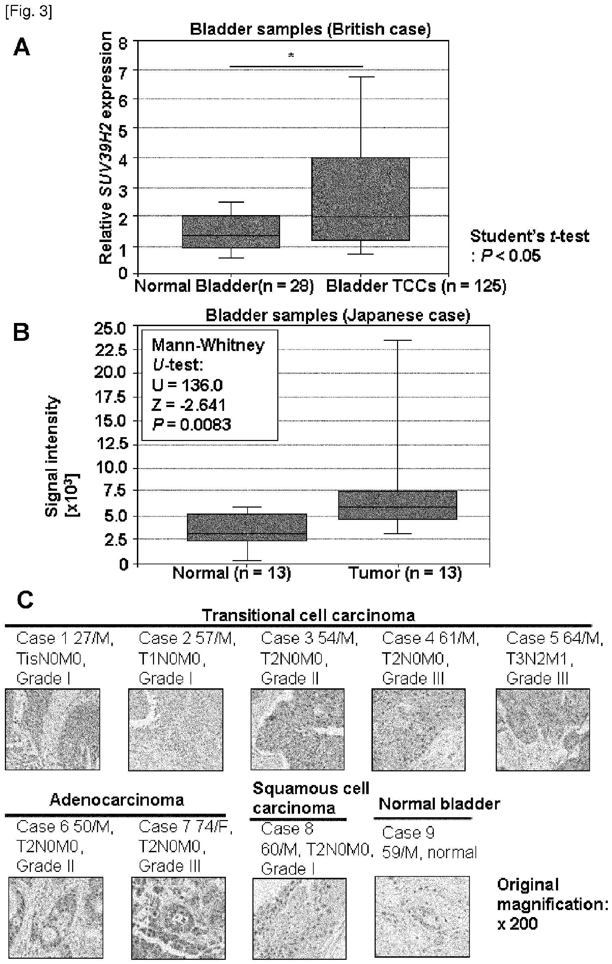

[fig.3]Figure 3 demonstrates the significant up-regulation of SUV39H2 in bladder cancer samples. Part A depicts the quantitative real-time-PCR measurements using 125 bladder cancer tissues and 28 normal bladder samples (British), and the result is shown by box-whisker plot. For statistical analysis, Student's t-test was adopted (*P < 0.05). Part B depicts the comparison of SUV39H2 expression between normal and tumor bladder tissues in Japanese patients. Signal intensity for each sample was analyzed by cDNA microarray, and the result is shown by box-whisker plot (median 50% boxed). Mann-Whitney's U-test was used for the statistical analysis. Part C depicts the immunohistochemistry analysis showing up-regulation of SUV39H2 in bladder cancer and normal bladder tissue purchased from BioChain. Clinical information for each section is represented above histological pictures. All tissue samples were purchased from BioChain. Original magnification: x200.

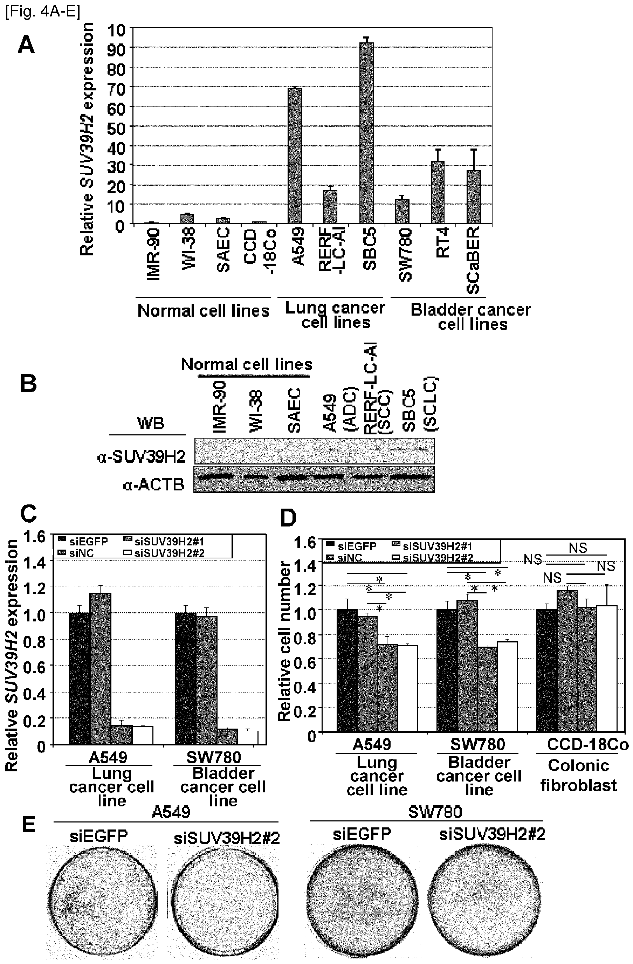

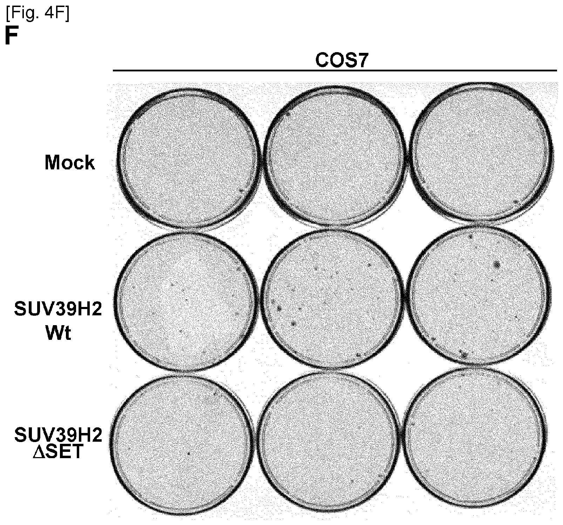

[fig.4A-E]Figure 4 demonstrates the growth suppression effect of SUV39H2 knockdown. Part A depicts the SUV39H2 mRNA expression levels in various cell lines. Quantitative real-time -PCR was performed using normal cell lines (IMR-90, WI-38, SAEC and CCD-18Co), three lung cancer cell lines (A549, RERF and SBC-5) and three bladder cancer cell lines (SW780, RT4 and SCaBER). Part B depicts the validation of SUV39H2 protein expression levels in various cell lines. Lysates from three normal cell lines (IMR-90, WI-38 and SAEC) and three lung cancer cell lines (A549, RERF and SBC5) were immunoblotted with anti-SUV39H2 antibody. ACTB was used as an internal control. Part C depicts the results of quantitative real-time PCR showing suppression of endogenous SUV39H2 expression by 2 independent SUV39H2 specific siRNAs (siSUV39H2#1, #2) in A549 and SW780 cells. siRNA targeting EGFP (siEGFP) and siNegative control (siNC) were used as controls. mRNA expression levels were normalized by GAPDH expressions, and values are relative to siEGFP (siEGFP = 1). Results are the mean +/- SD of 3 independent experiments. Part D depicts the effects of SUV39H2 knockdown on the proliferation of cancer cell lines (A549 and SW780) and a normal cell line (CCD-18Co). The cell numbers were measured by Cell Counting kit 8. Relative cell numbers are normalized to the number of siEGFP-treated cells (siEGFP = 1): results are the mean +/-SD of three independent experiments. P-values were calculated using Student's t-test (*, P < 0.05).. Part E and F depict the results of the colony formation assay to validate the effects of SUV39H2 expression on the growth regulation of cells. Part E depicts loss-of-function experiment using SUV39H2 siRNA. Giemsa staining was performed 9 days (A549) and 3 days (SW780) after treatment with siRNAs.
[fig.4F]Part F depicts the results of a clonogenecity assay of SUV39H2. Methylation activity of SUV39H2 is critical for its growth promoting effect. COS7 cells were transfected with FLAG-Mock, -SUV39H2 (WT or delta-SET) and, 14 days after transfection, stained with Giemsa.

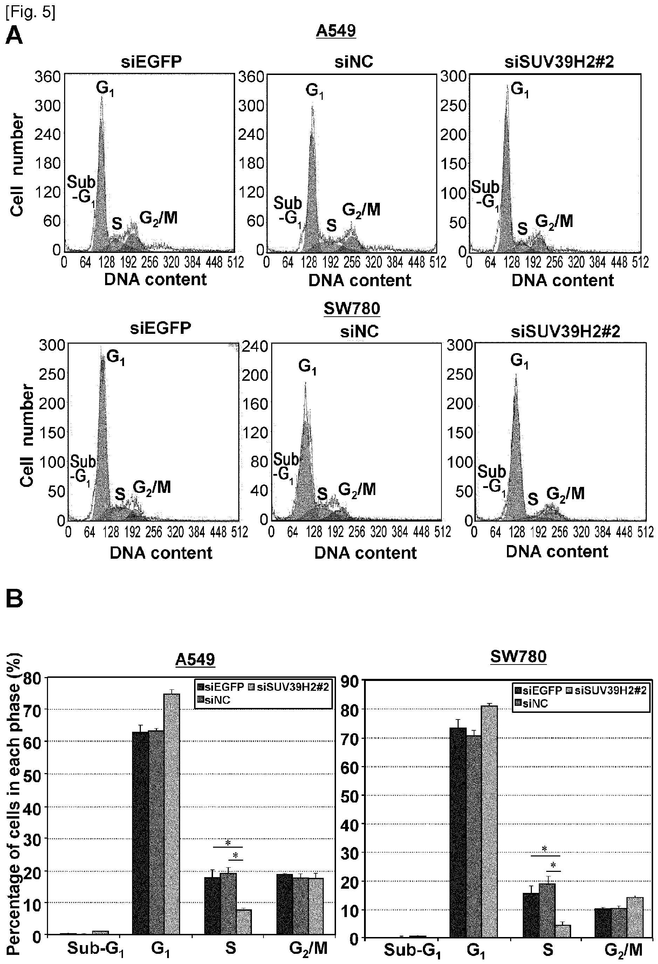

[fig.5]Figure 5 demonstrates the effect of inhibition of SUV39H2 expression on cell cycle distribution. Part A depicts effects of SUV39H2 knockdown on cell cycle kinetics in cancer cells. A549 and SW780 cells were treated with siRNAs and analyzed by FACS 72 hours after siRNA treatment. Representative histograms of this experiment are shown. Numerical analysis of the FACS result, classifying cells by cell cycle status. Mean +/- SD of three independent experiments. P values were calculated using Student's t-test. Part B depicts the histograms indicating the percentage of cells in each phase shown in Part A.

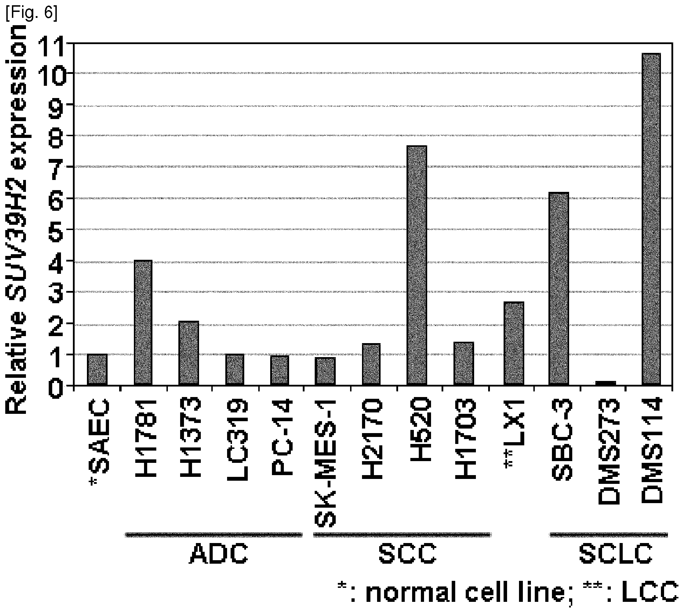

[fig.6]Figure 6 demonstrates the over-expression of SUV39H2 in lung cancer cell lines relative to a normal cell line (human small airway epithelial cells: SAEC). Expression levels of SUV39H2 were analyzed by quantitative real-time PCR. Relative SUV39H2 expression shows the ratio compared to the value in SAEC. ADC: adenocarcinoma; SCC: squamous cell carcinoma; LCC: large cell carcinoma; SCLC: small cell lung cancer.

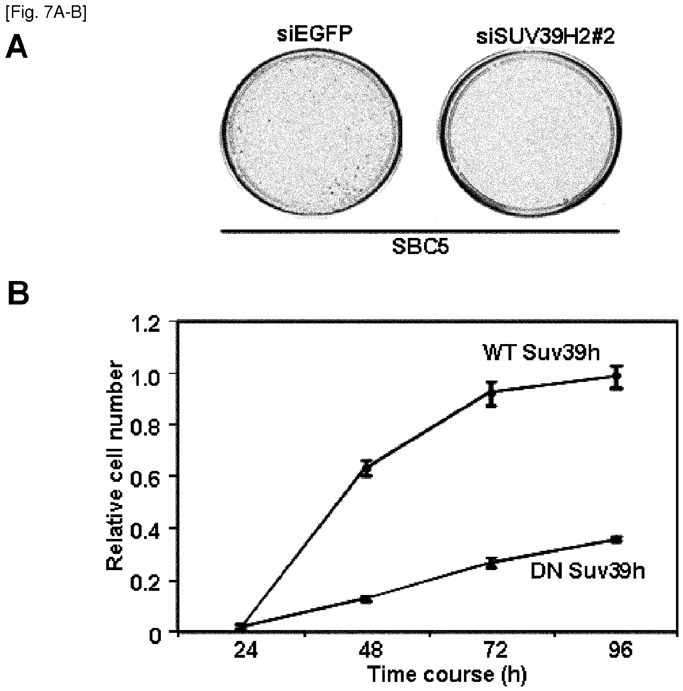

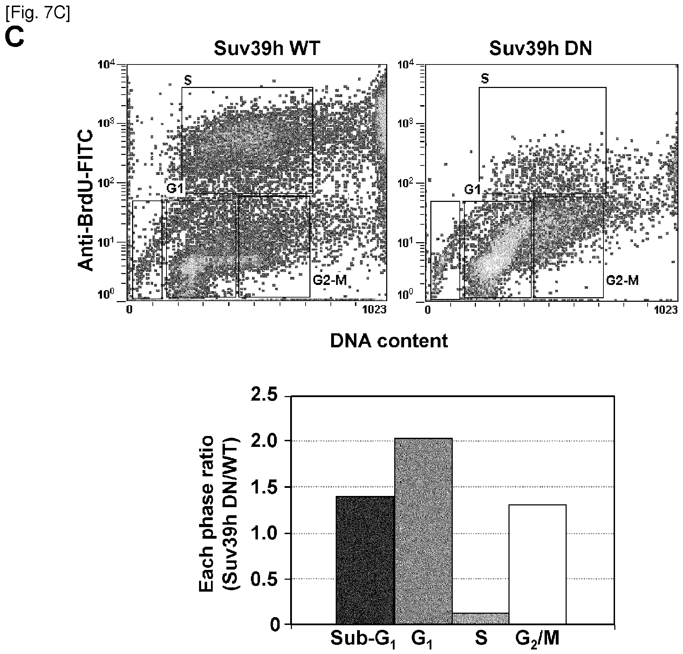

[fig.7A-B]Figure 7 demonstrates that SUV39H2 is crucial for cancer cell proliferation. Part A depicts the results of a colony formation assay of SBC5 cells. Giemsa staining was performed 10 days after siRNA treatment. Part B depicts the delayed cell growth of Suv39hDN MEFs as compared with Suv39hWT. 1 x 10⁵ cells of Suv39hWT and Suv39hDN(SUV39H1 and SUV39H2 double null) MEFs were seeded in 6-well tissue culture plates and cell growth assay was performed following the time course as indicated above.
[fig.7C]Part C depicts the cell cycle distribution of Suv39WT and Suv39DN mouse embryonic fibroblasts (MEFs). Suv39WT and Suv39DN MEFs were seeded in 6-well tissue culture plates for 72 hours and cell cycle distribution was analyzed by flow cytometry after coupled staining with fluorescein isothiocyanate (FITC)-conjugated anti-BrdU and 7-amino-actinomycin D (7-AAD) as described in Materials and methods. A representative histogram of DN-to-WT ratio of the each cell phase was shown.

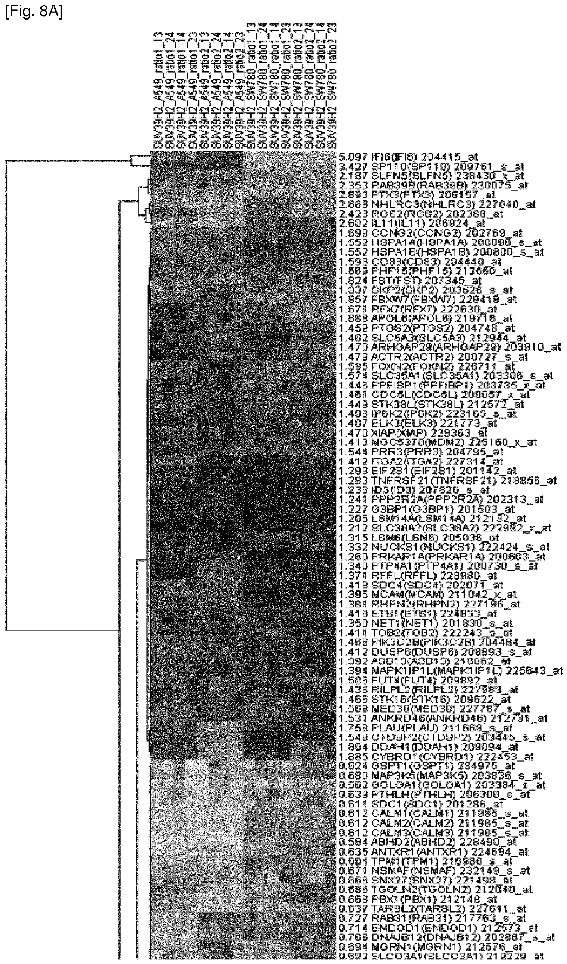

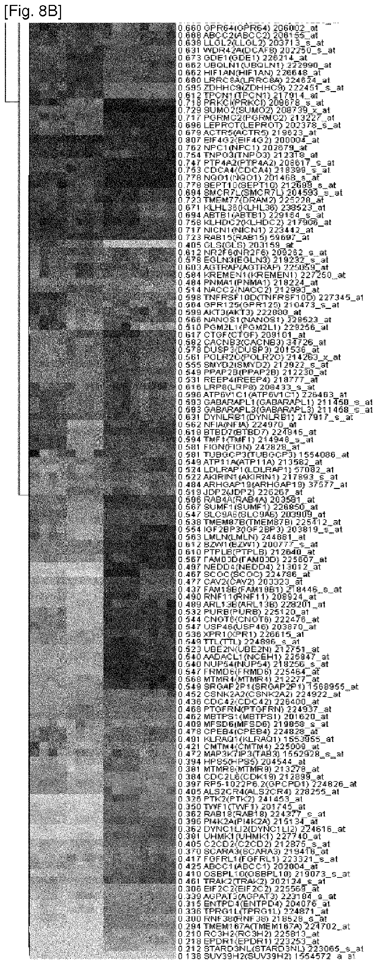

[fig.8A]Figure 8 presents the results of two-dimensional, unsupervised hierarchical cluster analysis of SW780 and A549 mRNA expression profiles after knockdown of SUV39H2 expressions. Differentially expressed genes were selected for this analysis. Up-regulated genes are shown in Table 7; Down-regulated genes are shown in Table 8.
[fig.8B]Figure 8B is a continuation of Figure 8A.

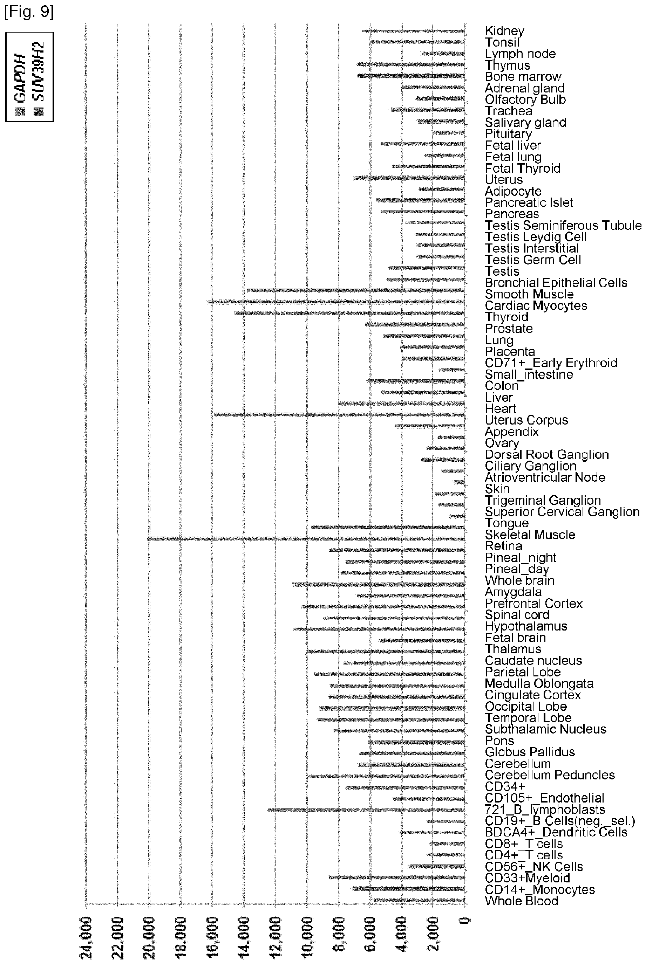

[fig.9]Figure 9 demonstrates the expression levels of SUV39H2 in 78 normal tissues. The data were derived from BioGPS (http://biogps.gnf.org/#goto=genereport&id=79723). GAPDH expression is shown as a control of the signal intensity. The facts that the bars corresponding to SUV39H2 can be hardly seen, confirm that the expression level of SUV39H2 as compared to GAPDH in those normal tissues is significantly low.

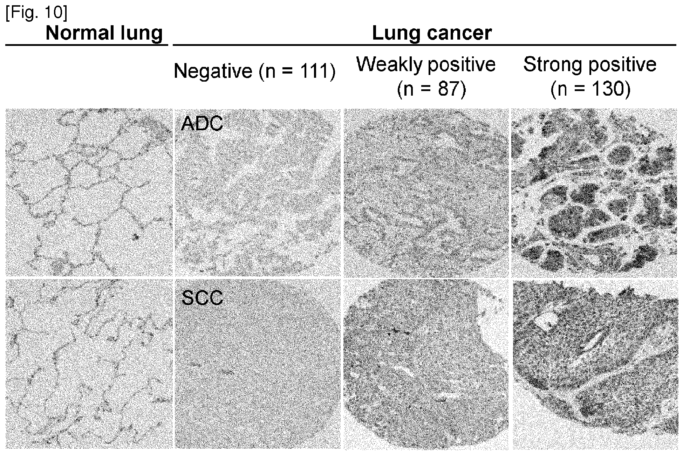

[fig. 10] Figure 10 demonstrates the representative cases for positive SUV39H2 expression in lung ADC, SCC tissues and normal lung tissues. Original magnification, x100.

### Description of Embodiments

Unless otherwise defined, all technical and scientific terms used herein have the same meaning as commonly understood by one of ordinary skill in the art to which the present invention belongs. Although methods and materials similar or equivalent to those described herein can be used in the practice or testing of the present invention, suitable methods and materials are described below. In addition, the materials, methods, and examples are illustrative only and not intended to be limiting.

### Definitions:

The words "a", "an", and "the" as used herein mean "at least one" unless otherwise specifically indicated.

The terms "polypeptide", "peptide", and "protein" are used interchangeably herein to refer to a polymer of amino acid residues. The terms apply to amino acid polymers in which one or more amino acid residue is a modified residue, or a non-naturally occurring residue, such as an artificial chemical mimetic of a corresponding naturally occurring amino acid, as well as to naturally occurring amino acid polymers.

The term "amino acid" refers to naturally occurring and synthetic amino acids, as well as amino acid analogs and amino acid mimetics that similarly functions to the naturally occurring amino acids. Naturally occurring amino acids are those encoded by the genetic code, as well as those modified after translation in cells (e.g., hydroxyproline, gamma-carboxyglutamate, and O-phosphoserine). The phrase "amino acid analog" refers to compounds that have the same basic chemical structure (an alpha carbon bound to a hydrogen, a carboxy group, an amino group, and an R group) as a naturally occurring amino acid but have a modified R group or modified backbones (e.g., homoserine, norleucine, methionine, sulfoxide, methionine methyl sulfonium). The phrase "amino acid mimetic" refers to chemical compounds that have different structures but similar functions to general amino acids.

Amino acids may be referred to herein by their commonly known three letter symbols or the one-letter symbols recommended by the IUPAC-IUB Biochemical Nomenclature Commission.

The terms "gene", "polynucleotides", "oligonucleotide", "nucleic acids", and "nucleic acid molecules" are used interchangeably unless otherwise specifically indicated and, similarly to the amino acids, are referred to by their commonly accepted single-letter codes. Similar to the amino acids, they encompass both naturally-occurring and non-naturally occurring nucleic acid polymers. The polynucleotide, oligonucleotide, nucleotides, nucleic acids, or nucleic acid molecules may be composed of DNA, RNA or a combination thereof.

In the context of the present description, the phrase "SUV39H2 gene" encompasses polynucleotides that encode human SUV39H2 gene or any of the functional equivalents of the human SUV39H2 gene. In the context of the present description, the SUV39H2 gene or its functional equivalent can be obtained from nature as naturally occurring proteins via conventional cloning methods or through chemical synthesis based on the selected nucleotide sequence. Methods for cloning genes using cDNA libraries and such are well known in the art.

The terms "isolated" or "purified" used in relation to a substance (e.g., polypeptide, antibody, polynucleotide, etc.) indicate that the substance is removed from its original environment (e.g., the natural environment if naturally occurring) and thus altered from its natural state. Examples of isolated nucleic acids include DNA (such as cDNA), RNA (such as mRNA), and derivatives thereof that are substantially free of other cellular material, or culture medium when produced by recombinant techniques, or substantially free of chemical precursors or other chemicals when chemically synthesized. In a preferred embodiment, nucleic acid molecules encoding antibodies are isolated or purified.

An "isolated" or "purified" antibody refers to antibodies that are substantially free of cellular material for example, carbohydrate, lipid, or other contaminating proteins from the cell or tissue source from which the protein (antibody) is derived, or substantially free of chemical precursors or other chemicals when chemically synthesized. The term "substantially free of cellular material" includes preparations of a polypeptide in which the polypeptide is separated from cellular components of the cells from which it is isolated or recombinantly produced.

Thus, a polypeptide that is substantially free of cellular material includes preparations of polypeptide having less than about 30%, 20%, 10%, or 5% (by dry weight) of heterologous protein (also referred to herein as a "contaminating protein"). When the polypeptide is recombinantly produced, in some embodiments it is also substantially free of culture medium, which includes preparations of polypeptide with culture medium less than about 20%, 10%, or 5% of the volume of the protein preparation. When the polypeptide is produced by chemical synthesis, in some embodiments it is substantially free of chemical precursors or other chemicals, which includes preparations of polypeptide with chemical precursors or other chemicals involved in the synthesis of the protein less than about 30%, 20%, 10%, 5% (by dry weight) of the volume of the protein preparation. That a particular protein preparation contains an isolated or purified polypeptide can be shown, for example, by the appearance of a single band following sodium dodecyl sulfate (SDS)-polyacrylamide gel electrophoresis of the protein preparation and Coomassie Brilliant Blue staining or the like of the gel.

As used herein, the term "biological sample" refers to a whole organism or a subset of its tissues, cells or component parts (e.g., body fluids, including but not limited to blood, mucus, lymphatic fluid, synovial fluid, cerebrospinal fluid, saliva, amniotic fluid, amniotic cord blood, urine, vaginal fluid and semen). "Biological sample" further refers to a homogenate, lysate, extract, cell culture or tissue culture prepared from a whole organism or a subset of its cells, tissues or component parts, or a fraction or portion thereof. Lastly, "biological sample" refers to a medium, for example, a nutrient broth or gel in which an organism has been propagated, which contains cellular components, for example, proteins or polynucleotides. In the context of the present description, a biological sample may preferably contain tissues or cells extracted from the lung, cervix, bladder, esophagus, or prostate, or an osteosarcoma or soft tissue tumor.

Unless otherwise defined, the term "cancer" refers to cancers over-expressing the SUV39H2 gene. Examples of cancers over-expressing SUV39H2 gene include, but are not limited to, lung cancer, cervical cancer, bladder cancer, esophageal cancer, osteosarcoma, prostate cancer and soft tissue tumor.

To the extent that the methods and compositions disclosed therein find utility in the context of "prevention" and "prophylaxis", such terms are interchangeably used herein to refer to any activity that reduces the burden of mortality or morbidity from disease. Prevention and prophylaxis can occur "at primary, secondary and tertiary prevention levels". While primary prevention and prophylaxis avoid the development of a disease, secondary and tertiary levels of prevention and prophylaxis encompass activities aimed at the prevention and prophylaxis of the progression of a disease and the emergence of symptoms as well as reducing the negative impact of an already established disease by restoring function and reducing disease-related complications. Alternatively, prevention and prophylaxis can include a wide range of prophylactic therapies aimed at alleviating the severity of the particular disorder, e.g. reducing the proliferation and metastasis of tumors.

To the extent that certain embodiments encompass the treatment and/or prophylaxis of cancer and/or the prevention of postoperative recurrence, such methods may include any of the following steps: the surgical removal of cancer cells, the inhibition of the growth of cancerous cells, the involution or regression of a tumor, the induction of remission and suppression of occurrence of cancer, the tumor regression, and the reduction or inhibition of metastasis. Effective treatment and/or the prophylaxis of cancer decreases mortality and improves the prognosis of individuals having cancer, decreases the levels of tumor markers in the blood, and alleviates detectable symptoms accompanying cancer. A treatment may also deemed "efficacious" if it leads to clinical benefit such as, reduction in expression of the SUV39H2 gene, or a decrease in size, prevalence, or metastatic potential of the cancer in the subject. When the treatment is applied prophylactically, "efficacious" means that it retards or prevents cancers from forming or prevents or alleviates a clinical symptom of cancer. Efficaciousness is determined in association with any known method for diagnosing or treating the particular tumor type.

### Genes and Polypeptides:

The present invention is based, at least in part, on the discovery that the gene encoding SUV39H2 is over-expressed in several cancers compared to non-cancerous tissue. The SUV39H2 (the suppressor of variegation 3-9 homolog 2), also known as KMT1B (Allis CD, et al Cell 2007;131: 633-636), is a SET-domain containing methyltransferase which selectively methylates H3K9.

Exemplary nucleic acid sequences of the SUV39H2 gene are set forth in SEQ ID NO: 21 (GenBank accession No. NM_001193424.1), 23 (GenBank accession No. NM_001193425.1), 25 (GenBank accession No. NM_001193426.1), 27 (GenBank accession No. NM_001193427.1) and 29 (GenBank accession No. NM_024670.3). Exemplary amino acid sequences of the polypeptide encoded by the SUV39H2 gene are SEQ ID NO: 22 (GenBank accession No. NP_001180353.1), 24 (GenBank accession No. NP_001180354.1 or NP_078946.1), 26 (GenBank accession No. NP_001180355.1),28 (GenBank accession No. NP_001180356.1), and 30 (GenBank accession No. NP_078946.1). However, the description is not limited to these sequences.

Herein, the polypeptide encoded by the SUV39H2 gene is referred to as the "SUV39H2 polypeptide" or "SUV39H2 protein", or simply "SUV39H2". In the context of the present description, the phrase "SUV39H2 gene" encompasses not only polynucleotides that encode the human SUV39H2 polypeptide but also polynucleotides that encode functional equivalents of the human SUV39H2 gene. The SUV39H2 gene can be obtained from nature as naturally occurring polynucleotides via conventional cloning methods or through chemical synthesis based on the selected nucleotide sequence. As noted above and discussed in greater detail below, methods for cloning genes using cDNA libraries and such are well known in the art. As noted above, the present description extends to "functional equivalents" of the protein (polypeptide) and deems such to be further examples of "SUV39H2 polypeptides". Herein, a "functional equivalent" of a protein (e.g., an SUV39H2 polypeptide) is a polypeptide that has a biological activity equivalent to that of the original reference protein. Accordingly, any polypeptide that retains the biological activity of the SUV39H2 protein is considered to be a functional equivalent thereof in the context of the present description. For example, functional equivalents of the SUV39H2 polypeptide may retain cell-proliferation promoting activity and/or methyltransferase activity of the native SUV39H2 polypeptide. In the context of the present description, functional equivalents of the SUV39H2 polypeptide include polymorphic variants, interspecies homologues, and those encoded by alleles of these polypeptides.

Preferred functional equivalents of the SUV39H2 polypeptide retain either or both of the cell-proliferation promoting activity and methyltransferase activity of the native SUV39H2 polypeptide. Functional equivalents of the SUV39H2 polypeptide that retain the methyltransferase activity of the SUV39H2 polypeptide are expected to retain the SET-domain of the native SUV39H2 polypeptide. In the aforementioned sequences, the SET-domain is located in the amino acid position 251-372 of the amino acid sequence shown in SEQ ID NO: 22, the amino acid position 191-312 of the amino acid sequence shown in SEQ ID NO: 24, the amino acid position 100-192 of the amino acid sequence shown in SEQ ID NO: 26, the amino acid position 40-132 of the amino acid sequence shown in SEQ ID NO: 28, and 191-312 of the amino acid sequence shown in SEQ ID NO: 30. An example of such a functional equivalent is set forth in the amino acid sequence of SEQ ID NO: 32.

Examples of functional equivalents include those wherein one or more, e.g., 1-5 amino acids or up to 5% of the original amino acids are substituted, deleted, added, or inserted to the natural occurring amino acid sequence of the SUV39H2 protein. Alternatively, the polypeptide may be composed of an amino acid sequence having at least about 80% homology (also referred to as sequence identity) to the sequence of the respective protein, more preferably at least about 90% to 95% homology, often about 96%, 97%, 98% or 99% homology. In other embodiments, the polypeptide can be encoded by a polynucleotide that hybridizes under stringent conditions to the natural occurring nucleotide sequence of the SUV39H2 gene.

Polypeptides described herein may have variations in amino acid sequence, molecular weight, isoelectric point, the presence or absence of sugar chains, or form, depending on the cell or host used to produce it or the purification method utilized. Nevertheless, so long as it is functionally equivalent to the human SUV39H2 protein, it is within the scope of functional equivalents of the SUV39H2 polypeptide.

With respect to functional equivalents composed of mutated or modified form of the native SUV39H2 polypeptide, wherein one or more, amino acids are substituted, deleted, added, or inserted to the naturally occurring sequence, it is generally known that modifications of one, two or more amino acids in a protein will not significantly impact or influence the function of the protein. In some cases, it may even enhance the desired function of the original protein. In fact, mutated or modified proteins, i.e., proteins having amino acid sequences modified by substituting, deleting, inserting and/ or adding one, two or more amino acid residues of a certain amino acid sequence, can retain the original biological activity (Mark et al., Proc Natl Acad Sci USA 81: 5662-6 (1984); Zoller and Smith, Nucleic Acids Res 10:6487-500 (1982); Dalbadie-McFarland et al., Proc Natl Acad Sci USA 79: 6409-13 (1982)). Accordingly, one of skill in the art will recognize that individual additions, deletions, insertions, and substitutions to an amino acid sequence which alter a single amino acid or a small percentage of amino acids (i.e., less than 5%, more preferably less than 3%, even more preferably less than 1%) or those considered to be a "conservative modifications", wherein the alteration of a protein results in a protein with similar functions, are acceptable in the context of the present description. Thus, the functional equivalents of the SUV39H2 polypeptide may have an amino acid sequence wherein one, two or even more amino acids are added, inserted, deleted, and/or substituted in a naturally occurring human SUV39H2 polypeptide sequence.

So long as the activity of the SUV39H2 protein is maintained, the site and number of amino acid mutations is not particularly limited. Accordingly, the mutation / modification (i.e., addition, deletion, insertion, or substitution to an amino acid sequence) may be introduced at the N- and C-terminals as well as intermediate sites. However, it is generally preferred to alter 5% or less of the amino acid sequence, more preferably less than 3%, even more preferably less than 1%. Accordingly, in a preferred embodiment, the number of amino acids to be mutated in such a mutant is generally 30 amino acids or fewer, preferably 20 amino acids or fewer, more preferably 10 amino acids or fewer, more preferably 6 amino acids or fewer, and even more preferably 3 amino acids or fewer.

An amino acid residue to be mutated is preferably mutated into a different amino acid in which the properties of the amino acid side-chain are conserved (a process known as conservative amino acid substitution). Examples of properties of amino acid side chains are hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (G, A, V, L, I, P); a hydroxyl group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W). Conservative substitution tables providing functionally similar amino acids are well known in the art. For example, the following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M) (see, e.g., Creighton, Proteins 1984).

Such conservatively modified polypeptides are included in functional equivalents of the SUV39H2 protein in the context of the present description. However, the present description is not restricted thereto and functional equivalents of the SUV39H2 protein can include non-conservative modifications, so long as the resulting modified peptide retains at least one biological activity of the SUV39H2 protein. The number of amino acids to be mutated in such a modified polypeptide is generally 20 amino acids or less, preferably, 10 amino acids or less, 6 amino acids or less, 5 amino acids or less, 4 amino acids or less, 3 amino acids or less, or 2 amino acids or less. Furthermore, the modified proteins do not exclude polymorphic variants, interspecies homologues, and those encoded by alleles of these proteins.

An example of functional equivalents of the SUV39H2 polypeptide modified by addition of several amino acid residues is a fusion protein of the SUV39H2 polypeptide and other polypeptides or peptides. Such fusion proteins can be made by techniques well known to a person skilled in the art, for example, by linking the DNA encoding the SUV39H2 gene with a DNA encoding other polypeptides or peptides, so that the frames match, inserting the fusion DNA into an expression vector and expressing it in a host. The "other" component of the fusion protein is typically a small epitope composed of several to a dozen amino acids. There is no restriction as to polypeptides or peptides fused to the SUV39H2 polypeptide so long as a resulting fusion protein retains any one of the objective biological activities of the SUV39H2 polypeptide.

Exemplary fusion proteins contemplated by the present description include fusions of the SUV39H2 polypeptide and an other small peptide such as FLAG (Hopp TP, et al., Biotechnology 6: 1204-10 (1988)), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, lck tag, alpha-tubulin fragment, B-tag, Protein C fragment, and the like. Examples of proteins that can be fused to the SUV39H2 polypeptide include GST (glutathione-S-transferase), Influenza agglutinin (HA), immunoglobulin constant region, beta-galactosidase, MBP (maltose-binding protein), and such.

Furthermore, functional equivalents of the SUV39H2 polypeptide do not exclude polymorphic variants, interspecies homologues, and those encoded by alleles of these polypeptides.

Methods known in the art to isolate functional equivalents include, for example, hybridization techniques (Sambrook and Russell, Molecular Cloning: A Laboratory Manual, 3rd ed., Cold Spring Harbor Lab. Press, 2001). One skilled in the art can readily isolate a DNA having high homology (i.e., sequence identity) with a whole or part of the human SUV39H2 DNA sequences (e.g., SEQ ID NO: 21, 23, 25, 27 or 29) encoding the human SUV39H2 polypeptide, and isolate functional equivalents of the human SUV39H2 polypeptide from the isolated DNA.

Hybridization conditions suitable for isolating a DNA encoding a functional equivalent of the human SUV39H2 gene can be routinely selected by a person skilled in the art. As used herein, the phrase "stringent (hybridization) conditions" refers to conditions under which a nucleic acid molecule will hybridize to its target sequence, typically in a complex mixture of nucleic acids, but not detectably to other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology-Hybridization with Nucleic Probes, "Overview of principles of hybridization and the strategy of nucleic acid assays" (1993).

Generally, stringent conditions are selected to be about 5-10 degrees C lower than the thermal melting point (Tm) for the specific sequence at a defined ionic strength and pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions may also be achieved with the addition of destabilizing agents such as formamide. For selective or specific hybridization, a positive signal is at least two times of background, preferably 10 times of background hybridization. Exemplary stringent hybridization conditions include the following: 50% formamide, 5x SSC, and 1% SDS, incubating at 42 degrees C, or, 5x SSC, 1% SDS, incubating at 65 degrees C, with wash in 0.2x SSC, and 0.1% SDS at 50 degrees C.

In the context of the present description, an optimal condition of hybridization for isolating a DNA encoding a polypeptide functionally equivalent to the human SUV39H2 protein can be routinely selected by a person skilled in the art. For example, hybridization may be performed by conducting pre-hybridization at 68 degrees C for 30 min or longer using "Rapid-hyb buffer" (Amersham LIFE SCIENCE), adding a labeled probe, and warming at 68 degrees C for 1 hour or longer. The following washing step can be conducted, for example, in a low stringent condition. An exemplary low stringent condition may include 42 degrees C, 2x SSC, 0.1% SDS, preferably 50 degrees C, 2x SSC, 0.1% SDS. High stringency conditions are often preferably used. An exemplary high stringency condition may include washing 3 times in 2x SSC, 0.01% SDS at room temperature for 20 min, then washing 3 times in 1x SSC, 0.1% SDS at 37 degrees C for 20 min, and washing twice in 1x SSC, 0.1% SDS at 50 degrees C for 20 min. However, several factors, such as temperature and salt concentration, can influence the stringency of hybridization and one skilled in the art can routinely adjust these and other factors to arrive at the desired stringency.

Thus, functional equivalents of the SUV39H2 polypeptide used in the present description
include polypeptides encoded by DNAs that hybridize under stringent conditions with a whole or part of the DNA sequence encoding the human SUV39H2 polypeptide. These functional equivalents include mammal homologues corresponding to the human SUV39H2 polypeptide (for example, polypeptides encoded by monkey, mouse, rat, rabbit or bovine SUV39H2 genes).

In place of hybridization, a gene amplification method, for example, the polymerase chain reaction (PCR) method, can be utilized to isolate a DNA encoding a functional equivalent of the human SUV39H2 polypeptide, using a primer synthesized based on the sequence information of the DNA (SEQ ID NO: 21,23,25,27 or 29) encoding the human SUV39H2 polypeptide (SEQ ID NO: 22,24,26,28 or 30), examples of primer sequences are pointed out in Semi-quantitative RT-PCR in EXAMPLE.

Functional equivalents of the human SUV39H2 polypeptide encoded by the DNA isolated through the above hybridization techniques or gene amplification techniques, normally have a high homology (also referred to as sequence identity) to the amino acid sequence of the human SUV39H2 polypeptide. "High homology" (also referred to as "high sequence identity") typically refers to the degree of identity between two optimally aligned sequences (either polypeptide or polynucleotide sequences). Typically, high homology or sequence identity refers to homology of 40% or higher, for example, 60% or higher, for example, 80% or higher, for example, 85%, 90%, 95%, 98%, 99%, or higher. The degree of homology or identity between two polypeptide or polynucleotide sequences can be determined by following the algorithm [Wilbur WJ & Lipman DJ. Proc Natl Acad Sci USA. 1983 Feb; 80 (3):726-30].

Percent sequence identity and sequence similarity can be readily determined using conventional techniques such as the BLAST and BLAST 2.0 algorithms, which are described [Altschul SF, et al., J Mol Biol. 1990 Oct 5; 215 (3):403-10; Nucleic Acids Res. 1997 Sep 1;25(17):3389-402]. Software for performing BLAST analyses is publicly available through the National Center for Biotechnology Information (on the worldwide web at ncbi.nlm.nih.gov/). The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits acts as seeds for initiating searches to find longer HSPs containing them.

The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached.

The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands. For amino acid sequences, the BLASTP program uses as defaults a word size (W) of 3, an expectation (E) of 10, and the BLOSUM62 scoring matrix [Henikoff S & Henikoff JG. Proc Natl Acad Sci USA. 1992 Nov 15;89(22):10915-9].

Also disclosed are partial peptides of the SUV39H2 polypeptide and their use in screening methods. A partial peptide of the SUV39H2 polypeptide has an amino acid sequence specific to the SUV39H2 polypeptide and is preferably composed of less than about 400 amino acids, usually less than about 200 and often less than about 100 amino acids, and at least 7 amino acids, preferably, 8 amino acids or more, 9 amino acids or more, 10 amino acids or more, 15 amino acids or more, 20 amino acids or more, 50 amino acids or more, or 80 amino acids or more.

The SUV39H2 polypeptide and functional equivalent thereof used in the present description can be obtained from nature as naturally occurring proteins via conventional purification methods or through chemical synthesis based on the selected amino acid sequence. For example, conventional peptide synthesis methods that can be adopted for the synthesis include:
(1) Peptide Synthesis, Interscience, New York, 1966;
(2) The Proteins, Vol. 2, Academic Press, New York, 1976;
(3) Peptide Synthesis (in Japanese), Maruzen Co., 1975;
(4) Basics and Experiment of Peptide Synthesis (in Japanese), Maruzen Co., 1985;
(5) Development of Pharmaceuticals (second volume) (in Japanese), Vol. 14 (peptide synthesis), Hirokawa, 1991;
(6) WO99/67288; and
(7) Barany G. & Merrifield R.B., Peptides Vol. 2, "Solid Phase Peptide Synthesis", Academic Press, New York, 1980, 100-118.

Alternatively, the SUV39H2 polypeptide and functional equivalent thereof can be obtained adopting any known genetic engineering methods for producing polypeptides (e.g., Morrison DA., et al., J Bacteriol. 1977 Oct;132(1):349-51; Clark-Curtiss JE & Curtiss R 3rd. Methods Enzymol. 1983;101:347-62). For example, first, a suitable vector comprising a polynucleotide encoding the objective polypeptide in an expressible form (e.g., downstream of a regulatory sequence comprising a promoter) is prepared, transformed into a suitable host cell, and then the host cell is cultured to produce the polypeptide. More specifically, a gene encoding the SUV39H2, polypeptide or functional equivalent thereof is expressed in host (e.g., animal) cells and such by inserting the gene into a vector for expressing foreign genes, for example, pSV2neo, pcDNA I, pcDNA3.1, pCAGGS, or pCD8.

In addition to the protein of interest, the vector may also contain a promoter to induce protein expression. Any commonly used promoters can be employed including, for example, the SV40 early promoter (Rigby in Williamson (ed.), Genetic engineering, vol. 3. Academic Press, London, 1982, 83-141), the EF- alpha promoter (Kim DW, et al. Gene. 1990 Jul 16;91(2):217-23), the CAG promoter (Niwa H, et al., Gene. 1991 Dec 15;108(2):193-9), the RSV LTR promoter (Cullen BR. Methods Enzymol. 1987;152:684-704), the SR alpha promoter (Takebe Y, et al., Mol Cell Biol. 1988 Jan;8(1):466-72), the CMV immediate early promoter (Seed B & Aruffo A. Proc Natl Acad Sci USA. 1987 May;84(10):3365-9), the SV40 late promoter (Gheysen D & Fiers W. J Mol Appl Genet. 1982;1(5):385-94), the Adenovirus late promoter (Kaufman RJ, et al., Mol Cell Biol. 1989 Mar;9(3):946-58), the HSV TK promoter, and the like.

The introduction of the vector into host cells to express the gene encoding the SUV39H2 polypeptide or functional equivalent thereof can be performed according to any methods, for example, the electroporation method (Chu G, et al., Nucleic Acids Res. 1987 Feb 11; 15(3):1311-26), the calcium phosphate method (Chen C & Okayama H. Mol Cell Biol. 1987 Aug;7(8):2745-52), the DEAE dextran method (Lopata MA, et al., Nucleic Acids Res. 1984 Jul 25;12(14):5707-17; Sussman DJ & Milman G. Mol Cell Biol. 1984 Aug;4(8):1641-3), the Lipofectin method (Derijard B, et al., Cell. 1994 Mar 25;76(6):1025-37; Lamb BT, et al., Nat Genet. 1993 Sep;5(1):22-30; Rabindran SK, et al., Science. 1993 Jan 8;259(5092):230-4), and the like.

The SUV39H2 polypeptide and functional equivalent thereof can also be produced in vitro by using an in vitro translation system.

### Methods for Diagnosing or Detecting Cancer:

The present description relates to the discovery that SUV39H2 can serve as a diagnostic marker of cancer and thus finds utility in the detection of cancers related thereto. As demonstrated herein, the expression of the SUV39H2 gene is specifically and significantly elevated in lung cancer, cervical cancer, bladder cancer, esophageal cancer, osteosarcoma, prostate cancer and soft tissue tumor (Figures 1, 3, 6, 10 and Table 6). Therefore, the SUV39H2 gene identified herein as well as its transcription and translation products find diagnostic utility as a marker for cancers as above, and by measuring the expression of the SUV39H2 gene in a sample. Those cancers can be diagnosed or detected by comparing the expression level of the SUV39H2 gene in a subject-derived sample with that in a normal sample. Specifically, the present description provides a method of diagnosing or detecting cancer by determining the expression level of the SUV39H2 gene in a subject-derived sample. Exemplary cancers that can be diagnosed or detected by the method of the present description include lung cancer, cervical cancer, bladder cancer, esophageal cancer, osteosarcoma, prostate cancer and soft tissue tumor. In the context of the present description, lung cancer includes NSCLC and SCLC. Furthermore, NSCLC includes lung adenocarcinoma (ADC), lung squamous cell carcinoma (SCC) and lung large cell carcinoma (LCC).

In the context of the present description the term "diagnosing" can encompass detection as well as predictions and likelihood analyses. It is an object of the present description to provide a method for diagnosing or detecting cancer using the expression level of the SUV39H2 gene as an index of cancer in a subject-derived biological sample, wherein an increased or elevated SUV39H2 expression level in the sample as compared to a control level indicates the presence or suspicion of cancer cells in the sample. The present description
provides a method for detecting or identifying cancer cells in a subject-derived tissue sample, the method including the step of determining the expression level of the SUV39H2 gene in the tissue sample, wherein an increase in the expression level as compared to a normal control level of the gene indicates the presence or suspicion of cancer cells in the tissue sample.

According to the present description, an intermediate result for examining the condition of a subject may be provided, i.e., a method of monitoring a subject. Such intermediate result may be combined with additional information to assist a doctor, nurse, or other practitioner to diagnose that a subject suffers from the disease. Alternatively, the present description may be used to detect cancerous cells in a subject-derived tissue, and provide a doctor with useful information to diagnose that the subject suffers from the disease.

Diagnostic methods may be used clinically in making decisions concerning treatment modalities, including therapeutic intervention, diagnostic criteria such as disease stages, and disease monitoring and surveillance for cancer. To improve the accuracy of diagnosis, the expression level of other cancer-associated genes, for example, genes known to be differentially expressed in cancer may also be determined. Furthermore, in the case where the expression levels of multiple cancer-related genes are compared, a similarity in the gene expression pattern between the sample and the reference that is cancerous indicates that the subject is suffering from or at a risk of developing lung cancer..

Accordingly, SUV39H2 expression results may be combined with additional information or another diagnostic indicator, including tissue pathology, levels of known tumor marker(s) in blood, and clinical course of the subject, to assist a doctor, nurse, or other healthcare practitioner in determining whether a test subject is afflicted with the disease. For example, the expression level of the SUV39H2 gene can be used in combination with another diagnostic indicator, including tissue pathology, levels of known tumor marker(s) in blood, and clinical course of the subject, etc. For example, some well-known diagnostic lung cancer markers in blood include CYFRA, ProGRP, NSC, SLX, CA19-9, CEA, TPA and BFP, cervical cancer markers in blood include STN, CA72-4 and SCC, bladder cancer markers in blood include NMP22, BFP, TPA, esophageal cancer markers in blood include SCC, CEA, TPA, BFP and CA19-9, prostate cancer markers in blood include PSA, PAP and BFP. Namely, in this particular embodiment the outcome of the gene expression analysis serves as an intermediate result for further diagnosis of a subject's disease state.

The method of diagnosing cancer will be described in more detail below.

A subject to be diagnosed by the present method is preferably a mammal. Exemplary mammals include, but are not limited to, e.g., human, non-human primate, mouse, rat, dog, cat, horse, and cow.

The method of the present description preferably utilizes a biological sample obtained or collected from the subject to be diagnosed. Any biological material can be used as the biological sample for the determination so long as it can include the objective transcription or translation product of the SUV39H2 gene. Examples of suitable subject-derived biological samples include, but are not limited to, bodily tissues such as biopsy specimen and fluids such as blood, sputum and urine. Preferably, the biological sample contains a cell population including an epithelial cell, more preferably a cancerous epithelial cell or an epithelial cell derived from a tissue suspected to be cancerous. Further, if necessary, the cell may be purified from the obtained bodily tissues and fluids, and then used as the biological sample.

In the context of the present description, any cancer that over-expresses the SUV39H2 gene can be diagnosed or detected by the method of the present description. Preferred cancers to be diagnosed or detected include lung cancer, cervical cancer, bladder cancer, esophageal cancer, osteosarcoma, prostate cancer and soft tissue tumor. In order to diagnose or detect such cancers, a subject-derived biological sample is preferably collected from the following organs: lung for lung cancer; uterine cervix for cervical cancer; bladder for bladder cancer; esophagus for esophageal cancer; bone for osteosarcoma; prostate for prostate cancer; and soft tissue for soft tissue tumor.

Preferably, a subject-derived biological sample is collected from an area suspected to be cancerous in the aforementioned organs. Therefore, a lung tissue sample, uterine cervix tissue sample, bladder tissue sample, esophageal tissue, bone tissue, prostate tissue, or soft tissue sample collected from a suspicious area can be preferably used as a subject-derived biological sample for diagnosis or detection of lung cancer, cervical cancer, bladder cancer, esophageal cancer, osteosarcoma, prostate cancer or soft tissue tumor, respectively. Such tissue samples can be obtained by biopsy or surgical resection.

According to the present description, the expression level of the SUV39H2 gene in the subject-derived biological sample is determined and then correlated to a particular healthy or disease state by comparison to a control sample. The expression level of the SUV39H2 gene can be determined at the transcription product level, using methods known in the art. For example, the mRNA of SUV39H2 may be quantified using probes by hybridization methods (e.g., Northern hybridization). The detection may be carried out on a chip or an array. The use of an array is preferable for detecting the expression level of a plurality of genes (e.g., various cancer specific genes) including the SUV39H2 gene. Those skilled in the art can prepare such probes utilizing the known sequence information for the SUV39H2 gene. For example, the cDNA of the SUV39H2 gene may be used as the probes. If necessary, the probe may be labeled with a suitable label, such as dyes, fluorescent and isotopes, and the expression level of the gene may be detected as the intensity of the hybridized labels.

Alternatively, the transcription product of the SUV39H2 gene may be quantified using primers by amplification-based detection methods (e.g., RT-PCR). Such primers can also be prepared based on the available sequence information of the SUV39H2 gene. For example, the primers (SEQ ID NOs: 5 and 6 for SUV39H2) used in the Example may be employed for the detection by RT-PCR or Northern blot, but the present invention is not restricted thereto.

A probe or primer suitable for use in the context of the present method will hybridize under stringent, moderately stringent, or low stringency conditions to the mRNA of SUV39H2. Details of "stringent conditions" are described in the section entitled "Genes and Polypeptides". Alternatively, diagnosis may involve detection of an SUV39H2 translation product i.e., polypeptide or protein), using methods known in the art. For example, the quantity of the SUV39H2 protein may be determined using an antibody against the SUV39H2 polypeptide and correlated to a disease or normal state. Herein, "antibody against the SUV39H2 polypeptide" refers to an antibody that is raised against the SUV39H2 polypeptide or fragment thereof and specifically binds to the SUV39H2 polypeptide. Herein, "specifically bind to the SUV39H2 polypeptide" means that an antibody binds to the SUV39H2 polypeptide, but not detectably to other polypeptides.

The quantity of the translation products/proteins may be determined using, for example immunoassay methods that use an antibody specifically recognizing the protein. Antibodies suitable for use in the context of the methods of the present invention may be monoclonal or polyclonal. Furthermore, any immunological fragments or modified antibody (e.g., chimeric antibody, scFv, Fab, F(ab')2, Fv, etc.) of the antibody may be used for the detection, so long as the fragment or modified antibody retains the binding ability to the SUV39H2 protein. Methods to prepare these kinds of antibodies for the detection of proteins are well known in the art, and any method may be employed in the present description to prepare such antibodies and equivalents thereof.

Alternatively, one may determine the expression level of the SUV39H2 gene based on its translation product, for example through study of the intensity of staining observed via immunohistochemical analysis using an antibody against the SUV39H2 protein. More particularly, the observation of strong staining indicates increased presence of the protein and at the same time high expression level of the SUV39H2 gene.

Furthermore, the translation product may be detected based on its biological activity. As discovered herein, the SUV39H2 protein was demonstrated herein to be involved in the growth of cancer cells. Thus, the cancer cell growth promoting activity of the SUV39H2 protein may be used as an index of the SUV39H2 protein existing in a biological sample. Alternatively, methyltransferase activity of the SUV39H2 protein may be also used for the determination of the expression level of the SUV39H2 gene.

Moreover, in addition to the expression level of the SUV39H2 gene, the expression level of other cancer-associated genes, for example, genes known to be differentially expressed in cancer may also be determined to confirm the diagnosis.

In the context of the present description, methods for detecting or identifying cancer in a subject or cancer cells in a subject-derived sample begin with a determination of SUV39H2 gene expression level. The expression level may be determined by any of the aforementioned techniques. Once determined, then, this level may be compared to a control level. In the context of the present description, gene expression levels are deemed to be "altered" or "increased" when the gene expression changes or increases by, for example, 10%, 25%, or 50% from, or at least 0.1 fold, at least 0.2 fold, at least 0.5 fold, at least 2 fold, at least 5 fold, or at least 10 fold or more compared to a control level. Accordingly, the expression level of cancer marker genes including SUV39H2 gene in a biological sample can be considered to be increased if it increases from a control level of the corresponding lung cancer marker gene by, for example, 10%, 25%, or 50%; or increases to more than 1.1 fold, more than 1.5 fold, more than 2.0 fold, more than 5.0 fold, more than 10.0 fold, or more.

In the context of the present description, the phrase "control level" refers to the expression level of the SUV39H2 gene detected in a control sample and encompasses both a normal control level and a cancer control level. The phrase "normal control level" refers to a level of the SUV39H2 gene expression detected in a normal healthy individual or in a population of individuals known not to be suffering from cancer. A normal individual is one with no clinical symptom of cancer. A normal control level can be determined using a normal cell obtained from a non-cancerous tissue. A "normal control level" may also be the expression level of the SUV39H2 gene detected in a normal healthy tissue or cell of an individual or population known not to be suffering from cancer. On the other hand, the phrase "cancer control level" or "cancerous control level" refers to an expression level of the SUV39H2 gene detected in the cancerous tissue or cell of an individual or population suffering from cancer.

An increase in the expression level of SUV39H2 detected in a subject-derived sample as compared to a normal control level indicates that the subject (from which the sample has been obtained) suffers from or is at risk of developing cancer. In the context of the present description, the subject-derived sample may be any tissues obtained from test subjects, e.g., patients known to have or suspected of having cancer. For example, tissues may include epithelial cells. More particularly, tissues may be epithelial cells suspected to be cancerous. A similarity between the expression level of a sample and the cancer control level indicates that the subject (from which the sample has been obtained) suffers from or is at risk of developing cancer. When the expression levels of other cancer-related genes are also measured and compared, a similarity in the gene expression pattern between the sample and the reference that is cancerous indicates that the subject is suffering from or at a risk of developing cancer.

The control level may be determined at the same time with a test biological sample by using a sample(s) previously collected and stored from a subject/subjects whose disease state (cancerous or non-cancerous) is/are known. Alternatively, the control level may be determined by a statistical method based on the results obtained by analyzing previously determined expression level(s) of the SUV39H2 gene in samples from subjects whose disease state are known. Furthermore, the control level can be a database of expression patterns from previously tested cells. Moreover, according to an aspect of the present description, the expression level of the SUV39H2 gene in a biological sample may be compared to multiple control levels, which control levels are determined from multiple reference samples. It is preferred to use a control level determined from a reference sample derived from a tissue type similar to that of the subject-derived biological sample. Moreover, it is preferred to use the standard value of the expression levels of the SUV39H2 gene in a population with a known disease state. The standard value may be obtained by any method known in the art. For example, a range of mean +/- 2 S.D. or mean +/- 3 S.D. may be used as standard value.

When the expression level of the SUV39H2 gene is increased as compared to the normal control level or is similar to the cancerous control level, the subject may be diagnosed as suffering from or at a risk of developing cancer. Furthermore, in the case where the expression levels of multiple cancer-related genes are compared, a similarity in the gene expression pattern between the sample and the reference that is cancerous indicates that the subject is suffering from or at a risk of developing cancer.

Differences between the expression levels of a test biological sample and the control level can be normalized to the expression level of control nucleic acids, e.g., housekeeping genes, whose expression levels are known not to differ depending on the cancerous or non-cancerous state of the cell. Exemplary control genes include, but are not limited to, beta-actin, glyceraldehyde 3 phosphate dehydrogenase, and ribosomal protein P1.

To facilitate the afore-mentioned uses, also disclosed are diagnostic reagents for diagnosing cancer. Such reagents can be selected from among:
(a) a reagent for detecting mRNA of the SUV39H2 gene;
(b) a reagent for detecting the SUV39H2 protein; and
(c) a reagent for detecting the biological activity of the SUV39H2 protein.

Specifically, such reagent is an oligonucleotide that hybridizes to a transcription product of the SUV39H2 gene, or an antibody that binds to the SUV39H2 polypeptide. In other words, also disclosed is a kit for use in diagnosis or detection of cancer, wherein the kit includes a reagent that binds to a transcription or translation product of the SUV39H2 gene.

The findings of the present description reveal that SUV39H2 is not only a useful diagnostic marker, but also a suitable target for cancer therapy. Therefore, cancer treatments targeting SUV39H2 can be achieved by the present invention. In the context of the present description, the phrase "cancer treatment targeting SUV39H2" refers to suppression or inhibition of SUV39H2 activity and/or expression in the cancer cells. Any anti-SUV39H2 agents may be used for the cancer treatment targeting SUV39H2. In the context of the present description preferred anti-SUV39H2 agents include following substance as active ingredient:
(a) a double-stranded molecule of the present invention,
(b) DNA encoding thereof, or
(c) a vector encoding thereof, all of which are discussed in greater detail below.

Accordingly, in a preferred embodiment, the present description provides a method of
(i) diagnosing whether a subject has the cancer to be treated, and/or (ii) selecting a subject for cancer treatment, which method includes the steps of:
   (a) determining the expression level of SUV39H2 in cancer cells or tissue(s) obtained from a subject who is suspected to have the cancer to be treated;
   (b) comparing the expression level of SUV39H2 with a normal control level;
   (c) diagnosing the subject as having the cancer to be treated, if the expression level of SUV39H2 is increased as compared to the normal control level; and
   (d) selecting the subject for cancer treatment, if the subject is diagnosed as having the cancer to be treated, in step (c).

Alternatively, such a method includes the steps of:
(a) determining the expression level of SUV39H2 in cancer cells or tissue(s) obtained from a subject who is suspected to have the cancer to be treated;
(b) comparing the expression level of SUV39H2 with a cancerous control level;
(c) diagnosing the subject as having the cancer to be treated, if the expression level of SUV39H2 is similar or equivalent to the cancerous control level; and
(d) selecting the subject for cancer treatment, if the subject is diagnosed as having the cancer to be treated, in step (c).

Exemplary cancers include, but are not limited to, lung cancer, cervical cancer, bladder cancer, esophageal cancer, osteosarcoma, prostate cancer and/or soft tissue tumor. Accordingly, prior to the administration of the double-stranded molecule of the present description as active ingredient, it is preferable to confirm whether the expression level of SUV39H2 in the cancer cells or tissues to be treated is enhanced as compared with normal cells of the same organ. Thus, in one embodiment, also disclosed is a method for treating a cancer (over)expressing SUV39H2, which method may include the steps of:
i) determining the expression level of SUV39H2 in cancer cells or tissue(s) obtained from a subject with the cancer to be treated;
ii) comparing the expression level of SUV39H2 with normal control; and
iii) administrating at least one component selected from the group consisting of
   (a) a double-stranded molecule of the present invention,
   (b) DNA encoding thereof, and
   (c) a vector encoding thereof,
   to a subject with a cancer over-expressing SUV39H2 as compared with normal control. Alternatively, also disclosed is a pharmaceutical composition comprising at least one component selected from the group consisting of:
   (a) a double-stranded molecule of the present description,
   (b) DNA encoding thereof, and
   (c) a vector encoding thereof,
   for use in administrating to a subject having a cancer overexpressing SUV39H2. In other words, also disclosed is a method for identifying a subject to be treated with:
   (a) a double-stranded molecule of the present invention,
   (b) DNA encoding thereof, or
   (c) a vector encoding thereof.

Such a method may include the step of determining an expression level of SUV39H2 in subject-derived cancer cells or tissue(s), wherein an increase of the level compared to a normal control level of the gene indicates that the subject has cancer which may be treated with a double-stranded molecule of the present description.

The method of treating a cancer will be described in more detail below.

A subject to be treated by the present method is preferably a mammal. Exemplary mammals include, but are not limited to, e.g., human, non-human primate, mouse, rat, dog, cat, horse, and cow. The expression level of SUV39H2 in cancer cells or tissues obtained from a subject is determined, for example, at the transcription (nucleic acid) product level, using methods known in the art. For example, hybridization methods (e.g., Northern hybridization), a chip or an array, probes, RT-PCR can be used to determine the transcription product level of SUV39H2.

Alternatively, the translation product may be detected for the treatment of the present description. For example, the quantity of observed protein (SEQ ID NO: 22, 24, 26, 28, and 30) may be determined.

As another method to detect the expression level of SUV39H2 gene based on its translation product, the intensity of staining may be measured via immunohistochemical analysis using an antibody against the SUV39H2 protein. Namely, in this measurement, strong staining indicates increased presence/level of the protein and, at the same time, high expression level of SUV39H2 gene.

Methods for detecting or measuring the SUV39H2 polypeptide and/or polynucleotide encoding thereof can be exemplified as described above.

### A Kit for Diagnosing Cancer:

Also disclosed is a kit for diagnosing or detecting cancer. Preferably, the cancer may be selected from the group consisting of lung cancer, cervical cancer, bladder cancer, esophageal cancer, osteosarcoma, prostate cancer and soft tissue tumor.

The kit includes at least one reagent for detecting the expression level of the SUV39H2 gene in a subject-derived biological sample, which reagent may be selected from the group of:
(a) a reagent for detecting an mRNA of the SUV39H2 gene;
(b) a reagent for detecting the SUV39H2 protein; and
(c) a reagent for detecting the biological activity of the SUV39H2 protein.

Suitable reagents for detecting an mRNA of the SUV39H2 gene include nucleic acids that specifically bind to or identify the SUV39H2 mRNA, such as oligonucleotides that have a sequence complementary to a part of the SUV39H2 mRNA. These kinds of oligonucleotides are exemplified by primers and probes that are specific to the SUV39H2 mRNA. These kinds of oligonucleotides may be prepared based on methods well known in the art. If needed, the reagent for detecting the SUV39H2 mRNA may be immobilized on a solid matrix. Moreover, more than one reagent for detecting the SUV39H2 mRNA may be included in the kit.

The probe or primer typically comprises a substantially purified oligonucleotide. The oligonucleotide typically comprises a region of nucleotide sequence that hybridizes under stringent conditions to at least about 2000, 1000, 500, 400, 350, 300, 250, 200, 150, 100, 50, or 25, consecutive sense strand nucleotide sequence of a nucleic acid comprising an SUV39H2 sequence, or an antisense strand nucleotide sequence of a nucleic acid comprising an SUV39H2 sequence, or of a naturally occurring mutant of these sequences. In particular, for example, in a preferred embodiment, an oligonucleotide having 5-50 in length can be used as a primer for amplifying the genes, to be detected. More preferably, mRNA or cDNA of the SUV39H2 gene can be detected with oligonucleotide probe or primer having 15-30bp in length. In preferred embodiments, length of the oligonucleotide probe or primer can be selected from 15-25bp. Assay procedures, devices, or reagents for the detection of gene by using such oligonucleotide probe or primer are well known (e.g. oligonucleotide microarray or PCR). In these assays, probes or primers can also comprise tag or linker sequences. Further, probes or primers can be modified with detectable label or affinity ligand to be captured. Alternatively, in hybridization based detection procedures, a polynucleotide having a few hundreds (e.g., about 100-200) bases to a few kilo (e.g., about 1000-2000) bases in length can also be used for a probe (e.g., northern blotting assay or cDNA microarray analysis).

On the other hand, suitable reagents for detecting the SUV39H2 protein include antibodies to the SUV39H2 protein. The antibody may be monoclonal or polyclonal. Furthermore, any immunological fragment or modified antibody (e.g., chimeric antibody, scFv, Fab, F(ab')2, Fv, etc.) of the antibody may be used as the reagent, so long as the fragment or modified antibody retains the ability to bind the SUV39H2 protein.

Methods to prepare these kinds of antibodies for the detection of proteins are well known in the art, and any method may be employed in the present description to prepare such antibodies and equivalents thereof.

The antibody may be labeled with signal generating molecules via direct linkage or an indirect labeling technique. Labels and methods for labeling antibodies and detecting the binding of antibodies to their targets are well known in the art and any labels and methods may be employed for the present description. Moreover, more than one reagent for detecting the SUV39H2 protein may be included in the kit.

Alternatively, expression of the SUV39H2 protein in a biological sample may be detected and measured using its biological activity as an index. The biological activity can be determined by, for example, measuring the cell proliferating activity due to the expressed the SUV39H2 protein in the biological sample. For example, the cell proliferating activity of a subject derived biological sample can be determined by culturing a cell in the presence of the subject-derived biological sample, and detecting the speed of proliferation, measuring the cell cycle, or measuring the colony forming ability. More than one reagent for detecting the biological activity of the SUV39H2 protein may be included in the kit.

The kit may contain more than one of the aforementioned reagents. Furthermore, the kit may include a solid matrix and reagent for binding a probe against the SUV39H2 mRNA or antibody against the SUV39H2 protein, a medium and container for culturing cells, positive and negative control reagents, and a secondary antibody for detecting an antibody against the SUV39H2 protein. For example, tissue samples obtained from subject suffering from cancer or not may serve as useful control reagents.

A kit may further include other materials desirable from a commercial and user standpoint, including buffers, diluents, filters, needles, syringes, and package inserts (e.g., written, tape, CD-ROM, etc.) with instructions for use. These reagents and such may be contained in a container with a label. Suitable containers include bottles, vials, and test tubes. The containers may be formed from a variety of materials, such as glass or plastic.

As an embodiment, when the reagent is a probe against the SUV39H2 mRNA, the reagent may be immobilized on a solid matrix, such as a porous strip, to form at least one detection site. The measurement or detection region of the porous strip may include a plurality of sites, each containing a nucleic acid (probe). A test strip may also contain sites for negative and/or positive control. Alternatively, control sites may be located on a strip separated from the test strip. Optionally, the different detection sites may contain different amounts of immobilized nucleic acids, i.e., a higher amount in the first detection site and lesser amounts in subsequent sites. Upon the addition of test sample, the number of sites displaying a detectable signal provides a quantitative indication of the amount of the SUV39H2 mRNA present in the sample. The detection sites may be configured in any suitably detectable shape and are typically in the shape of a bar or dot spanning the width of a test strip.

The kit may further include a positive control sample. The positive control sample may be prepared by collecting SUV39H2 positive samples and then those SUV39H2 level are assayed. In one embodiment, the SUV39H2 positive tissue samples may be composed of cancer cells expressing SUV39H2. Such cancer cells include, but are not limited to, cancer selected from the group consisting of lung cancer, cervical cancer, bladder cancer, esophageal cancer, osteosarcoma, prostate cancer and soft tissue tumor. The SUV39H2 level of the positive control sample may be, for example, more than cut off value.

Alternatively, the SUV39H2 positive samples may also be a clinical cancer tissue(s) obtained from a cancer patient(s). Such cancer includes, but are not limited to, lung cancer, cervical cancer, bladder cancer, esophageal cancer, osteosarcoma, prostate cancer and soft tissue tumor. Alternatively, positive control samples may be prepared by determined a cut-off value and preparing a sample containing an amount of an SUV39H2 mRNA or protein more than the cut-off value. Herein, the phrase "cut-off value" refers to the value dividing between a normal range and a cancerous range. For example, one skilled in the art may be determine a cut-off value using a receiver operating characteristic (ROC) curve.

The kit may also or alternatively include an SUV39H2 standard sample providing a cut-off value amount of an SUV39H2 mRNA or polypeptide. The kit may further include a negative control sample, such as a non-SUV39H2 expressing cells or tissue, or a blood sample derived from a subject without cancer. In the context of the present description, the negative control sample may be prepared from non-cancerous cell lines or non-cancerous tissues such as a normal lung tissue(s), cervical tissue(s), bladder tissue(s), esophageal tissue(s), bone tissue(s), prostate tissue(s) and soft tissue(s), or may be prepared by preparing a sample containing an SUV39H2 mRNA or protein less than cut-off value.

### Screening for an Anti-Cancer Substance:

Using the SUV39H2 gene, SUV39H2 polypeptide or functional equivalent thereof, or transcriptional regulatory region of the gene, it is possible to screen for substances that alter the expression of the SUV39H2 gene or the biological activities of the SUV39H2 polypeptide. Such substances may be used as candidate pharmaceuticals for treating or preventing diseases associated with SUV39H2 over-expression such as cancer. Thus, the present invention provides methods of screening for candidate substances for either or both of the treatment or prevention of cancer using the SUV39H2 gene, an SUV39H2 polypeptide or functional equivalent thereof, or a transcriptional regulatory region of the SUV39H2 gene.

Substances isolated and identified by the screening methods of the present invention as suitable candidates are expected to reduce, suppress and/or inhibit the expression of the SUV39H2 gene, or the activity of the translation product of the SUV39H2 gene, and thus, is a candidate for either or both of treating and preventing cancer (in particular, lung cancer, cervical cancer, bladder cancer, esophageal cancer, osteosarcoma, prostate cancer and soft tissue tumor). Namely, the substances screened through the methods of the present invention are deemed to have a clinical benefit and can be further tested for its ability to limit or prevent cancer cell growth in animal models or test subjects.

In the context of the present invention, substances to be identified through the screening methods of the present invention may be any substance or composition including several substances. Furthermore, the test substance exposed to a cell or protein according to the screening methods of the present invention may be a single substance or a combination of substances. When a combination of substances is used in the methods, the substances may be contacted sequentially or simultaneously.

Any test substance, for example, cell extracts, cell culture supernatant, products of fermenting microorganism, extracts from marine organism, plant extracts, purified or crude proteins, peptides, non-peptide substances, synthetic micromolecular substances (including nucleic acid constructs, such as antisense RNA, siRNA, Ribozymes, and aptamer, etc.) and natural substances can be used in the screening methods of the present invention. The test substance of the present invention can be also obtained using any of the numerous approaches in combinatorial library methods known in the art, including (1) biological libraries, (2) spatially addressable parallel solid phase or solution phase libraries, (3) synthetic library methods requiring deconvolution, (4) the "one-bead one-compound" library method and (5) synthetic library methods using affinity chromatography selection. The biological library methods using affinity chromatography selection is limited to peptide libraries, while the other four approaches are applicable to peptide, non-peptide oligomer or small molecule libraries of compounds (Lam, Anticancer Drug Des 1997, 12: 145-67). Examples of methods for the synthesis of molecular libraries can be found in the art (DeWitt et al., Proc Natl Acad Sci USA 1993, 90: 6909-13; Erb et al., Proc Natl Acad Sci USA 1994, 91: 11422-6; Zuckermann et al., J Med Chem 37: 2678-85, 1994; Cho et al., Science 1993, 261: 1303-5; Carell et al., Angew Chem Int Ed Engl 1994, 33: 2059; Carell et al., Angew Chem Int Ed Engl 1994, 33: 2061; Gallop et al., J Med Chem 1994, 37: 1233-51). Libraries of compounds may be presented in solution (see Houghten, Bio/Techniques 1992, 13: 412-21) or on beads (Lam, Nature 1991, 354: 82-4), chips (Fodor, Nature 1993, 364: 555-6), bacteria (US Pat. No. 5,223,409), spores (US Pat. No. 5,571,698; 5,403,484, and 5,223,409), plasmids (Cull et al., Proc Natl Acad Sci USA 1992, 89: 1865-9) or phage (Scott and Smith, Science 1990, 249: 386-90; Devlin, Science 1990, 249: 404-6; Cwirla et al., Proc Natl Acad Sci USA 1990, 87: 6378-82; Felici, J Mol Biol 1991, 222: 301-10; US Pat. Application 2002103360).

A substance in which a part of the structure of the substance screened by the screening methods of the present invention is converted addition, deletion and/or replacement, is included in substances obtained by the screening methods of the present invention.

Furthermore, when the screened test substance is a protein, for obtaining a DNA encoding the protein, either the whole amino acid sequence of the protein may be determined to deduce the nucleic acid sequence coding for the protein, or partial amino acid sequence of the obtained protein may be analyzed to prepare an oligo DNA as a probe based on the sequence, and screen cDNA libraries with the probe to obtain a DNA encoding the protein. The obtained DNA is confirmed it's usefulness in preparing the test substance which is a candidate for treating or preventing cancer, or a post-operative recurrence thereof, or the inhibition of cancer cell growth.

Test substances useful in the screenings described herein include antibodies that specifically bind to the SUV39H2 protein or partial peptides thereof that lack the biological activity of the original proteins in vivo.

Although the construction of test substance libraries is well known in the art, herein below, additional guidance in identifying test substances and construction libraries of such substances for the present screening methods are provided. It is herein revealed that suppression of either of the expression level or biological activity of the SUV39H2 protein leads to suppression of the growth of cancer cells. Therefore, when a substance suppresses the expression and/or activity of SUV39H2, such suppression is indicative of a potential therapeutic effect in a subject. In the context of the present invention, a potential therapeutic effect refers to a clinical benefit with a reasonable expectation. Examples of such clinical benefit include but are not limited to;
(a) reduction in expression of the SUV39H2 gene,
(b) a decrease in size, prevalence, or metastatic potential of the cancer in the subject,
(c) preventing cancers from forming, or
(d) preventing or alleviating a clinical symptom of cancer.

### (i) Molecular modeling:

Construction of test substance libraries is facilitated by knowledge of the molecular structure of compounds known to have the properties sought, and/or the molecular structure of SUV39H2. One approach to preliminary screening of test substances suitable for further evaluation is computer modeling of the interaction between the test substance and its target.

Computer modeling technology allows the visualization of the three-dimensional atomic structure of a selected molecule and the rational design of new compounds that will interact with the molecule. The three-dimensional construct typically depends on data from x-ray crystallographic analysis or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.

An example of the molecular modeling system described generally above includes the CHARMM and QUANTA programs, Polygen Corporation, Waltham, Mass. CHARMM performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as Rotivinen et al. Acta Pharmaceutica Fennica 1988, 97: 159-66; Ripka, New Scientist 1988, 54-8; McKinlay & Rossmann, Annu Rev Pharmacol Toxiciol 1989, 29: 111-22; Perry & Davies, Prog Clin Biol Res 1989, 291: 189-93; Lewis & Dean, Proc R Soc Lond 1989, 236: 125-40, 141-62; and, with respect to a model receptor for nucleic acid components, Askew et al., J Am Chem Soc 1989, 111: 1082-90.

Other computer programs that screen and graphically depict chemicals are available from companies such as BioDesign, Inc., Pasadena, Calif., Allelix, Inc, Mississauga, Ontario, Canada, and Hypercube, Inc., Cambridge, Ontario. See, e.g., DesJarlais et al., J Med Chem 1988, 31: 722-9; Meng et al., J Computer Chem 1992, 13: 505-24; Meng et al., Proteins 1993, 17: 266-78; Shoichet et al., Science 1993, 259: 1445-50.

Once a putative inhibitor has been identified, combinatorial chemistry techniques can be employed to construct any number of variants based on the chemical structure of the identified putative inhibitor, as detailed below. The resulting library of putative inhibitors, or "test substances" may be screened using the methods of the present invention to identify test substances treating or preventing a cancer, such as lung cancer, cervical cancer, bladder cancer, esophageal cancer, osteosarcoma, prostate cancer and soft tissue tumor.

### (ii) Combinatorial chemical synthesis:

Combinatorial libraries of test substances may be produced as part of a rational drug design program involving knowledge of core structures existing in known inhibitors. This approach allows the library to be maintained at a reasonable size, facilitating high throughput screening. Alternatively, simple, particularly short, polymeric molecular libraries may be constructed by simply synthesizing all permutations of the molecular family making up the library. An example of this latter approach would be a library of all peptides of six amino acids in length. Such a peptide library could include every 6 amino acid sequence permutation. This type of library is termed a linear combinatorial chemical library.

Preparation of combinatorial chemical libraries is well known to those of skill in the art, and may be generated by either chemical or biological synthesis. Combinatorial chemical libraries include, but are not limited to, peptide libraries (see, e.g., US Patent 5,010,175; Furka, Int J Pept Prot Res 1991, 37: 487-93; Houghten et al., Nature 1991, 354: 84-6). Other chemistries for generating chemical diversity libraries can also be used. Such chemistries include, but are not limited to: peptides (e.g., PCT Publication No. WO 91/19735), encoded peptides (e.g., WO 93/20242), random bio-oligomers (e.g., WO 92/00091), benzodiazepines (e.g., US Patent 5,288,514), diversomers such as hydantoins, benzodiazepines and dipeptides (DeWitt et al., Proc Natl Acad Sci USA 1993, 90:6909-13), vinylogous polypeptides (Hagihara et al., J Amer Chem Soc 1992, 114: 6568), nonpeptidal peptidomimetics with glucose scaffolding (Hirschmann et al., J Amer Chem Soc 1992, 114: 9217-8), analogous organic syntheses of small compound libraries (Chen et al., J. Amer Chem Soc 1994, 116: 2661), oligocarbamates (Cho et al., Science 1993, 261: 1303), and/or peptidylphosphonates (Campbell et al., J Org Chem 1994, 59: 658), nucleic acid libraries (see Ausubel, Current Protocols in Molecular Biology 1995 supplement; Sambrook et al., Molecular Cloning: A Laboratory Manual, 1989, Cold Spring Harbor Laboratory, New York, USA), peptide nucleic acid libraries (see, e.g., US Patent 5,539,083), antibody libraries (see, e.g., Vaughan et al., Nature Biotechnology 1996, 14(3):309-14 and PCT/US96/10287), carbohydrate libraries (see, e.g., Liang et al., Science 1996, 274: 1520-22; US Patent 5,593,853), and small organic molecule libraries (see, e.g., benzodiazepines, Gordon EM. Curr Opin Biotechnol. 1995 Dec 1;6(6):624-31.; isoprenoids, US Patent 5,569,588; thiazolidinones and metathiazanones, US Patent 5,549,974; pyrrolidines, US Patents 5,525,735 and 5,519,134; morpholino compounds, US Patent 5,506,337; benzodiazepines, 5,288,514, and the like).

Materials and methods for the preparation of combinatorial libraries are commercially available (see, e.g., 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA). In addition, numerous combinatorial libraries are themselves commercially available (see, e.g., ComGenex, Princeton, N.J., Tripos, Inc., St. Louis, MO, 3D Pharmaceuticals, Exton, PA, Martek Biosciences, Columbia, MD, etc.).

### (iii) Other candidates:

Another approach uses recombinant bacteriophage to produce libraries. Using the "phage method" (Scott & Smith, Science 1990, 249: 386-90; Cwirla et al., Proc Natl Acad Sci USA 1990, 87: 6378-82; Devlin et al., Science 1990, 249: 404-6), very large libraries can be constructed (e.g., 10⁶-10⁸ chemical entities). A second approach uses primarily chemical methods, of which the Geysen method (Geysen et al., Molecular Immunology 1986, 23: 709-15; Geysen et al., J Immunologic Method 1987, 102: 259-74); and the method of Fodor et al. (Science 1991, 251: 767-73) are examples. Furka et al. (14th International Congress of Biochemistry 1988, Volume #5, Abstract FR:013; Furka, Int J Peptide Protein Res 1991, 37: 487-93), Houghten (US Patent 4,631,211) and Rutter et al. (US Patent 5,010,175) describe methods to produce a mixture of peptides that can be tested as agonists or antagonists.

Aptamers are macromolecules composed of nucleic acid that bind tightly to a specific molecular target. Tuerk and Gold (Science. 249:505-510 (1990)) discloses SELEX (Systematic Evolution of Ligands by Exponential Enrichment) method for selection of aptamers. In the SELEX method, a large library of nucleic acid molecules (e.g., 10¹⁵ different molecules) can be used for screening.

In addition to the full length of SUV39H2 polypeptide, fragments of the polypeptides may be used for the screening method of the present invention, so long as the fragment utilized retains at least one biological activity of the natural occurring SUV39H2 polypeptide. Such examples of biological activities contemplated by the present invention include cell proliferation promoting activity and methyltransferase activity of the native SUV39H2 polypeptide

SUV39H2 polypeptides or functional equivalent thereof may be further linked to other substances, so long as the resulting polypeptides retain at least one biological activity of the natural occurring SUV39H2 polypeptide. Useful substances include: peptides, lipids, sugar and sugar chains, acetyl groups, natural and synthetic polymers, etc. These kinds of modifications may be performed to confer additional functions or to stabilize the polypeptide and fragments.

### (I) General Screening Methods:

Using the SUV39H2 gene, the SUV39H2 polypeptide or a transcriptional regulatory region of the SUV39H2 gene, substances that alter the expression of the SUV39H2 gene or the biological activity of the SUV39H2 polypeptide can be identified. As demonstrated herein, the SUV39H2 gene is over-expressed in cancer, and involved in cancer cell growth and/or survival. Therefore, substances that alter the expression of the SUV39H2 gene or the biological activity of the SUV39H2 polypeptide can be candidate therapeutics for cancer.

Antagonists that bind to the SUV39H2 polypeptide may reduce, suppress or inhibit the biological activity to mediate cell proliferation of cancer. As such, they are potential candidates for treating the cancer. Therefore, the present invention provides a method for identifying candidates for either or both of treating and preventing cancer expressing the SUV39H2 gene, by identifying substances that bind to the SUV39H2 polypeptide.

In some embodiments, the SUV39H2 polypeptide may be immobilized on an appropriate support. Examples of supports that can be used for binding proteins include, for example, insoluble polysaccharides, for example, agarose, cellulose and dextran; and synthetic resins, for example, polyacrylamide, polystyrene and silicon; for example, commercial available beads and plates (e.g., multi-well plates, biosensor chip, etc.) prepared from the above materials can be used. When using beads, they can be filled into a column. Alternatively, the use of magnetic beads is also known in the art, and enables one to readily isolate proteins bound on the beads via magnetism.

The binding of a protein to a support can be conducted according to routine methods, for example, chemical bonding and physical adsorption, for example. Alternatively, a protein can be bound to a support via antibodies that specifically recognize the protein. Moreover, binding of a protein to a support can be also conducted by means of avidin and biotin.

The immobilized polypeptide can be exposed to synthetic chemical compounds, natural substance banks, or a random phage peptide display library, and the methods of screening using high-throughput based on combinatorial chemistry techniques (Wrighton et al., Science 273: 458-63 (1996); Verdine, Nature 384: 11-3 (1996)) to isolate not only proteins but also chemical compounds that bind to the protein (including agonist and antagonist) are well known to one skilled in the art.

Furthermore, in the screening method of the present invention, substances that suppress the expression level of the SUV39H2 gene can be also identified as candidate therapeutics for cancer. The expression level of a polypeptide or functional equivalent thereof can be detected according to any method known in the art. For example, a reporter assay can be used. Suitable reporter genes and host cells are well known in the art. The reporter construct required for the screening can be prepared by using the transcriptional regulatory region of the SUV39H2 gene. When the transcriptional regulatory region of the gene has been known to those skilled in the art, a reporter construct can be prepared by using the previous sequence information. When the transcriptional regulatory region remains unidentified, a nucleotide segment containing the transcriptional regulatory region can be isolated from a genome library based on the nucleotide sequence information of the gene. Specifically, the reporter construct required for the screening can be prepared by connecting reporter gene sequence to the transcriptional regulatory region of the SUV39H2 gene. The transcriptional regulatory region of the SUV39H2 gene is the region from a start codon to at least 500bp upstream, for example, 1000bp, for example, 5000 or 10000bp upstream. A nucleotide segment containing the transcriptional regulatory region can be isolated from a genome library or can be propagated by PCR. Methods for identifying a transcriptional regulatory region, and also assay protocol are well known (Sambrook and Russell, Molecular Cloning: A Laboratory Manual, 3rd Ed., Chapter 17, 2001, Cold Springs Harbor Laboratory Press).

Furthermore, in the screening methods of the present invention, substances that inhibit a biological activity of the SUV39H2 polypeptide can be also identified as candidate therapeutics for cancer.

Various low-throughput and high-throughput enzyme assay formats are known in the art and can be readily adapted for detection or measuring of a biological activity of the SUV39H2 polypeptide. For high-throughput assays, a substrate can conveniently be immobilized on a solid support. Following the reaction, the substrate converted by the polypeptide can be detected on the solid support by the methods described above. Alternatively, the contact step can be performed in solution, after which a substrate can be immobilized on a solid support, and the substrate converted by the polypeptide can be detected. To facilitate such assays, the solid support can be coated with streptavidin and the substrate labeled with biotin, or the solid support can be coated with antibodies against the substrate. The skilled person can determine suitable assay formats depending on the desired throughput capacity of the screen.

The assays of the present invention are also suitable for automated procedures that facilitate high-throughput screening. A number of well-known robotic systems have been developed for solution phase chemistries. These systems include automated workstations like the automated synthesis apparatus developed by Takeda Chemical Industries, Ltd. (Osaka, Japan) and many robotic systems utilizing robotic arms (Zymate II, Zymark Corporation, Hopkinton, Mass.; Orca, Hewlett Packard, Palo Alto, Calif.), which mimic the manual synthetic operations performed by a chemist. Any of the above devices are suitable for use with the present invention. The nature and implementation of modifications to these devices (if any) so that they can operate as discussed herein will be apparent to persons skilled in the relevant art. In addition, numerous combinatorial libraries are themselves commercially available (see, e.g., ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, etc.).

In the present invention, it is revealed that suppression of the expression level of the SUV39H2 gene or biological activity of the SUV39H2 polypeptide lead to suppression of the growth of cancer cells. Therefore, when a substance suppresses the expression of the SUV39H2 gene or the activity of the SUV39H2 polypeptide, the suppression is indicative of a potential therapeutic effect in a subject. In the present invention, a potential therapeutic effect refers to a clinical benefit with a reasonable expectation. In the present invention, such clinical benefit includes;
(a) reduction in expression of the SUV39H2 gene,
(b) decrease in size, prevalence, or metastatic potential of the cancer in a subject,
(c) preventing cancers from forming, or
(d) preventing or alleviating a clinical symptom of cancer.

### (II) Screening for a substance that binds the SUV39H2 polypeptide:

In the course of present invention, the SUV39H2 gene is revealed to be over-expressed in various cancers such as lung cancer, cervical cancer, bladder cancer, esophageal cancer, osteosarcoma, prostate cancer and soft tissue tumor, in spite of no or little expression in normal organs without testis (Figures 1, 2, 3, 6, 9, 10 and Tables 3, 4, 5, 6). Therefore, using the SUV39H2 polypeptide, the present invention provides a method of screening for a substance that binds to the SUV39H2 polypeptide. Due to the expression of the SUV39H2 gene in cancer, a substance that binds to the SUV39H2 polypeptide is expected to suppress the proliferation of cancer cells, and thus be useful for either or both of treating and preventing cancer. Therefore, the present invention also provides a method of screening for a candidate substance that suppresses the proliferation of cancer cells, and a method of screening for a candidate substance for either or both of treating and preventing cancer using the SUV39H2 polypeptide. Specially, in an embodiment, the screening method of the present invention includes the steps of:
(a) contacting a test substance with an SUV39H2 polypeptide or functional equivalent thereof;
(b) detecting the binding activity between the polypeptide or functional equivalent thereof and the test substance; and
(c) selecting the test substance that binds to the polypeptide or functional equivalent.

According to the present invention, the therapeutic effect of the test substance on inhibiting cancer cell growth and treating or preventing cancer associated with over-expression of the SUV39H2 may be evaluated or estimated. Therefore, the present invention also provides a method of screening for a candidate substance for inhibiting cancer cell growth or either or both of treating and preventing cancer, which includes the steps of:
(a) contacting a test substance with the SUV39H2 polypeptide or a functional equivalent thereof;
(b) detecting the binding between the polypeptide (or functional equivalent) and the test substance; and
(c) correlating the binding of (b) with the potential therapeutic effect of the test substance.

In the context of the present invention, the therapeutic effect may be correlated with the binding properties of the test substance. For example, when the test substance binds to the polypeptide (or functional equivalent), the test substance may identified or selected as the candidate substance having the therapeutic effect. Alternatively, when the test substance does not bind to the polypeptide (or functional equivalent), the test substance may identified as the substance having no significant therapeutic effect.

Alternatively, according to the present invention, the potential therapeutic effect of a test substance on either or both of treating and preventing a cancer associated with over-expression of the SUV39H2 can be evaluated or estimated by:
(a) contacting a test substance with an SUV39H2 polypeptide or functional equivalent thereof;
(b) detecting the binding activity between the polypeptide or functional equivalent and the test substance; and
(c) correlating the potential therapeutic effect and the test substance, wherein the potential therapeutic effect is shown, when a substance binds to the polypeptide or functional equivalent.

The method of the present invention will be described in more detail below.

The SUV39H2 polypeptide to be used for the screening method of the present invention may be a recombinant polypeptide or a protein derived from the nature or a partial peptide thereof. The polypeptide to be contacted with a test substance can be, for example, a purified polypeptide, a soluble protein, a form bound to a carrier or a fusion protein fused with other polypeptides. In preferred embodiments, the polypeptide is isolated from cells expressing SUV39H2, or chemically synthesized to be contacted with a test substance in vitro.

As a method of screening for proteins that bind to the SUV39H2 polypeptide, many methods well known by a person skilled in the art can be used. Such a screening can be conducted by, for example, the immunoprecipitation method, specifically, in the following manner. The gene encoding the SUV39H2 polypeptide is expressed in host (e.g., animal) cells and so on by inserting the gene to an expression vector for foreign genes, such as pSV2neo, pcDNA I, pcDNA3.1, pCAGGS and pCD8.

The promoter to be used for the expression may be any promoter that can be used commonly and include, for example, the SV40 early promoter (Rigby in Williamson (ed.), Genetic Engineering, vol. 3. Academic Press, London, 83-141 (1982)), the EF-alpha promoter (Kim et al., Gene 91: 217-23 (1990)), the CAG promoter (Niwa et al., Gene 108: 193 (1991)), the RSV LTR promoter (Cullen, Methods in Enzymology 152: 684-704 (1987)) the SR alpha promoter (Takebe et al., Mol Cell Biol 8: 466 (1988)), the CMV immediate early promoter (Seed and Aruffo, Proc Natl Acad Sci USA 84: 3365-9 (1987)), the SV40 late promoter (Gheysen and Fiers, J Mol Appl Genet 1: 385-94 (1982)), the Adenovirus late promoter (Kaufman et al., Mol Cell Biol 9: 946 (1989)), the HSV TK promoter and so on.

The introduction of the gene into host cells to express a foreign gene can be performed according to any methods, for example, the electroporation method (Chu et al., Nucleic Acids Res 15: 1311-26 (1987)), the calcium phosphate method (Chen and Okayama, Mol Cell Biol 7: 2745-52 (1987)), the DEAE dextran method (Lopata et al., Nucleic Acids Res 12: 5707-17 (1984); Sussman and Milman, Mol Cell Biol 4: 1641-3 (1984)), the Lipofectin method (Derijard B., Cell 76: 1025-37 (1994); Lamb et al., Nature Genetics 5: 22-30 (1993): Rabindran et al., Science 259: 230-4 (1993)) and so on.

The SUV39H2 polypeptide can be expressed as a fusion protein including a recognition site (epitope) of a monoclonal antibody by introducing the epitope of the monoclonal antibody, whose specificity has been revealed, to the N- or C- terminus of the polypeptide. A commercially available epitope-antibody system can be used (Experimental Medicine 13: 85-90 (1995)). Vectors that can express a fusion protein with, for example, beta-galactosidase, maltose binding protein, glutathione S-transferase, green fluorescence protein (GFP) and so on by the use of its multiple cloning sites are commercially available. A fusion protein prepared by introducing only small epitopes composed of several to a dozen amino acids so as not to change the property of the SUV39H2 polypeptide by the fusion is also provided herein. Epitopes, such as polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage) and such, and monoclonal antibodies recognizing them can be used as the epitope-antibody system for screening proteins binding to the SUV39H2 polypeptide (Experimental Medicine 13: 85-90 (1995)).

In the context of immunoprecipitation, an immune complex is formed by adding these antibodies to cell lysate prepared using an appropriate detergent. The immune complex is composed of the SUV39H2 polypeptide, a polypeptide including the binding ability with the SUV39H2 polypeptide, and an antibody. Immunoprecipitation can be also conducted using antibodies against the SUV39H2 polypeptide, besides using antibodies against the above epitopes, which antibodies can be prepared as described above. An immune complex can be precipitated, for example, by Protein A sepharose or Protein G sepharose when the antibody is a mouse IgG antibody. If the SUV39H2 polypeptide is prepared as a fusion protein with an epitope, such as GST, an immune complex can be formed in the same manner as in the use of the antibody against the SUV39H2 polypeptide, using a substance specifically binding to these epitopes, such as glutathione-Sepharose 4B.

Immunoprecipitation can be performed by following or according to, for example, the methods in the literature (Harlow and Lane, Antibodies, 511-52, Cold Spring Harbor Laboratory publications, New York (1988)).

SDS-PAGE is commonly used for analysis of immunoprecipitated proteins and the bound protein can be analyzed by the molecular weight of the protein using gels with an appropriate concentration. Since the protein bound to the SUV39H2 polypeptide is difficult to detect by a common staining method, such as Coomassie staining or silver staining, the detection sensitivity for the protein can be improved by culturing cells in culture medium containing radioactive isotope, ³⁵S-methionine or ³⁵S-cysteine, labeling proteins in the cells, and detecting the proteins. The target protein can be purified directly from the SDS-polyacrylamide gel and its sequence can be determined, when the molecular weight of a protein has been revealed.

Alternatively, West-Western blotting analysis (Skolnik et al., Cell 65: 83-90 (1991)) can be used to screen for proteins binding to the SUV39H2 polypeptide. Specifically, a protein binding to the SUV39H2 polypeptide can be obtained by preparing a cDNA library from cultured cells expected to express a protein binding to the SUV39H2 polypeptide using a phage vector (e.g., ZAP), expressing the protein on LB-agarose, fixing the protein expressed on a filter, reacting the purified and labeled SUV39H2 polypeptide with the above filter, and detecting the plaques expressing proteins bound to the SUV39H2 polypeptide according to the label. The SUV39H2 polypeptide may be labeled by utilizing the binding between biotin and avidin, or by utilizing an antibody that specifically binds to the SUV39H2 polypeptide, or a peptide or polypeptide (for example, GST) that is fused to the SUV39H2 polypeptide. Methods using radioisotope or fluorescence and such may be also used.

The terms "label" and "detectable label" are used herein to refer to any composition detectable by spectroscopic, photochemical, biochemical, immunochemical, electrical, optical or chemical means. Such labels include biotin for staining with labeled streptavidin conjugate, magnetic beads (e.g., DYNABEADS™), fluorescent dyes (e.g., fluorescein, Texas red, rhodamine, green fluorescent protein, and the like), radiolabels (e.g., ³H, ¹²⁵I, .³⁵S, ¹⁴C, or ³²P), enzymes (e.g., horse radish peroxidase, alkaline phosphatase and others commonly used in an ELISA), and calorimetric labels for example colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,275,149; and 4,366,241. Means of detecting such labels are well known to those of skill in the art. Thus, for example, radiolabels can be detected using photographic film or scintillation counters, fluorescent markers can be detected using a photodetector to detect emitted light. Enzymatic labels are typically detected by providing the enzyme with a substrate and detecting, the reaction product produced by the action of the enzyme on the substrate, and calorimetric labels are detected by simply visualizing the colored label.

Alternatively, in another embodiment, the screening method of the present invention may utilize a two-hybrid cell system ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman, Cell 68: 597-612 (1992)", "Fields and Sternglanz, Trends Genet 10: 286-92 (1994)").

In the two-hybrid system, an SUV39H2 polypeptide is fused to an SRF-binding region or GAL4-binding region and expressed in yeast cells. A cDNA library is prepared from cells expected to express a protein binding to the SUV39H2 polypeptide, such that the library, when expressed, is fused to the VP16 or GAL4 transcriptional activation region. The cDNA library is then introduced into the above yeast cells and the cDNA derived from the library is isolated from the positive clones detected (when a protein binding to the polypeptide of the present invention is expressed in yeast cells, the binding of the two activates a reporter gene, making positive clones detectable). A protein encoded by the cDNA can be prepared by introducing the cDNA isolated above to E. coli and expressing the protein. Examples of suitable reporter genes include, but are not limited to, the Ade2 gene, lacZ gene, CAT gene, luciferase gene and such can be used in addition to the HIS3 gene.

A substance binding to the SUV39H2 polypeptide can also be screened using affinity chromatography. For example, the SUV39H2 polypeptide may be immobilized on a carrier of an affinity column, and a test substance, containing a protein capable of binding to the polypeptide of the present invention, is applied to the column. A test substance herein may be, for example, cell extracts, cell lysates, etc. After loading the test substance, the column is washed, and substances bound to the polypeptide of the present invention can be prepared. When the test substance is a protein, the amino acid sequence of the obtained protein is analyzed, an oligo DNA is synthesized based on the sequence, and cDNA libraries are screened using the oligo DNA as a probe to obtain a DNA encoding the protein.

A biosensor using the surface plasmon resonance phenomenon may be used as a mean for detecting or quantifying the bound substance in the present invention. When such a biosensor is used, the interaction between the SUV39H2 polypeptide and a test substance can be observed real-time as a surface plasmon resonance signal, using only a minute amount of polypeptide and without labeling (for example, BIAcore, Pharmacia). Therefore, it is possible to evaluate the binding between the polypeptide of the present invention and a test substance using a biosensor such as BIAcore.

The methods of screening for molecules that bind when the immobilized SUV39H2 polypeptide is exposed to synthetic chemical compounds, or natural substance banks or a random phage peptide display library, and the methods of screening using high-throughput based on combinatorial chemistry techniques (Wrighton et al., Science 273: 458-64 (1996); Verdine, Nature 384: 11-13 (1996); Hogan, Nature 384: 17-9 (1996)) to isolate not only proteins but chemical compounds that bind to the SUV39H2 protein (including agonist and antagonist) are well known to those skilled in the art.

In the present invention, it is revealed that suppressing the expression level of the SUV39H2 gene, reduces cell growth. Thus, by screening for candidate substances that bind to the SUV39H2 polypeptide, candidate substances that have the potential to treat or prevent cancer can be identified. Potential of these candidate substances to treat or prevent cancer may be evaluated by second and/or further screening to identify therapeutic agent for cancer. For example, when a substance binding to the SUV39H2 polypeptide inhibits activities of cancer, it may be concluded that such substance has SUV39H2 specific therapeutic effect.

### (III) Screening for a substance that suppresses the biological activity of the SUV39H2 polypeptide:

In the course of the present invention, the SUV39H2 gene is revealed to be highly over-expressed in various cancers including lung cancer, cervical cancer, bladder cancer, esophageal cancer, osteosarcoma, prostate cancer and soft tissue tumor.

Further, the suppression of the SUV39H2 gene by small interfering RNA (siRNA) resulted in growth inhibition and/or cell death of cancer cells. Accordingly, it is clear that the SUV39H2 polypeptide is involved in cancer cell survival, and thus, substances that inhibit a biological activity of the SUV39H2 polypeptide may serve as suitable candidate substances for cancer therapy.

Thus, the present invention also provides a method of screening for a candidate substance for either or both of the treatment and prevention of an SUV39H2-associated cancer using a biological activity of the SUV39H2 polypeptide as an index.

Exemplary SUV39H2-associated cancers include lung cancer, cervical cancer, bladder cancer, esophageal cancer, osteosarcoma, prostate cancer and soft tissue tumor.

Specifically, the present invention provides the following methods:
A method of screening for a candidate substance for either or both of treating and preventing cancer, including steps of:
   (a) contacting a test substance with an SUV39H2 polynucleotide ;
   (b) detecting a biological activity of the SUV39H2 polypeptide of step (a);
   (c) comparing the biological activity detected in step (b) with that detected in the absence of the test substance;
   (d) selecting the test substance that reduces or inhibits the biological activity of the SUV39H2 polypeptide or functional equivalent thereof.

In the context of the present invention, the therapeutic effect of the test substance on suppressing the biological activity (e.g., the cell-proliferation promoting activity or the methyltransferase activity) of SUV39H2 polypeptide or a candidate substance for either or both of treating and preventing cancer may be evaluated. Therefore, the present invention also provides a method of screening for a substance for suppressing the biological activity of the SUV39H2 polypeptide, or a candidate substance for either or both of the treatment and prevention of an SUV39H2-associated cancer, using the SUV39H2 polypeptide, including the following steps:
(a) contacting a test substance with the SUV39H2 polypeptide ; and
(b) detecting the biological activity of the polypeptide or functional equivalent of step (a), and
(c) correlating the biological activity of (b) with the therapeutic effect of the test substance.

In the context of the present invention, the therapeutic effect may be correlated with the biological activity of the SUV39H2 polypeptide. For example, when the test substance suppresses or inhibits the biological activity of the SUV39H2 polypeptide or a functional equivalent thereof as compared to a level detected in the absence of the test substance, the test substance may identified or selected as the candidate substance having the therapeutic effect. Alternatively, when the test substance does not suppress or inhibit the biological activity of the SUV39H2 polypeptide as compared to a level detected in the absence of the test substance, the test substance may be identified as the substance having no significant therapeutic effect.

Alternatively, in some embodiments, the present invention provides a method for evaluating or estimating a therapeutic effect of a test substance on either or both of treating and preventing cancer or inhibiting cancer associated with over-expression of the SUV39H2 gene, the method including steps of:
(a) contacting a test substance with an SUV39H2 polypeptide;
(b) detecting the biological activity of the polypeptide or functional equivalent thereof of step (a); and
(c) correlating the potential therapeutic effect and the test substance, wherein the potential therapeutic effect is shown, when a substance suppresses the biological activity of the SUV39H2 polypeptide as compared to the biological activity of said polypeptide detected in the absence of the test substance. In the context of expression, binding and biological activity, the term "suppress" is used interchangeably with the terms "reduce" and "inhibit" to encompass effects ranging from partial to full. Accordingly, the phrase "suppress the biological activity" as defined herein refers to at least 10% suppression of the biological activity of SUV39H2 in comparison with in absence of the substance, more preferably at least 25%, 50% or 75% suppression and most preferably at 90% suppression.

As above, examples of such SUV39H2-associated cancers include lung cancer, cervical cancer, bladder cancer, esophageal cancer, osteosarcoma, prostate cancer and soft tissue tumor.

In the context of the present invention, the therapeutic effect may be correlated with the biological activity of the SUV39H2 polypeptide. For example, when the test substance suppresses or inhibits the biological activity of the SUV39H2 polypeptide as compared to a level detected in the absence of the test substance, the test substance may identified or selected as the candidate substance having the therapeutic effect. Alternatively, when the test substance does not suppress or inhibit the biological activity of the SUV39H2 polypeptide as compared to a level detected in the absence of the test substance, the test substance may identified as the substance having no significant therapeutic effect.

The method of the present invention will be described in more detail below.

Any polypeptide can be used for the screening methods of the present invention, so long as it possesses the biological activity of the SUV39H2 polypeptide. Examples of such biological activities include cell proliferation promoting activity and methyltransferase activity of the SUV39H2 polypeptide. For example, an SUV39H2 polypeptide can be used and polypeptides functionally equivalent to the SUV39H2 polypeptide can also be used. Such polypeptides may be expressed endogenously or exogenously by cells. For details of the functional equivalent of the SUV39H2 polypeptide, see the item "Genes and Polypeptides".

Substances isolated by the screening methods of the present invention are candidate antagonists (inhibitors) of the SUV39H2 polypeptide. The term "antagonist" refers to molecules that inhibit the function of the polypeptide by binding thereto. The term also refers to molecules that reduce or inhibit expression of the gene encoding the SUV39H2 polypeptide. Moreover, a substance isolated by the screening methods of the present invention is a candidate for substances which inhibit the in vivo interaction of the SUV39H2 polypeptide with molecules (including DNAs and proteins).

When the biological activity to be detected in the method of the present invention is cell proliferation promoting activity, it can be detected, for example, by preparing cells which express the SUV39H2 polypeptide, culturing the cells in the presence of a test substance, and determining the speed of cell proliferation, measuring the cell cycle and such, as well as by measuring cell survival or colony forming activity, e.g. by MTT assay, colony formation assay or FACS.

Substances that reduce the speed of proliferation of the cells expressing the SUV39H2 gene are selected as candidate substance for either or both of treating and preventing cancer. In some embodiments, cells expressing SUV39H2 gene are isolated and cultured cells exogenously or endogenously expressing SUV39H2 gene in vitro.

More specifically, the method includes the steps of:
(a) contacting a test substance with cells expressing the SUV39H2 gene;
(b) measuring cell proliferation promoting activity; and
(c) selecting the test substance that reduces the cell proliferation promoting activity in the comparison with the cell proliferation promoting activity in the absence of the test substance.

In preferable embodiments, the method of the present invention may further include the steps of:
(d) selecting the test substance that have substantially no effect to the cells expressing little or no detectable the SUV39H2 gene.

When the biological activity to be detected in the method of the present invention is methyltransferase activity, the methyltransferase activity can be determined by contacting an SUV39H2 polypeptide with a substrate (e.g., histone H3 or fragment thereof including Lysine 9 (e.g., SEQ ID NO: 31)) and a co-factor (e.g., S-adenosyl-L-methionine) under a condition suitable for methylation of the substrate and detecting the methylation level of the substrate.

In the context of the present invention, the methyltransferase activity of an SUV39H2 polypeptide can be determined by methods known in the art (See, for example,WO01/094,620, Hamamoto et al, Nature 2004;6(8):731-740, O'carraol et al, Mol Cell Biol 2000;20(24):9423-9433, Rea et al, Nature 2000;406:593-599).

For example, the SUV39H2 polypeptide and a substrate can be incubated with a labeled methyl donor, under suitable assay conditions. A histone H3 peptides (i.e., histone H3 or fragment thereof), and S-adenosyl-[methyl-¹⁴C]-L-methionine, or S-adenosyl-[methyl-³H]-L-methionine preferably can be used as the substrate and methyl donor, respectively. Transfer of the radiolabel to the histone H3 peptides can be detected, for example, by SDS-PAGE electrophoresis and fluorography. Alternatively, following the reaction, the histone H3 peptides can be separated from the methyl donor by filtration, and the amount of radiolabel retained on the filter quantitated by scintillation counting. Other suitable labels that can be attached to methyl donors, such as chromogenic and fluorescent labels, and methods of detecting transfer of these labels to histones and histone peptides, are known in the art. An example of the methyltransferase assay will be described in "Example 5: Screening for inhibitors of methyltransferase activity of SUV39H2".

Alternatively, the methyltransferase activity of the SUV39H2 polypeptide can be determined using an unlabeled methyl donor (e.g., S-adenosyl-L-methionine) and reagents that selectively recognize methylated substrate (e.g., histone H3 peptide). For example, after incubation of the SUV39H2 polypeptide, substrate to be methylated and methyl donor, under the condition capable of methylation of the substrate, methylated substrate can be detected by immunological method. Any immunological techniques using an antibody recognizing methylated substrate can be used for the detection. For example, an antibody against methylated histone is commercially available (abcam Ltd.). ELISA or Immunoblotting with antibodies recognizing methylated histone can be used for the present invention.

In the context of the present invention, the histone H3 or fragment thereof (e.g., SEQ ID NO: 31) can be preferably used as a substrate to be methylated by the SUV39H2 polypeptide. The histone H3 fragment to be used as a substrate preferably retains lysine 9. Such histone H3 fragment is composed of preferably at least 10 amino acid residues, more preferably at least 15 amino acid residues, and further more preferably at least 20 amino acid residues. An example of such histone H3 fragment includes a peptide having amino acid sequence of SEQ ID NO: 31. Alternatively, a modified peptide of the histone H3 or fragment thereof may be used for which the methyltransferase has increased affinity/activity. Such peptides can be designed by exchanging and/or adding and/or deleting amino acids and testing the substrate in serial experiments for methyltransferase assay using the SUV39H2 polypeptide.

In the context of the present description, any functional equivalent of the SUV39H2 polypeptide can be used so long as it retains the methyltransferase activity of the original (native, wild-type) SUV39H2 polypeptide. To that end,, the functional equivalent of the SUV39H2 polypeptide preferably retains a SET-domain of the SUV39H2 polypeptide (see, the item "Gene and polypeptide"). An example of such functional equivalent includes the polypeptide having an amino acid sequence of SEQ ID NO: 32.

The SUV39H2 polypeptide or functional equivalent thereof may be expressed as a fusion protein including a recognition site (epitope) of a monoclonal antibody by introducing the epitope of the monoclonal antibody, whose specificity has been revealed, to the N- or C- terminus of the polypeptide. A commercially available epitope-antibody system can be used (Experimental Medicine 13: 85-90 (1995)). Vectors which can express a fusion protein with, for example, beta-galactosidase, maltose binding protein, glutathione S-transferase (GST), green fluorescence protein (GFP) and so on by the use of its multiple cloning sites are commercially available. Also, a fusion protein prepared by introducing only small epitopes consisting of several to a dozen amino acids so as not to change the property of the SUV39H2 polypeptide by the fusion is also reported. Epitopes, such as polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage) and such.

The present description contemplates the use of an agent that enhances the methylation of the substance. A preferred enhancing agent for the methylation is SAHH or a functional equivalent thereof. Such agents enhance the methylation of the substance and thereby enable determination of the methyltransferase activity with higher sensitivity. Accordingly, SUV39H2 may be contacted with substrate and cofactor under the existence of the enhancing agent.

Methyltransferase activity can be also be detected by preparing cells that express the SUV39H2 polypeptide, culturing the cells in the presence of a test substance, and determining the methylation level of a histone, for example, by using an antibody that specifically binds to methylated histone.

More specifically, the method includes the step of:
[1] contacting a test substance with cells expressing the SUV39H2 gene;
[2] detecting a methylation level of histone H3 lysine 9 (H3K9) ; and
[3] selecting the test substance that reduces the methylation level in the comparison with the methylation level in the absence of the test substance.

As noted above, the phrase "suppress the biological activity" is defined herein as preferably at least 10% suppression of the biological activity of the SUV39H2 polypeptide in comparison with in absence of the substance, more preferably at least 25%, 50% or 75% suppression and most preferably at 90% suppression. Accordingly, a test substance may be characterized as "reducing the methylation level" if it provides a reduction on the order of 10%, more preferably at least 25%, 50% or 75% reduction and most preferably at 90% reduction.

In the preferred embodiments, control cells that do not express the SUV39H2 gene are used. Accordingly, the present invention also provides a method of screening for a candidate substance for inhibiting the cell growth or a candidate substance for either or both of treating and preventing SUV39H2 associating cancer, using the SUV39H2 polypeptide or functional equivalents thereof including the steps as follows:
(a) culturing cells which express an SUV39H2 polypeptide or a functional equivalent thereof, and control cells that do not express an SUV39H2 polypeptide or a functional equivalent thereof in the presence of the test substance;
(b) detecting the biological activity (cell-proliferation or methyltransferase activity) of the cells which express the SUV39H2 polypeptide or functional equivalent thereof and control cells; and
(c) selecting the test substance that inhibits the biological activity (cell proliferation or methyltransferase activity) in the cells that express the SUV39H2 polypeptide or functional equivalent thereof as compared to biological activity (cell-proliferation promoting activity or methyltransferase activity) detected in the control cells and in the absence of said test substance.

### (IV) Screening for a substance altering the expression of the SUV39H2 gene

The present description also provides a method of screening for a substance that inhibits the expression of the SUV39H2 gene. A substance that inhibits the expression of the SUV39H2 gene is expected to suppress the proliferation of cancer cells (e.g., lung cancer, cervical cancer, bladder cancer, esophageal cancer, osteosarcoma, prostate cancer or soft tissue tumor), and thus may be useful for either or both of treating and preventing cancer (e.g., lung cancer, cervical cancer, bladder cancer, esophageal cancer, osteosarcoma, prostate cancer or soft tissue tumor). Therefore, the present description also provides a method of screening for a substance that suppresses the proliferation of cancer cells over-expressing the SUV39H2 gene, such as lung cancer, cervical cancer, bladder cancer, esophageal cancer, osteosarcoma, prostate cancer and soft tissue tumor cells and a method of screening for a candidate substance for either or both of treating and preventing cancer associating with SUV39H2 over-expression such as lung cancer, cervical cancer, bladder cancer, esophageal cancer, osteosarcoma, prostate cancer and soft tissue tumor.

In the context of the present description, such screening method may include, for example, the following steps:
(a) contacting a test substance with a cell expressing the SUV39H2 gene;
(b) detecting the expression level of the SUV39H2 gene in the cell; and
(c) selecting the test substance that reduces the expression level of the SUV39H2 gene in comparison with the expression level detected in the absence of the test substance.

Furthermore, the present description provides a method of evaluating or estimating therapeutic effect of a test substance on suppresses the proliferation of cancer cells, or either or both of treating and preventing cancer, the method includes steps of:
(a) contacting a substance with a cell expressing the SUV39H2 gene;
(b) detecting the expression level of the SUV39H2 gene ; and
(c) correlating the potential therapeutic effect of the test substance with the expression level detected in step (b), wherein the potential therapeutic effect is shown, when the test substance reduces the expression level of the SUV39H2 gene in comparison with the expression level detected in absence of the test substance.

In the context of the present description, the therapeutic effect may be correlated with the expression level of the SUV39H2 gene. For example, when the test substance reduces the expression level of the SUV39H2 gene as compared to a level detected in the absence of the test substance, the test substance may identified or selected as the candidate substance having the therapeutic effect. Alternatively, when the test substance does not reduce the expression level of the SUV39H2 gene as compared to a level detected in the absence of the test substance, the test substance may identified as the substance having no significant therapeutic effect.

Further, in the course of the present description, the downstream genes of the SUV39H2 gene affected by the knockdown of SUV39H2 were identified. Table 8 indicates the list of genes down-regulated in SW780 and A549 cells transfected with SUV39H2 siRNA . Table 7 indicates the list of genes up-regulated in SW780 and A549 cells transfected with SUV39H2 siRNA. The expression level of these downstream genes can be used as indexes of the expression level of the SUV39H2 gene.

Therefore, the present description also provides the method of screening for a candidate substance for either or both of treating and preventing cancer associating with SUV39H2 over-expression (e.g., lung cancer, cervical cancer, bladder cancer, esophageal cancer, osteosarcoma, prostate canter or soft tissue tumor) or preventing proliferation of cancer cells over-expressing SUV39H2 (e.g., lung cancer, cervical cancer, bladder cancer, esophageal cancer, osteosarcoma, prostate canter or soft tissue tumor cells), the method including steps of:
(a) contacting a test substance with a cell expressing the SUV39H2 gene and a downstream gene of the SUV39H2 gene selected from the genes shown in Table 7 and Table 8;
(b) detecting the expression level of the downstream gene; and
(c) selecting the test substance that alters the expression level of the downstream gene in comparison with the expression level detected in the absence of the test substance.

More specifically, when a downstream gene is a gene selected from among the genes shown in Table 8, test substances that reduces its expression level should be selected as a candidate substance. On the other hand, when a downstream gene is a gene selected from among the genes shown in Table 7, test substances that increases its expression level should be selected as a candidate substance. Therefore, in preferred embodiments, the present screening method includes the steps of:
(a) contacting a test substance with a cell expressing the SUV39H2 gene and a downstream gene of the SUV39H2 gene selected from the genes shown in Table 8;
(b) detecting the expression level of the downstream gene; and
(c) selecting the test substance that reduces the expression level of the downstream gene in comparison with the expression level detected in the absence of the test substance.

Alternatively, in some embodiments, the present description also provides a method for evaluating or estimating a therapeutic effect of a candidate substance for either or both of treating and preventing cancer associating with SUV39H2 over-expression, or preventing proliferation of a cancer cell(s) over-expressing SUV39H2, the method including steps of:
(a) contacting a test substance with a cell expressing the SUV39H2 gene and a downstream gene of the SUV39H2 gene selected from the genes shown in Table 8;
(b) detecting the expression level of the downstream gene; and;
(c) correlating the potential therapeutic effect and the test substance, wherein the potential therapeutic effect is shown, when a substance reduces the expression level of the downstream gene as compared to a control.

Alternatively, the present screening method may include the steps of:
(a) contacting a test substance with a cell expressing the SUV39H2 gene and a downstream gene of the SUV39H2 gene selected from the genes shown in Table 7;
(b) detecting the expression level of the downstream gene; and
(c) selecting the test substance that increases the expression level of the downstream gene in comparison with the expression level detected in the absence of the test substance.

Alternatively, in some embodiments, the present description also provides a method for evaluating or estimating a therapeutic effect of a candidate substance for either or both of treating and preventing cancer associating with SUV39H2 over-expression, or preventing proliferation of a cancer cell(s) over-expressing SUV39H2, the method including steps of:
(a) contacting a test substance with a cell expressing the SUV39H2 gene and a downstream gene of the SUV39H2 gene selected from the genes shown in Table 7;
(b) detecting the expression level of the downstream gene; and
(c) correlating the potential therapeutic effect and the test substance, wherein the potential therapeutic effect is shown, when a substance increases the expression level of the downstream gene as compared to a control.

The screening method are described in more detail below. Cells expressing the SUV39H2 and downstream genes shown in Table 7 and Table 8 include, for example, cell lines established from lung cancer, cervical cancer, bladder cancer, esophageal cancer, osteosarcoma, prostate canter or soft tissue tumor. Such cells can be used for the above screening methods of the present description (e.g., SW780, RT4, A549, LC319, SBC5). The expression level can be estimated by methods well known to one skilled in the art, for example, RT-PCR, Northern blot assay, Western blot assay, immunostaining and flow cytometry analysis. As noted above, the phrase "reduce the expression level" is defined herein as preferably at least 10% reduction of expression level of SUV39H2 or the downstream genes in comparison to the expression level in the absence of the test substance, more preferably at least 25%, 50% or 75% reduced level and most preferably at 95% reduced level. Test substances herein include, for example, chemical substances, double-strand molecules, and so on. Methods for preparation of chemical substances are described in the above description. Methods for preparation of double-stranded molecules will be described bellow. In the screening method, test substances that reduces the expression level of the SUV39H2 gene can be selected as candidate substances to be used for the treatment or prevention of cancer associating SUV39H2 over-expression, such as lung cancer, cervical cancer, bladder cancer, esophageal cancer, osteosarcoma, prostate cancer and soft tissue tumor. Potential of these candidate substances to treat or prevent cancers may be evaluated by either of second or further screening to identify therapeutic substance for cancers or both. In some embodiments, cells expressing SUV39H2 gene are isolated and cultured cells exogenously or endogenously expressing SUV39H2 gene in vitro.

Alternatively, the screening method of the present invention may include the following steps:
(a) contacting a test substance with a cell into which a vector, including the transcriptional regulatory region of the SUV39H2 gene, and a reporter gene that is expressed under the control of the transcriptional regulatory region, has been introduced;
(b) detecting the expression level or activity of the reporter gene; and
(c) selecting the test substance that reduces the expression level or activity of the reporter gene. in comparison with the expression level or activity detected in absence of the test substance.

Furthermore, the present description provides a method of evaluating or estimating therapeutic effect of a test substance on either or both of treating and preventing cancer or inhibiting cancer cell growth, the method including steps of:
(a) contacting a test substance with a cell into which a vector, including the transcriptional regulatory region of the SUV39H2 gene, and a reporter gene that is expressed under the control of the transcriptional regulatory region, has been introduced;
(b) detecting the expression level or activity of the reporter gene; and
(c) correlating the potential therapeutic effect of the test substance with the expression level or activity detected in step (b), wherein the potential therapeutic effect is shown, when the test substance reduces the expression level or activity of the reporter gene in comparison with the expression level or activity detected in absence of the test substance.

In the context of the present description, the therapeutic effect may be correlated with the expression level or activity of the reporter gene. For example, when the test substance reduces the expression level or activity of the reporter gene as compared to a level detected in the absence of the test substance, the test substance may identified or selected as the candidate substance having the therapeutic effect. Alternatively, when the test substance does not reduce the expression level or activity of said reporter gene as compared to a level detected in the absence of the test substance, the test substance may identified as the substance having no significant therapeutic effect.

Suitable reporter genes and host cells are well known in the art. For example, reporter genes are luciferase, green fluorescence protein (GFP), Discosoma sp. Red Fluorescent Protein (DsRed), Chrolamphenicol Acetyltransferase (CAT), lacZ and beta-glucuronidase (GUS), and host cell is COS7, HEK293, HeLa and so on. The reporter construct required for the screening can be prepared by connecting reporter gene sequence to the transcriptional regulatory region of SUV39H2. The transcriptional regulatory region of SUV39H2 herein is the region from start codon to at least 500 bp upstream, preferably 1,000 bp, more preferably 5000 or 10,000 bp upstream. A nucleotide segment containing the transcriptional regulatory region can be isolated from a genome library or can be propagated by PCR. The reporter construct required for the screening can be prepared by connecting reporter gene sequence to the transcriptional regulatory region of any one of these genes. Methods for identifying a transcriptional regulatory region, and also assay protocol are well known (Molecular Cloning third edition chapter 17, 2001, Cold Springs Harbor Laboratory Press).

The vector containing the reporter construct is infected to host cells and the expression or activity of the reporter gene is detected by method well known in the art (e.g., using luminometer, absorption spectrometer, flow cytometer and so on). In some embodiments, cells are isolated and cultured cells into which a vector, composed of the transcriptional regulatory region of the SUV39H2 gene and a reporter gene that is expressed under the control of the transcriptional regulatory region, has been introduced in vitro. "Reduces the expression or activity" as defined herein are preferably at least 10% reduction of the expression or activity of the reporter gene in comparison with in absence of the substance, more preferably at least 25%, 50% or 75% reduction and most preferably at 95% reduction.

It is herein revealed that suppressing (reducing, inhibiting) the expression of the SUV39H2 gene suppresses (reduces, inhibits) cell growth. Thus, by screening for a candidate substance that reduces the expression or activity of the reporter gene, a candidate substance that has the potential to treat or prevent cancers can be identified. Potential of these candidate substances to treat or prevent cancers may be evaluated by second and/or further screening to identify therapeutic substance for cancers.

### Double-Stranded Molecules:

As used herein, the term "isolated double-stranded molecule" refers to a nucleic acid molecule that inhibits expression of a target gene and includes, for example, short interfering RNA (siRNA; e.g., double-stranded ribonucleic acid (dsRNA) or small hairpin RNA (shRNA)) and short interfering DNA/RNA (siD/R-NA; e.g., double-stranded chimera of DNA and RNA (dsD/R-NA) or small hairpin chimera of DNA and RNA (shD/R-NA)).

Herein, "double-stranded molecule" is also referred to as "double-stranded nucleic acid ", " double-stranded nucleic acid molecule", "double-stranded polynucleotide", "double-stranded polynucleotide molecule", "double-stranded oligonucleotide" and "double-stranded oligonucleotide molecule".

As used herein, the term "target sequence" refers to a nucleotide sequence within mRNA or cDNA sequence of a target gene, which will result in suppression of translation of the whole mRNA of the target gene if the double-stranded molecule targeting the sequence is introduced within a cell expressing the gene. A nucleotide sequence within the mRNA or cDNA sequence of a target gene can be determined to be a target sequence when a double-stranded molecule having a sequence corresponding to the target sequence inhibits expression of the gene in a cell expressing the gene. When a target sequence is shown by cDNA sequence, a sense strand sequence of a double-stranded cDNA, i.e., a sequence that mRNA sequence is converted into DNA sequence, is used for defining a target sequence. A double-stranded molecule is composed of a sense strand that has a sequence corresponding to a target sequence and an antisense strand that has a complementary sequence to the target sequence, and the antisense strand hybridizes with the sense strand at the complementary sequence to form a double-stranded molecule.

Herein, the phrase " corresponding to" means converting a target sequence according to the kind of nucleic acid that constitutes a sense strand of a double-stranded molecule. For example, when a target sequence is shown in DNA sequence and a sense strand of a double-stranded molecule has an RNA region, base "t"s within the RNA region are replaced with base "u"s. On the other hand, when a target sequence is shown in an RNA sequence and a sense strand of a double-stranded molecule has a DNA region, base "u"s within the DNA region are replaced with "t"s.

For example, when a target sequence is the RNA sequence shown in SEQ ID NO: 19 or 20 and the sense strand of the double-stranded molecule has the 3' side half region composed of DNA, "a sequence corresponding to a target sequence" is "5'-CUUUGGUUGTTCATGCACA-3"' (for SEQ ID NO: 19), or "5'-CUGGAAUCAGCTTAGTCAA-3"' (for SEQ ID NO: 20).

Also, a complementary sequence to a target sequence for an antisense strand of a double-stranded molecule can be defined according to the kind of nucleic acid that constitutes the antisense strand. For example, when a target sequence is the RNA sequence shown in SEQ ID NO: 19 or 20 and the antisense strand of the double-stranded molecule has the 5' side half region composed of DNA, " a complementary sequence to a target sequence " is "3'-GAAACCAACAAGTACGTGT-5"' (for SEQ ID NO: 19) or "3'-GACCUUAGUCGAATCAGTT-5"' (for SEQ ID NO: 20).

On the other hand, when a double-stranded molecule is composed of RNA, the sequence corresponding to a target sequence shown in SEQ ID NO: 19 or 20 is the RNA sequence of SEQ ID NO: 19 or 20, and the complementary sequence corresponding to a target sequence shown in SEQ ID NO: 19 or 20 is the RNA sequence of "3'- GAAACCAACAAGUACGUGU -5'" (for SEQ ID NO:19) or "3'- GACCU-UAGUCGAAUCAGUU -5'" (for SEQ ID NO:20).

A double-stranded molecule may have either of one or two 3'overhangs having 2 to 5 nucleotides in length (e.g., uu) or a loop sequence that links a sense strand and an antisense strand to form hairpin structure, or both, in addition to a sequence corresponding to a target sequence and complementary sequence thereto.

As used herein, the term "siRNA" refers to a double-stranded RNA molecule which prevents translation of a target mRNA. Standard techniques of introducing siRNA into the cell are used, including those in which DNA is a template from which RNA is transcribed. Alternatively, siRNA may also be directly introduced in cells to be treated. Methods of introducing siRNA in a subject are well known in the art. For example, an administration of siRNA in conjunction with a delivery substance is preferable for the introduction of siRNA.

The siRNA includes an SUV39H2 sense nucleic acid sequence (also referred to as "sense strand"), an SUV39H2 antisense nucleic acid sequence (also referred to as "antisense strand") or both. The siRNA may be constructed such that a single transcript has both the sense and complementary antisense nucleic acid sequences of the target gene, e.g., a hairpin. The siRNA may either be a dsRNA or shRNA.

As used herein, the term "dsRNA" refers to a construct of two RNA molecules composed of complementary sequences to one another and that have annealed together via the complementary sequences to form a double-stranded RNA molecule. The nucleotide sequence of two strands may include not only the "sense" or "antisense" RNAs selected from a protein coding sequence of target gene sequence, but also RNA molecule having a nucleotide sequence selected from non-coding region of the target gene.

The term "shRNA", as used herein, refers to an siRNA having a stem-loop structure, composed of first and second regions complementary to one another, i.e., sense and antisense strands. The degree of complementarity and orientation of the regions are sufficient such that base pairing occurs between the regions, the first and second regions are joined by a loop region, the loop results from a lack of base pairing between nucleotides (or nucleotide analogs) within the loop region. The loop region of an shRNA is a single-stranded region intervening between the sense and antisense strands and may also be referred to as "intervening single-strand".

As used herein, the term "siD/R-NA" refers to a double-stranded polynucleotide molecule which is composed of both RNA and DNA, and includes hybrids and chimeras of RNA and DNA and prevents translation of a target mRNA. Herein, a hybrid indicates a molecule wherein a polynucleotide composed of DNA and a polynucleotide composed of RNA hybridize to each other to form the double-stranded molecule; whereas a chimera indicates that one or both of the strands composing the double stranded molecule may contain RNA and DNA. Standard techniques of introducing siD/R-NA into the cell are used. The siD/R-NA includes an SUV39H2 sense nucleic acid sequence (also referred to as "sense strand"), an SUV39H2 antisense nucleic acid sequence (also referred to as "antisense strand") or both. The siD/R-NA may be constructed such that a single transcript has both the sense and complementary antisense nucleic acid sequences from the target gene, e.g., a hairpin. The siD/R-NA may either be a dsD/R-NA or shD/R-NA.

As used herein, the term "dsD/R-NA" refers to a construct of two molecules composed of complementary sequences to one another and that have annealed together via the complementary sequences to form a double-stranded polynucleotide molecule. The nucleotide sequence of two strands may include not only the "sense" or "antisense" polynucleotides sequence selected from a protein coding sequence of target gene sequence, but also polynucleotide having a nucleotide sequence selected from non-coding region of the target gene. One or both of the two molecules constructing the dsD/R-NA are composed of both RNA and DNA (chimeric molecule), or alternatively, one of the molecules is composed of RNA and the other is composed of DNA (hybrid double-strand).

The term "shD/R-NA", as used herein, refers to an siD/R-NA having a stem-loop structure, composed of a first and second regions complementary to one another, i.e., sense and antisense strands. The degree of complementarity and orientation of the regions are sufficient such that base pairing occurs between the regions, the first and second regions are joined by a loop region, the loop results from a lack of base pairing between nucleotides (or nucleotide analogs) within the loop region. The loop region of an shD/R-NA is a single-stranded region intervening between the sense and antisense strands and may also be referred to as "intervening single-strand".

As used herein, an "isolated nucleic acid" is a nucleic acid removed from its original environment (e.g., the natural environment if naturally occurring) and thus, synthetically altered from its natural state. In the present description, examples of isolated nucleic acid includes DNA, RNA, and derivatives thereof.

In the context of the present description, a double-stranded molecule against the SUV39H2 gene will hybridize to target mRNA, decrease or inhibit production of the SUV39H2 protein encoded by the SUV39H2 gene by associating with the normally single-stranded mRNA transcript of the gene, thereby interfering with translation and thus, inhibiting expression of the protein. As demonstrated herein, the expression of the SUV39H2 gene in several cancer cell lines is inhibited by dsRNA (Figures 4C and 4D). Therefore the present invention provides isolated double-stranded molecules that are capable of inhibiting the expression of the SUV39H2 gene when introduced into a cell expressing the gene. The target sequence of double-stranded molecule may be designed by an siRNA design algorithm such as that mentioned below.

The target sequence for the SUV39H2 gene includes, for example, a nucleotide sequence of SEQ ID NO: 19 or 20.

The double-stranded molecule of the present invention is described in more detail below.

Methods for designing double-stranded molecules having the ability to inhibit target gene expression in cells are known. (See, for example, US Patent No. 6,506,559). For example, a computer program for designing siRNAs is available from the Ambion website (ambion.com/techlib/misc/siRNA_finder.html).

Such a computer program selects target nucleotide sequences for double-stranded molecules based on the following protocol.

### Selection of Target Sites:

1. Beginning with the AUG start codon of the transcript, scan downstream for AA di-nucleotide sequences. Record the occurrence of each AA and the 3' adjacent 19 nucleotides as potential siRNA target sites. Tuschl et al. recommend to avoid designing siRNA to the 5' and 3' untranslated regions (UTRs) and regions near the start codon (within 75 bases) as these may be richer in regulatory protein binding sites, and UTR-binding proteins and/or translation initiation complexes may interfere with binding of the siRNA endonuclease complex.
2. Compare the potential target sites to the appropriate genome database (human, mouse, rat, etc.) and eliminate from consideration any target sequences with significant homology to other coding sequences. BLAST, which can be found on the NCBI server at: ncbi.nlm.nih.gov/BLAST/, is used (Altschul SF et al., Nucleic Acids Res 1997 Sep 1, 25(17): 3389-402).
3. Select qualifying target sequences for synthesis. Selecting several target sequences along the length of the gene to evaluate is typical.

Using the above protocol, the target sequence of the isolated double-stranded molecules of the present invention were designed as SEQ ID NO: 19 or 20.

Double-stranded molecules targeting the above-mentioned target sequences were respectively examined for their ability to suppress the growth of cells expressing the target genes. Accordingly, the present description provides double-stranded molecule targeting the sequences of SEQ ID NO: 19 or 20 for SUV39H2 gene.

Examples of double-stranded molecules of the present description that target the above-mentioned target sequence of the SUV39H2 gene include isolated polynucleotides that contain the nucleic acid sequences corresponding to either of target sequences or complementary sequences to the target sequences, or both. Preferred examples of polynucleotides targeting the SUV39H2 gene include those containing the sequence corresponding to SEQ ID NO: 19 or 20 and/or complementary sequences to these sequences. In an embodiment, a double-stranded molecule is composed of two polynucleotides, one polynucleotide has a sequence corresponding to a target sequence, i.e., sense strand, and another polypeptide has a complementary sequence to the target sequence, i.e., antisense strand. The sense strand polynucleotide and the antisense strand polynucleotide hybridize to each other to form double-stranded molecule. Examples of such double-stranded molecules include dsRNA and dsD/ R-NA.

In another embodiment, a double-stranded molecule is composed of a polynucleotide that has both a sequence corresponding to a target sequence, i.e., a sense strand, and a complementary sequence to the target sequence, i.e., an antisense strand. Generally, the sense strand and the antisense strand are linked by an intervening strand, and hybridize to each other to form a hairpin loop structure. Examples of such double-stranded molecule include shRNA and shD/R-NA.

In other words, a double-stranded molecule of the present description is composed of a sense strand polynucleotide having a nucleotide sequence of the target sequence and anti-sense strand polynucleotide having a nucleotide sequence complementary to the target sequence, and both of polynucleotides hybridize to each other to form the double-stranded molecule. In the double-stranded molecule including the polynucleotides, a part of the polynucleotide of either or both of the strands may be RNA, and when the target sequence is defined with a DNA sequence, the nucleotide "t" within the target sequence and complementary sequence thereto is replaced with "u".

In one embodiment of the present description, such a double-stranded molecule of the present invention includes a stem-loop structure, composed of the sense and antisense strands. The sense and antisense strands may be joined by a loop. Accordingly, the present description also provides the double-stranded molecule composed of a single polynucleotide containing both the sense strand and the antisense strand linked or flanked by an intervening single-strand.

The double-stranded molecule may be directed to a single target SUV39H2 gene sequence or may be directed to a plurality of target SUV39H2 gene sequences.

A double-stranded molecule the above-mentioned targeting sequence of the SUV39H2 gene include isolated polynucleotides that contain the nucleic acid sequences of target sequences and/or complementary sequences to the target sequence. Example of polynucleotide targeting the SUV39H2 gene includes that containing the sequence of SEQ ID NO: 19 or 20, and/or complementary sequences to these nucleotides. However, the present description is not limited to this example, and minor modifications in the aforementioned nucleic acid sequences are acceptable so long as the modified molecule retains the ability to suppress the expression of the SUV39H2 gene. Herein, the phrase "minor modification" as used in connection with a nucleic acid sequence indicates one, two or several substitution, deletion, addition or insertion of nucleic acids to the sequence.

In the context of the present description, the term "several" as applies to nucleic acid substitutions, deletions, additions and/or insertions may mean 3-7, preferably 3-5, more preferably 3-4, even more preferably 3 nucleic acid residues.

According to the present description, a double-stranded molecule can be tested for its ability to reduce, suppress or inhibit expression using the methods utilized in the Examples. In the Examples herein below, double-stranded molecules composed of sense strands of various portions of mRNA of the SUV39H2 genes or antisense strands complementary thereto were tested in vitro for their ability to decrease production of the SUV39H2 gene product in cancer cell lines according to standard methods. Furthermore, for example, reduction in the SUV39H2 gene product in cells contacted with the candidate double-stranded molecule compared to cells cultured in the absence of the candidate molecule can be detected by, e.g., RT-PCR using primers for the SUV39H2 mRNA mentioned under Example 1, item "Quantitative RT-PCR". Sequences which decrease the production of the SUV39H2 gene product in in vitro cell-based assays can then be tested for their inhibitory effects on cell growth. Sequences which inhibit cell growth in in vitro cell-based assay can then be tested for their in vivo ability using animals with cancer, e.g., nude mouse xenograft models, to confirm decreased production of the SUV39H2 product and decreased cancer cell growth.

When the isolated polynucleotide is RNA or derivatives thereof, base "t" should be replaced with "u" in the nucleotide sequences. As used herein, the term "complementary" refers to Watson-Crick or Hoogsteen base pairing between nucleotides units of a polynucleotide, and the term "binding" means the physical or chemical interaction between two polynucleotides. When the polynucleotide includes modified nucleotides and/or non-phosphodiester linkages, these polynucleotides may also bind each other as same manner. Generally, complementary polynucleotide sequences hybridize under appropriate conditions to form stable duplexes containing few or no mismatches. However, the present description extends to complementary sequences that include mismatches of one or more nucleotides. In addition, the sense strand and antisense strand of the isolated polynucleotide can form double-stranded molecule or hairpin loop structure by the hybridization. In a preferred embodiment, such duplexes contain no more than 1 mismatch for every 10 matches. In an especially preferred embodiment, where the strands of the duplex are fully complementary, such duplexes contain no mismatches.

The complementary or antisense polynucleotide is preferably less than 1,000 nucleotides in length for the SUV39H2 gene. Preferably, the polynucleotide is less than 500, 200, 100, 75, 50, or 25 nucleotides in length for all of the genes. The isolated polynucleotides are useful for forming double-stranded molecules against the SUV39H2 gene or preparing template DNAs encoding the double-stranded molecules. When the polynucleotides are used for forming double-stranded molecules, the sense strand of polynucleotide may be longer than 19 nucleotides, preferably longer than 21 nucleotides, and more preferably has a length of between about 19 and 25 nucleotides.

Accordingly, the present description provides the double-stranded molecules comprising a sense strand and an antisense strand, wherein the sense strand comprises a nucleotide sequence corresponding to a target sequence. In preferable embodiments, the sense strand hybridizes with antisense strand at the target sequence to form the double-stranded molecule having between 19 and 25 nucleotide pair in length.

The double-stranded molecule serves as a guide for identifying homologous sequences in mRNA for the RISC complex, when the double-stranded molecule is introduced into cells. The identified target RNA is cleaved and degraded by the nuclease activity of Dicer, through which the double-stranded molecule eventually decreases or inhibits production (expression) of the polypeptide encoded by the RNA. Thus, a double-stranded molecule be defined by its ability to generate a single-strand that specifically hybridizes to the mRNA of the SUV39H2 gene under stringent conditions. Herein, the portion of the mRNA that hybridizes with the single-strand generated from the double-stranded molecule is referred to as "target sequence" or "target nucleic acid" or "target nucleotide". In the present description, nucleotide sequence of the "target sequence" can be shown using not only the RNA sequence of the mRNA, but also the DNA sequence of cDNA synthesized from the mRNA.

Alternatively, the double-stranded molecules may be double-stranded molecules, wherein the sense strand is hybridize with antisense strand at the target sequence to form the double-stranded molecule having less than 500, 200, 100, 75, 50 or 25 nucleotides pair in length. Preferably, the double-stranded molecules have between about 19 and about 25 nucleotides pair in length. Further, the sense strand of the double-stranded molecule may preferably include less than 500, 200, 100, 75, 50, 30, 28, 27, 26, 25 nucleotides, more preferably, between about 19 and about 25 nucleotides.

The double-stranded molecules may contain one or more modified nucleotides and/or non-phosphodiester linkages. It is well known in the art to introduce chemical modifications capable of increasing stability, availability and/or cell uptake of the double-stranded molecule. A person skilled in the art will be aware of wide array of chemical modification which may be incorporated into the present molecules (WO03/070744; WO2005/045037). For example, in one embodiment, modifications can be used to provide improved resistance to degradation or improved uptake. Examples of such modifications include, but are not limited to, phosphorothioate linkages, 2'-O-methyl ribonucleotides (especially on the sense strand of a double-stranded molecule), 2'-deoxy-fluoro ribonucleotides, 2'-deoxy ribonucleotides, "universal base" nucleotides, 5'-C- methyl nucleotides, and inverted deoxybasic residue incorporation (US20060122137).

In another embodiment, modifications can be used to enhance the stability or to increase targeting efficiency of the double-stranded molecule. Examples of such modifications include, but are not limited to, chemical cross linking between the two complementary strands of a double-stranded molecule, chemical modification of a 3' or 5' terminus of a strand of a double-stranded molecule, sugar modifications, nucleobase modifications, and/or backbone modifications, 2 -fluoro modified ribonucleotides and 2'-deoxy ribonucleotides (WO2004/029212). In another embodiment, modifications can be used to increase or decrease affinity for the complementary nucleotides in the target mRNA and/or the complementary double-stranded molecule strand (WO2005/044976). For example, an unmodified pyrimidine nucleotide can be substituted for a 2-thio, 5-alkynyl, 5-methyl, or 5-propynyl pyrimidine. Additionally, an unmodified purine can be substituted with a 7-deaza, 7-alkyl, or 7-alkenyl purine. In another embodiment, when the double-stranded molecule is a double-stranded molecule with a 3' overhang, the 3'- terminal nucleotide overhanging nucleotides may be replaced by deoxyribonucleotides (Elbashir SM et al., Genes Dev 2001 Jan 15, 15(2): 188-200).

For further details, see, e.g., US20060234970. However, the present description should not be construed as limited to these examples; any of a number of chemical modifications may be employed for the double-stranded molecules so long as the resulting molecule retains the ability to inhibit the expression of the target gene.

The double-stranded molecules may include both DNA and RNA, e.g., dsD/R-NA or shD/R-NA. For example, a hybrid polynucleotide of a DNA strand and an RNA strand or a DNA-RNA chimera polynucleotide shows increased stability and thus are contemplated herein. Mixing of DNA and RNA, i.e., a hybrid type double-stranded molecule composed of a DNA strand (polynucleotide) and an RNA strand (polynucleotide), a chimera type double-stranded molecule containing both DNA and RNA on any or both of the single strands (polynucleotides), or the like may be formed for enhancing stability of the double-stranded molecule.

The hybrid of a DNA strand and an RNA strand may either have a DNA sense strand that is coupled to an RNA antisense strand, or vice versa, so long as the resulting double stranded molecule can inhibit expression of the target gene when introduced into a cell expressing the gene. In a preferred embodiment, the sense strand polynucleotide is DNA and the antisense strand polynucleotide is RNA.

Also, the chimera type double-stranded molecule may have either or both of the sense and antisense strands composed of DNA and RNA, or have any one of the sense and antisense strands composed of DNA and RNA so long as the resulting double stranded molecule has an activity to inhibit expression of the target gene when introduced into a cell expressing the gene. In order to enhance stability of the double-stranded molecule, the molecule preferably contains as much DNA as possible, whereas to induce inhibition of the target gene expression, the molecule is required to be RNA within a range to induce sufficient inhibition of the expression.

A preferred chimera type double-stranded molecule contains an upstream partial region (i.e., a region flanking to the target sequence or complementary sequence thereof within the sense or antisense strands) of RNA. Preferably, the upstream partial region indicates the 5' side (5'-end) of the sense strand and the 3' side (3'-end) of the antisense strand. Alternatively, regions flanking to 5'-end of sense strand and/or 3'-end of antisense strand may be referred to as the upstream partial region. That is, in preferred embodiments, a region flanking to the 3'-end of the antisense strand, or both of a region flanking to the 5'-end of sense strand and a region flanking to the 3'-end of antisense strand are composed of RNA. For instance, a chimera or hybrid type double-stranded molecule may include following combinations:
sense strand:
   5'-[---DNA---]-3'
   3'-(RNA)-[DNA]-5'
:antisense strand,
sense strand:
   5'-(RNA)-[DNA]-3'
   3'-(RNA)-[DNA]-5'
:antisense strand, and
sense strand:
   5'-(RNA)-[DNA]-3'
   3'-(---RNA---)-5'
:antisense strand.

The upstream partial region preferably is a domain composed of 9 to 13 nucleotides counted from the terminus of the target sequence or complementary sequence thereto within the sense or antisense strands of the double-stranded molecules. Moreover, preferred examples of such chimera type double-stranded molecules include those having a strand length of 19 to 21 nucleotides in which at least the upstream half region (5' side region for the sense strand and 3' side region for the antisense strand) of the polynucleotide is RNA and the other half is DNA. In such a chimera type double-stranded molecule, the effect to inhibit expression of the target gene is much higher when the entire antisense strand is RNA (US20050004064).

In the context of the present description, the double-stranded molecule may form a hairpin, such as a short hairpin RNA (shRNA) and short hairpin composed of DNA and RNA (shD/R-NA). The shRNA or shD/R-NA is a sequence of RNA or mixture of RNA and DNA making a tight hairpin turn that can be used to silence gene expression via RNA interference. The shRNA or shD/R-NA includes the sense target sequence and the antisense target sequence on a single strand wherein the sequences are separated by a loop sequence. Generally, the hairpin structure is cleaved by the cellular machinery into dsRNA or dsD/R-NA molecules, which are then bound to the RNA-induced silencing complex (RISC). This complex binds to and cleaves mRNAs that match the target sequence of the dsRNA or dsD/R-NA.

A loop sequence composed of an arbitrary nucleotide sequence can be located between the sense and antisense sequence in order to form the hairpin loop structure. Such loop sequence may be joined to 5' or 3' end of a sense strands to form the hairpin loop structure. Thus, the present description also provides a double-stranded molecule having the general formula 5'-[A]-[B]-[A']-3' or 5'-[A']-[B]-[A]-3', wherein [A] is the sense strand containing a sequence corresponding to a target sequence, [B] is an intervening single-strand and [A'] is the antisense strand containing a complementary sequence to [A]. The target sequence may be selected from among, for example, nucleotide sequence of SEQ ID NO: 19 or 20.

The present description is not limited to these examples, and the target sequence in [A] may be modified sequences from these examples so long as the double-stranded molecule retains the ability to suppress the expression of the targeted the SUV39H2 gene. The region [A] hybridizes to [A'] to form a loop composed of the region [B]. The intervening single-stranded portion [B], i.e., loop sequence may be preferably 3 to 23 nucleotides in length. The loop sequence, for example, can be selected from among the sequences found, e.g., at the Ambion website (ambion.com/techlib/tb/tb_506.html). Furthermore, loop sequence composed of 23 nucleotides also provides active siRNA (Jacque JM et al., Nature 2002 Jul 25, 418(6896): 435-8, Epub 2002 Jun 26):
CCC, CCACC, or CCACACC: Jacque JM et al., Nature 2002 Jul 25, 418(6896): 435-8, Epub 2002 Jun 26;
UUCG: Lee NS et al., Nat Biotechnol 2002 May, 20(5): 500-5; Fruscoloni P et al., Proc Natl Acad Sci USA 2003 Feb 18, 100(4): 1639-44, Epub 2003 Feb 10; and
UUCAAGAGA: Dykxhoorn DM et al., Nat Rev Mol Cell Biol 2003 Jun, 4(6): 457-67.

Examples of preferred double-stranded molecules having hairpin loop structure are shown below. In the following structure, the loop sequence can be selected from among AUG, CCC, UUCG, CCACC, CTCGAG, AAGCUU, CCACACC, and UUCAAGAGA; however, the present description is not limited thereto:
CUUUGGUUGUUCAUGCACA-[B]-UGUGCAUGAACAACCAAAG (for target sequence SEQ ID NO: 19), and
CUGGAAUCAGCUUAGUCAA-[B]- UUGACUAAGCUGAUUCCAG (for target sequence SEQ ID NO: 20).

Additionally, several nucleotides can be added to 3' end of the sense strand and/or antisense strand of the target sequence, as 3' overhangs so as to enhance the inhibition activity of the double-stranded molecules. The preferred examples of nucleotides constituting a 3' overhang include "t" and "u", but are not limited thereto . The number of nucleotides to be added is at least 2, generally 2 to 10, preferably 2 to 5. The added nucleotides form single strand at the 3' end of the sense strand and/or antisense strand of the double-stranded molecule. In cases where double-stranded molecules is composed of a single polynucleotide to form a hairpin loop structure, a 3' overhang sequence may be added to the 3' end of the single polynucleotide.

The method for preparing the double-stranded molecule is not particularly limited though it is preferable to use one of the standard chemical synthetic method known in the art. According to one chemical synthesis method, sense and antisense single-stranded polynucleotides are separately synthesized and then annealed together via an appropriate method to obtain a double-stranded molecule. In one specific embodiment, the synthesized single-stranded polynucleotides are mixed in a molar ratio of preferably at least about 3:7, more preferably about 4:6, and most preferably substantially equimolar amount (i.e., a molar ratio of about 5:5). Next, the mixture is heated to a temperature at which double-stranded molecules dissociate and then is gradually cooled down. The annealed double-stranded polynucleotide can be purified by usually employed methods known in the art. Example of purification methods include methods utilizing agarose gel electrophoresis or wherein remaining single-stranded polynucleotides are optionally removed by, e.g., degradation with appropriate enzyme.

The regulatory sequences flanking the SUV39H2 sequences may be identical or different, such that their expression can be modulated independently, or in a temporal or spatial manner. The double-stranded molecules can be transcribed intracellularly by cloning the SUV39H2 gene templates into a vector containing, e.g., an RNA pol III transcription unit from the small nuclear RNA (snRNA) U6 or the human H1 RNA promoter.

### Vector Encoding a Double-Stranded Molecule of the Present Invention:

Also included in the present description are vectors encoding one or more of the double-stranded molecules described herein, and a cell containing such a vector.

A vector of the present description preferably encodes a double-stranded molecule in an expressible form. Herein, the phrase "in an expressible form" indicates that the vector, when introduced into a cell, will express the molecule carried, contained or encoded therein. In a preferred embodiment, the vector includes one or more regulatory elements necessary for expression of the double-stranded molecule. Accordingly, in one embodiment, the expression vector encodes the nucleic acid sequences and is adapted for expression of said nucleic acid sequences. Such vectors may be used for producing the present double-stranded molecules, or directly as an active ingredient for treating cancer.

Vectors can be produced, for example, by cloning an SUV39H2 sequence into an expression vector so that regulatory sequences are operatively-linked to the SUV39H2 sequence in a manner to allow expression (by transcription of the DNA molecule) of both strands (Lee NS et al., Nat Biotechnol 2002 May, 20(5): 500-5). For example, the RNA molecule that is the antisense to mRNA is transcribed by a first promoter (e.g., a promoter sequence flanking to the 3' end of the cloned DNA) and the RNA molecule that is the sense strand to the mRNA is transcribed by a second promoter (e.g., a promoter sequence flanking to the 5' end of the cloned DNA). The sense and antisense strands hybridize in vivo to generate a double-stranded molecule constructs for silencing of the gene. Alternatively, two vector constructs respectively encoding the sense and antisense strands of the double-stranded molecule are utilized to respectively express the sense and anti-sense strands and then forming a double-stranded molecule construct. Furthermore, the cloned sequence may encode a construct having a secondary structure (e.g., hairpin); accordingly, a single transcript of a vector may contain both the sense and complementary antisense sequences of the target gene.

The present description contemplates a vector that includes each or both of a combination of polynucleotides, including a sense strand nucleic acid and an antisense strand nucleic acid, wherein the antisense strand includes a nucleotide sequence which is complementary to said sense strand, wherein the transcripts of said sense strand and said antisense strand hybridize to each other to form said double-stranded molecule, and wherein said vector, when introduced into a cell expressing the SUV39H2 gene, inhibits expression of said gene.

The vectors may also be equipped so to achieve stable insertion into the genome of the target cell (see, e.g., Thomas KR & Capecchi MR, Cell 1987, 51: 503-12 for a description of homologous recombination cassette vectors). See, e.g., Wolff et al., Science 1990, 247: 1465-8; US Patent Nos. 5,580,859; 5,589,466; 5,804,566; 5,739,118; 5,736,524; 5,679,647; and WO 98/04720. Examples of DNA-based delivery technologies include "naked DNA", facilitated (bupivacaine, polymers, peptide-mediated) delivery, cationic lipid complexes, and particle-mediated ("gene gun") or pressure-mediated delivery (see, e.g., US Patent No. 5,922,687).

The vectors include, for example, viral or bacterial vectors. Examples of expression vectors include attenuated viral hosts, such as vaccinia or fowlpox (see, e.g., US Patent No. 4,722,848). This approach involves the use of vaccinia virus, e.g., as a vector to express nucleotide sequences that encode the double-stranded molecule. Upon introduction into a cell expressing the target gene, the recombinant vaccinia virus expresses the molecule and thereby suppresses the proliferation of the cell. Another example of useable vector includes Bacille Calmette Guerin (BCG). BCG vectors are described in Stover et al., Nature 1991, 351: 456-60. A wide variety of other vectors are useful for therapeutic administration and production of the double-stranded molecules; examples include adeno and adeno-associated virus vectors, retroviral vectors, Salmonella typhi vectors, detoxified anthrax toxin vectors, and the like. See, e.g., Shata et al., Mol Med Today 2000, 6: 66-71; Shedlock et al., J Leukoc Biol 2000, 68: 793-806; and Hipp et al., In Vivo 2000, 14: 571-85.

### Method of Treating Cancer Using a Double-Stranded Molecule of the Present Description:

dsRNAs against the SUV39H2 gene were tested for their ability to inhibit cell growth. The dsRNA against the SUV39H2 gene effectively knocked down the expression of the gene in several cancer cell lines coincided with suppression of cell proliferation (Figure 4).

Therefore, the present description provides methods for inhibiting cell growth, i.e., a cancer cell, by inducing dysfunction of the SUV37H2 gene via inhibiting the expression of the SUV39H2 gene. The SUV39H2 gene expression can be inhibited by any of the aforementioned double-stranded molecules which specifically target of SUV39H2 gene.

Such ability of the present double-stranded molecules and vectors to inhibit cell growth of cancerous cell indicates that they can be used for methods for treating cancer, as well as treating or preventing a post-operative, secondary, or metastatic recurrence thereof. Exemplary cancers include, but is not limited to, e.g., lung cancer, cervical cancer, bladder cancer, esophageal cancer, osteosarcoma, prostate cancer and soft tissue tumor.. Thus, the present description provides methods to treat patients with cancer by administering a double-stranded molecule against the SUV39H2 gene or a vector expressing the molecule without adverse effect because the SUV39H2 gene was minimally detected in normal organs (Figure 1).

Therapeutic and prophylactic methods are described in more detail below.

The growth of cells expressing the SUV39H2 gene may be inhibited by contacting the cells with a double-stranded molecule against the SUV39H2 gene, a vector expressing the molecule or a composition containing the same. The cell may be further contacted with a transfection agent. Suitable transfection agents are known in the art. The phrase "inhibition of cell growth" indicates that the cell proliferates at a lower rate or has decreased viability as compared to a cell not exposed to the molecule. Cell growth may be measured by any number of methods known in the art, e.g., using the colony formation assay or the MTT cell proliferation assay.

The growth of any kind of cell may be suppressed according to the present method so long as the cell expresses or over-expresses the target gene of the double-stranded molecule. Exemplary cells include lung cancer, cervical cancer, bladder cancer, esophageal cancer, osteosarcoma, prostate cancer and soft tissue tumor.

Thus, patients suffering from or at risk of developing a disease associated with SUV39H2 over-expression may be treated by administering the present double-stranded molecule, at least one vector expressing the molecule or composition containing the molecule. For example, cancer patients may be treated according to the present methods. The type of cancer may be identified by standard methods according to the particular type of tumor to be diagnosed. More preferably, patients treated by the methods are selected by detecting the expression of the SUV39H2 gene in a biopsy sample from the patient by RT-PCR or immunoassay. Preferably, before the treatment the biopsy specimen from the subject is confirmed for the SUV39H2 gene over-expression by methods known in the art, for example, immunohistochemical analysis or RT-PCR.

For inhibiting cell growth, a double-stranded molecule may be directly introduced into the cells in a form to achieve binding of the molecule with corresponding mRNA transcripts. Alternatively, as described above, a DNA encoding the double-stranded molecule may be introduced into cells by means of a vector. For introducing the double-stranded molecules and vectors into the cells, transfection-enhancing agent, such as FuGENE (Roche diagnostics), Lipofectamine 2000 (Invitrogen), Oligofectamine (Invitrogen), and Nucleofector (Wako pure Chemical), may be employed.

As noted in the "Definitions" section above, a treatment is deemed "efficacious" if it leads to clinical benefit such as, reduction in expression of the SUV39H2 gene, or a decrease in size, prevalence, or metastatic potential of the cancer in the subject. When the treatment is applied prophylactically, "efficacious" means that it retards or prevents cancers from forming or prevents or alleviates a clinical symptom of cancer. Efficaciousness is determined in association with any known method for diagnosing or treating the particular tumor type.

Those of skill in the art understand that the double-stranded molecule degrades the SUV39H2 mRNA in substoichiometric amounts. Without wishing to be bound by any theory, it is believed that the double-stranded molecule causes degradation of the target mRNA in a catalytic manner. Thus, compared to standard cancer therapies, significantly less a double-stranded molecule needs to be delivered at or near the site of cancer to exert therapeutic effect.

One skilled in the art can readily determine an optimal effective amount of the double-stranded molecule to be administered to a given subject, by taking into account factors such as body weight, age, sex, type of disease, symptoms and other conditions of the subject; the route of administration; and whether the administration is regional or systemic. Generally, an effective amount of the double-stranded molecule is an intercellular concentration at or near the cancer site of from about 1 nanomolar (nM) to about 100 nM, preferably from about 2 nM to about 50 nM, more preferably from about 2.5 nM to about 10 nM. It is contemplated that greater or smaller amounts of the double-stranded molecule can be administered. The precise dosage required for a particular circumstance may be readily and routinely determined by one of skill in the art.

The present methods can be used to inhibit the growth or metastasis of cancer expressing, more particularly over-expressing the SUV39H2 gene; for example, lung cancer, cervical cancer, bladder cancer, esophageal cancer, osteosarcoma, prostate cancer and soft tissue tumor.

In particular, a double-stranded molecule containing a target sequence of the SUV39H2 gene (i.e., SEQ ID NO: 19 or 20) is particularly preferred for the treatment of cancer.

For treating cancer, the double-stranded molecule can also be administered to a subject in combination with a pharmaceutical substance different from the double-stranded molecule. Alternatively, the double-stranded molecule can be administered to a subject in combination with another therapeutic method designed to treat cancer. For example, the double-stranded molecule can be administered in combination with therapeutic methods currently employed for treating cancer or preventing cancer metastasis (e.g., radiation therapy, surgery and treatment using chemotherapeutic agents, such as cisplatin, carboplatin, cyclophosphamide, 5-fluorouracil, adriamycin, daunorubicin or tamoxifen).

In the context of the present methods, the double-stranded molecule can be administered to the subject either as a naked double-stranded molecule, in conjunction with a delivery reagent, or as a recombinant plasmid or viral vector that expresses the double-stranded molecule.

Suitable delivery reagents for administration in conjunction with the present a double-stranded molecule include the Mirus Transit TKO lipophilic reagent; lipofectin; lipofectamine; cellfectin; or polycations (e.g., polylysine), or liposomes. A preferred delivery reagent is a liposome.

Liposomes can aid in the delivery of the double-stranded molecule to a particular tissue, such as lung tumor tissue, cervical cancer, bladder cancer, esophageal cancer, osteosarcoma, prostate cancer and soft tissue tumor, and can also increase the blood half-life of the double-stranded molecule. Liposomes suitable for use in the present description are formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example, as described in Szoka et al., Ann Rev Biophys Bioeng 1980, 9: 467; and US Pat. Nos. 4,235,871; 4,501,728; 4,837,028; and 5,019,369.

Preferably, the liposomes encapsulating the present double-stranded molecule include a ligand molecule that can deliver the liposome to the cancer site. Ligands that bind to receptors prevalent in tumor or vascular endothelial cells, such as monoclonal antibodies that bind to tumor antigens or endothelial cell surface antigens, are preferred.

Particularly preferably, the liposomes encapsulating the present double-stranded molecule are modified so as to avoid clearance by the mononuclear macrophage and reticuloendothelial systems, for example, by having opsonization-inhibition moieties bound to the surface of the structure. In one embodiment, a liposome can include both opsonization-inhibition moieties and a ligand.

Opsonization-inhibiting moieties for use in preparing the liposomes are typically large hydrophilic polymers that are bound to the liposome membrane. As used herein, an opsonization inhibiting moiety is "bound" to a liposome membrane when it is chemically or physically attached to the membrane, e.g., by the intercalation of a lipid-soluble anchor into the membrane itself, or by binding directly to active groups of membrane lipids. These opsonization-inhibiting hydrophilic polymers form a protective surface layer which significantly decreases the uptake of the liposomes by the macrophage-monocyte system ("MMS") and reticuloendothelial system ("RES"); e.g., as described in US Pat. No. 4,920,016. Liposomes modified with opsonization-inhibition moieties thus remain in the circulation much longer than unmodified liposomes. For this reason, such liposomes are sometimes called "stealth" liposomes.

Stealth liposomes are known to accumulate in tissues fed by porous or "leaky" microvasculature. Thus, target tissue characterized by such microvasculature defects, for example, solid tumors, will efficiently accumulate these liposomes; see Gabizon et al., Proc Natl Acad Sci USA 1988, 18: 6949-53. In addition, the reduced uptake by the RES lowers the toxicity of stealth liposomes by preventing significant accumulation in liver and spleen. Thus, liposomes that are modified with opsonization-inhibition moieties can deliver the present double-stranded molecule to tumor cells.

Opsonization inhibiting moieties suitable for modifying liposomes are preferably water-soluble polymers with a molecular weight from about 500 to about 40,000 daltons, and more preferably from about 2,000 to about 20,000 daltons. Such polymers include polyethylene glycol (PEG) or polypropylene glycol (PPG) derivatives; e.g., methoxy PEG or PPG, and PEG or PPG stearate; synthetic polymers such as polyacrylamide or poly N-vinyl pyrrolidone; linear, branched, or dendrimeric polyamidoamines; polyacrylic acids; polyalcohols, e.g., polyvinylalcohol and polyxylitol to which carboxylic or amino groups are chemically linked, as well as gangliosides, such as ganglioside GM.sub.1. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, e.g., galacturonic acid, glucuronic acid, mannuronic acid, hyaluronic acid, pectic acid, neuraminic acid, alginic acid, carrageenan; aminated polysaccharides or oligosaccharides (linear or branched); or carboxylated polysaccharides or oligosaccharides, e.g., reacted with derivatives of carbonic acids with resultant linking of carboxylic groups.

Preferably, the opsonization-inhibiting moiety is a PEG, PPG, or derivatives thereof. Liposomes modified with PEG or PEG-derivatives are sometimes called "PEGylated liposomes".

The opsonization inhibiting moiety can be bound to the liposome membrane by any one of numerous well-known techniques. For example, an N-hydroxysuccinimide ester of PEG can be bound to a phosphatidyl-ethanolamine lipid-soluble anchor, and then bound to a membrane. Similarly, a dextran polymer can be derivatized with a stearylamine lipid-soluble anchor via reductive amination using Na(CN)BH₃ and a solvent mixture such as tetrahydrofuran and water in a 30:12 ratio at 60 degrees C.

Vectors expressing a double-stranded molecule are discussed above. Such vectors expressing at least one double-stranded molecule can also be administered directly or in conjunction with a suitable delivery reagent, including the Mirus Transit LT1 lipophilic reagent; lipofectin; lipofectamine; cellfectin; polycations (e.g., polylysine) or liposomes. Methods for delivering recombinant viral vectors, which express a double-stranded molecule of the present invention, to an area of cancer in a subject are within the skill of the art.

The double-stranded molecule can be administered to the subject by any means suitable for delivering the double-stranded molecule into cancer sites. For example, the double-stranded molecule can be administered by gene gun, electroporation, or by other suitable parenteral or enteral administration routes.

Suitable enteral administration routes include oral, rectal, or intranasal delivery.

Suitable parenteral administration routes include intravascular administration (e.g., intravenous bolus injection, intravenous infusion, intra-arterial bolus injection, intra-arterial infusion and catheter instillation into the vasculature); peri- and intra-tissue injection (e.g., peri-tumoral and intra-tumoral injection); subcutaneous injection or deposition including subcutaneous infusion (such as by osmotic pumps); direct application to the area at or near the site of cancer, for example, by a catheter or other placement device (e.g., a suppository or an implant including a porous, non-porous, or gelatinous material); and inhalation. It is preferred that injections or infusions of the double-stranded molecule or vector be given at or near the site of cancer.

The double-stranded molecule can be administered in a single dose or in multiple doses. Where the administration of the double-stranded molecule is by infusion, the infusion can be a single sustained dose or can be delivered by multiple infusions. Injection of the substance directly into the tissue is at or near the site of cancer preferred. Multiple injections of the substance into the tissue at or near the site of cancer are particularly preferred.

One skilled in the art can also readily determine an appropriate dosage regimen for administering the double-stranded molecule to a given subject. For example, the double-stranded molecule can be administered to the subject once, for example, as a single injection or deposition at or near the cancer site. Alternatively, the double-stranded molecule can be administered once or twice daily to a subject for a period of from about three to about twenty-eight days, more preferably from about seven to about ten days. In a preferred dosage regimen, the double-stranded molecule is injected at or near the site of cancer once a day for seven days. Where a dosage regimen includes multiple administrations, it is understood that the effective amount of a double-stranded molecule administered to the subject can include the total amount of a double-stranded molecule administered over the entire dosage regimen.

In the context of the present description, a cancer over-expressing the SUV39H2 gene can be treated with at least one active ingredient selected from the group consisting of:
(a) a double-stranded molecule of the present description,
(b) DNA encoding thereof, and
(c) a vector encoding thereof.

The cancers to be treated include, but are not limited to, lung cancer, cervical cancer, bladder cancer, esophageal cancer, osteosarcoma, prostate cancer and soft tissue tumor.

Accordingly, prior to the administration of the double-stranded molecule as active ingredient, it is preferable to confirm whether the expression level of the SUV39H2 gene in the cancer cells or tissues to be treated is enhanced as compared with normal cells of the same organ. Thus, in one embodiment, the present description provides a method for treating a cancer (over)expressing the SUV39H2, gene which method may include the steps of:
i) determining the expression level of the SUV39H2 gene in cancer cells or tissue(s) obtained from a subject with the cancer to be treated;
ii) comparing the expression level of the SUV39H2 gene with normal control; and
iii) administrating at least one component selected from the group consisting of
   (a) a double-stranded molecule of the present description,
   (b) DNA encoding said double-stranded molecule, and
   (c) a vector encoding said double-stranded molecule,
   to a subject with a cancer over-expressing the SUV39H2 gene compared with normal control. Alternatively, the present description also provides a pharmaceutical composition comprising at least one component selected from the group consisting of:
   (a) a double-stranded molecule of the present description,
   (b) DNA encoding said double-stranded molecule, and
   (c) a vector encoding said double-stranded molecule,
   for use in administrating to a subject having a cancer over-expressing the SUV39H2 gene. In other words, the present invention further provides a method for identifying a subject to be treated with:
   (a) a double-stranded molecule of the present description,
   (b) DNA encoding said double-stranded molecule, or
   (c) a vector encoding said double-stranded molecule,
which method may include the step of determining an expression level of the SUV39H2 gene in subject-derived cancer cells or tissue(s), wherein an increase of the level compared to a normal control level of the gene indicates that the subject has cancer which may be treated with a double-stranded molecule.

The method of treating a cancer is described in more detail below.

A subject to be treated by the present method is preferably a mammal. Exemplary mammals include, but are not limited to, e.g., human, non-human primate, mouse, rat, dog, cat, horse, and cow.

According to the present description, the expression level of the SUV39H2 gene in cancer cells or tissues obtained from a subject is determined. The expression level can be determined at the transcription (nucleic acid) product level, using methods known in the art. For example, hybridization methods (e.g., Northern hybridization), a chip or an array, probes, RT-PCR can be used to determine the transcription product level of the SUV39H2 gene.

Alternatively, the translation product may be detected for the treatment of the present description. For example, the quantity of observed protein (SEQ ID NO: 22, 24, 26, 28 or 30) may be determined.

As another method to detect the expression level of the SUV39H2 gene based on its translation product, the intensity of staining may be measured via immunohistochemical analysis using an antibody against the SUV39H2 protein. Namely, in this measurement, strong staining indicates increased presence/level of the protein and, at the same time, high expression level of the SUV39H2 gene.

Methods for detecting or measuring either of the SUV39H2 polypeptide or polynucleotide encoding thereof, or both can be exemplified as described above (A method for diagnosing cancer).

### Compositions containing a double-stranded molecule of the present description:

In addition to the above, the present description also provides pharmaceutical composition that include the present double-stranded molecule or the vector coding for the molecules.

In the context of the present description, the term "composition" is used to refer to a product including that include the specified ingredients in the specified amounts, as well as any product that results, directly or indirectly, from combination of the specified ingredients in the specified amounts. Such terms, when used in relation to the modifier "pharmaceutical" (as in "pharmaceutical composition"), are intended to encompass products including a product that includes the active ingredient(s), and any inert ingredient(s) that make up the carrier, as well as any product that results, directly or indirectly, from combination, complexation or aggregation of any two or more of the ingredients, or from dissociation of one or more of the ingredients, or from other types of reactions or interactions of one or more of the ingredients. Accordingly, in the context of the present description, the term "pharmaceutical composition" refers to any product made by admixing a molecule or compound of the present description and a pharmaceutically or physiologically acceptable carrier.

The phrase "pharmaceutically acceptable carrier" or "physiologically acceptable carrier", as used herein, means a pharmaceutically or physiologically acceptable material, composition, substance or vehicle, including but not limited to, a liquid or solid filler, diluent, excipient, solvent or encapsulating material.

The term "active ingredient" herein refers to a substance in composition that is biologically or physiologically active. Particularly, in the context of pharmaceutical composition, the term "active ingredient" refers to a substance that shows an objective pharmacological effect. For example, in case of pharmaceutical compositions for use in the treatment or prevention of cancer, active ingredients in the agents or compositions may lead to at least one biological or physiologically action on cancer cells and/or tissues directly or indirectly. Preferably, such action may include reducing or inhibiting cancer cell growth, damaging or killing cancer cells and/or tissues, and so on. Before being formulated, the "active ingredient" may also be referred to as "bulk", "drug substance" or "technical product".

The compositions are described in additional detail below. The double-stranded molecule is preferably formulated as pharmaceutical compositions prior to administering to a subject, according to techniques known in the art. Pharmaceutical composition is characterized as being at least sterile and pyrogen-free.

As used herein, "pharmaceutical composition" includes formulations for human and veterinary use.

In the context of the present description, suitable pharmaceutical formulations of the present invention include those suitable for oral, rectal, nasal, topical (including buccal, sub-lingual, and transdermal), vaginal or parenteral (including intramuscular, subcutaneous and intravenous) administration, or for administration by inhalation or insufflation. Other formulations include implantable devices and adhesive patches that release a therapeutic agent. When desired, the above-described formulations may be adapted to give sustained release of the active ingredient. Methods for preparing pharmaceutical compositions are within the skill known in the art, for example, as described in Remington's Pharmaceutical Science, 17th ed., Mack Publishing Company, Easton, Pa. (1985).

The present pharmaceutical composition contains the double-stranded molecule or vector encoding that (e.g., 0.1 to 90% by weight), or a physiologically acceptable salt of the molecule, mixed with a physiologically acceptable carrier medium. Preferred physiologically acceptable carrier media are water, buffered water, normal saline, 0.4% saline, 0.3% glycine, hyaluronic acid and the like.

According to the present description, the composition may contain plural kinds of the double-stranded molecules, each of the molecules may be directed to the same target sequence, or different target sequences of SUV39H2. For example, the composition may contain double-stranded molecules directed to the SUV39H2 gene or gene product. Alternatively, for example, the composition may contain double-stranded molecules directed to one, two or more target sequences of the SUV39H2 gene.

Furthermore, the present composition may contain a vector coding for one or plural double-stranded molecules. For example, the vector may encode one, two or several kinds of the present double-stranded molecules. Alternatively, the present composition may contain plural kinds of vectors, each of the vectors coding for a different double-stranded molecule.

Moreover, the present double-stranded molecule may be contained as liposomes in the present composition. See under the item of "Methods of Treating Cancer Using the Double-Stranded Molecules" for details of liposomes.

Pharmaceutical compositions can also include conventional pharmaceutical excipients and/or additives. Examples of suitable pharmaceutical excipients include stabilizers, antioxidants, osmolality adjusting agents, buffers, and pH adjusting agents. Suitable additives include physiologically biocompatible buffers (e.g., tromethamine hydrochloride), additions of chelants (such as, for example, DTPA or DTPA-bisamide) or calcium chelate complexes (for example, calcium DTPA, CaNaDTPA-bisamide), or, optionally, additions of calcium or sodium salts (for example, calcium chloride, calcium ascorbate, calcium gluconate or calcium lactate). Pharmaceutical compositions can be packaged for use in liquid form, or can be lyophilized.

For solid compositions, conventional nontoxic solid carriers can be used; for example, pharmaceutical grades of mannitol, lactose, starch, magnesium stearate, sodium saccharin, talcum, cellulose, glucose, sucrose, magnesium carbonate, and the like.

For example, a solid pharmaceutical composition for oral administration can include any of the carriers and excipients listed above and 10-95%, preferably 25-75%, of one or more double-stranded molecules. A pharmaceutical composition for aerosol (inhalational) administration can include 0.01-20% by weight, preferably 1-10% by weight, of one or more double-stranded molecules encapsulated in a liposome as described above, and propellant. A carrier can also be included as desired; e.g., lecithin for intranasal delivery.

In addition to the above, the present composition may contain other pharmaceutical active ingredients so long as they do not inhibit the in vivo function of the present double-stranded molecules. For example, the composition may contain chemotherapeutic agents conventionally used for treating cancers. The pharmaceutical compositions may also contain other active ingredients such as antimicrobial agents, immunosuppressants or preservatives. Furthermore, it should be understood that, in addition to the ingredients particularly mentioned above, the formulations may include other agents conventional in the art having regard to the type of formulation in question; for example, those suitable for oral administration may include flavoring agents.

In another embodiment, the present description also provides the use of the double-stranded nucleic acid molecule of the present description or a vector encoding the double-stranded nucleic acid molecule in manufacturing a pharmaceutical composition for treating a cancer characterized by the expression of the SUV39H2 gene. For example, the present description
relates to use of double-stranded nucleic acid molecule inhibiting the expression of the SUV39H2 gene in a cell, which molecule includes a sense strand and an antisense strand complementary thereto, hybridized to each other to form the double-stranded nucleic acid molecule and target to a sequence of SEQ ID NO: 19 or 20, or a vector encoding the double-stranded nucleic acid molecule for manufacturing a pharmaceutical composition for treating cancer expressing the SUV39H2 gene.

The present description further provides the double-stranded nucleic acid molecules or a vector encoding the double-stranded nucleic acid molecule for use in treating a cancer expressing the SUV39H2 gene.

The present description further provides a method or process for manufacturing a pharmaceutical composition for either or both of treating and preventing cancers characterized by the expression of the SUV39H2 gene, wherein the method or process includes a step for formulating a pharmaceutically or physiologically acceptable carrier with a double-stranded nucleic acid molecule inhibiting the expression of the SUV39H2 gene in a cell, which over-expresses the gene, which molecule includes a sense strand and an antisense strand complementary thereto, hybridized to each other to form the double-stranded nucleic acid molecule and target to a sequence of SEQ ID NO: 19 or 20 or a vector encoding the double-stranded nucleic acid molecule as active ingredients.

In another embodiment, the present description also provides a method or process for manufacturing a pharmaceutical composition for either or both of treating and preventing cancers characterized by the expression of the SUV39H2 gene, wherein the method or process includes a step for admixing an active ingredient with a pharmaceutically or physiologically acceptable carrier, wherein the active ingredient is a double-stranded nucleic acid molecule inhibiting the expression of the SUV39H2 gene in a cell, which over-expresses the gene, which molecule includes a sense strand and an antisense strand complementary thereto, hybridized to each other to form the double-stranded nucleic acid molecule and targets to a sequence of SEQ ID NO: 19 or 20 or a vector encoding the double-stranded nucleic acid molecule.

Hereinafter, the present description is described in more detail with reference to the Examples. However, the following materials, methods and examples only illustrate aspects of the present description and in no way are intended to limit the scope of the present invention. As such, methods and materials similar or equivalent to those described herein can be used in the practice or testing of the present description.

### Examples

The present invention will be further described in the following examples, which do not limit the scope of the present invention described in the claims.

### Example 1: General Methods

### Tissue samples and RNA preparation

Bladder tissue samples and RNA preparation have been previously described [ Wallard MJ, et al, Br J Cancer 2006; 94: 569-577]. Briefly, 125 surgical specimens of primary urothelial carcinoma were collected, either through cystectomy or transurethral resection of bladder tumor (TURBT), and snap frozen in liquid nitrogen. Twenty eight specimens of normal bladder urothelial tissue were collected from areas of macroscopically normal bladder urothelium in patients with no evidence of malignancy. Vimentin is primarily expressed in mesenchymally derived cells, and was used as a stromal marker. Uroplakin is a marker of urothelial differentiation and is preserved in up to 90% of epithelially derived tumors [Olsburgh J et al, J Pathol 2003; 199: 41-49]. Use of tissues for this study was approved by Cambridge shire Local Research Ethics Committee (Ref 03/018).

### Cell culture

Cancer cell lines used in this study were as follows: lung adenocarcinoma (ADC), NCI-H1781, NCI-H1373, LC319, A549 and PC-14; lung squamous cell carcinoma (SCC) SK-MES-1, NCI-H2170, NCI-H520, NCI-H1703 and RERF-LC-AI; lung large cell carcinoma (LCC) LX1; small cell lung cancer (SCLC) SBC-3, SBC-5, DMS273 and DMS114; bladder cancer SW780, RT4 and SCaBER. Human small airway epithelial cells (SAEC), normal human fibroblasts (IMR-90, WI-38 and CCD-18Co) were used as normal control cells. All cell lines were grown in monolayers in appropriate media supplemented with 10% fetal bovine serum and 1% antibiotic/antimycotic solution (Sigma). All cells were maintained at 37 degrees C in humid air with 5% CO₂ (all cell lines except for SW780), or without CO₂ (SW780). Cells were transfected with FuGENE6 (ROCHE, Basel, Switzerland) according to the manufacturer's protocols.

### Expression profiling in cancer using cDNA microarrays

A genome-wide cDNA microarray was established with 36,864 cDNAs selected from the UniGene database of the National Center for Biotechnology Information (NCBI). This microarray system was constructed essentially as described previously [Kitahara O et al, Cancer Res 2001; 61: 3544-3549]. Briefly, the cDNAs were amplified by RT-PCR using poly (A)⁺ RNAs isolated from various human organs as templates; the lengths of the amplicons ranged from 200 to 1100 bp, without any repetitive or poly (A) sequences. Many types of tumor and corresponding non-neoplastic tissues were prepared in 8-micrometer sections, as described previously [Kitahara O et al, Cancer Res 2001; 61: 3544-3549]. A total of 30,000-40,000 cancer or noncancerous cells were collected selectively using the EZ cut system (SL Microtest GmbH, Germany) according to the manufacturer's protocol. Extraction of total RNA, T7-based amplification, and labeling of probes were performed as described previously [Kitahara O et al, Cancer Res 2001; 61: 3544-3549]. A measure of 2.5-microgram aliquots of twice-amplified RNA (aRNA) from each cancerous and non cancerous tissue was then labeled respectively with Cy3-dCTP or Cy5-dCTP.

### Quantitative real-time PCR

As described previously, 125 bladder cancer and 28 normal bladder tissues were prepared in Cambridge Addenbrooke's Hospital. For quantitative RT-PCR reactions, specific primers for all GAPDH (housekeeping gene), SDH (housekeeping gene) and SUV39H2 were designed (Primer sequences in Table 1) [Yoshimatsu M et al, Int J Cancer 2011; 128: 562-573]. PCR reactions were performed using the LightCycler (registered trademark) 480 System (Roche Applied Science, Mannheim, Germany) following the manufacture's protocol. 50% SYBR GREEN universal PCR Master Mix without UNG (Applied Biosystems, Warrington, UK), 50 nM each of the forward and reverse primers and 2 microliter of reversely-transcribed cDNA were applied. Amplification conditions were 5 min at 95 degrees C and then 45 cycles each consisting of 10 sec at 95 degrees C, 1 min at 55 degrees C and 10 sec at 72 degrees C. Then, reactions were heated for 15 sec at 95 degrees C, 1 min at 65 degrees C to draw the melting curve, and cooled to 50 degrees C for 10 sec. Reaction conditions for target gene amplification were as described above and the equivalent of 5 ng of reverse transcribed RNA was used in each reaction. mRNA levels were normalized to GAPDH and SDH expression.

**[Table 1]**

| **Primer sequences for quantitative RT-PCR** | |
|---|---|
| **Gene name** | **Primer sequence** |
| *GAPDH* (housekeeping gene) - f | 5' GCAAATTCCATGGCACCGTC 3' (SEQ ID NO: 1) |
| *GAPDH* (housekeeping gene) - r | 5' TCGCCCCACTTGATTTTGG 3' (SEQ ID NO: 2) |
| *SDH* (housekeeping gene) - f | 5' TGGGAACAAGAGGGCATCTG 3' (SEQ ID NO: 3) |
| *SDH* (housekeeping gene) - r | 5' CCACCACTGCATCAAATTCATG 3' (SEQ ID NO: 4) |
| *SUV39H2* - f | 5' TGGGGTGTAAAGACCCTTGTG 3' (SEQ ID NO: 5) |
| *SUV39H2* - r | 5' ATTCCCTTGTTGTCATAGAAC 3' (SEQ ID NO: 6) |

### Immunohistochemistry

Immunohistochemistry analysis was performed using a specific polyclonal rabbit-SUV39H2 antibody produced by SIGMA GENOSYS as described previously [Takawa M, et al. Cancer Sci 2011;102:1298-305, Toyokawa G, et al. Neoplasia 2011;13:887-98, Toyokawa G, et al. Mol Cancer 2011;10:65]. For all tissue samples, EnVision+ kit/horseradish peroxidase (Dako, Glostrup, Denmark) was applied. Briefly, slides of paraffin-embedded tumor specimens were processed under high pressure (125 degrees C, 30 s) in antigen-retrieval solution, high pH 9 (S2367, Dako Cytomation, Carpinteria, CA, USA), treated with peroxidase blocking regent, and then treated with protein blocking regent (K130, X0909, Dako Cytomation). Tissue sections were incubated with a rabbit anti-SUV39H2 polyclonal antibody followed by HRP-conjugated secondary antibody (Dako Cytomation). Antigen was visualized with substrate chromogen (Dako liquid DAB chromogen; Dako Cytomation). Finally, tissue specimens were stained with Mayer's haematoxylin (Muto pure chemicals Ltd, Tokyo, Japan, Hematoxylin QS, Vector Laboratories) for 20 s to discriminate the nucleus from the cytoplasm. Because the intensity of staining within each tumor tissue core was mostly homogeneous, the intensity of SUV39H2 staining was semiquantitatively evaluated using the following criteria: negative (no appreciable staining in tumor cells) and positive (brown staining appreciable in more than 30% of the nucleus of tumor cells).

### Immunocytochemistry

HeLa cells were transfected with pCAGGS-n3FC-SUV39H2 (3xFLAG-SUV39H2). 48 hours after transfection, cultured cells were fixed by 4 % paraformaldehyde in 0.1 M phosphate buffer (pH 7.4) at room temperature for 30 minutes and permeabilized with 0.5 % Triton X-100 in PBS (Sigma). Fixed cells were blocked with 5 % BSA in PBS for 1 hour and incubated with a monoclonal mouse-FLAG antibody overnight at 4 degrees C. Then, they were incubated with Alexa Fluor 594 conjugated second antibody (Molecular Probes) and Alexa Fluor 488 Phalloidin (Molecular Probes). Nuclei were stained with 4',6-diamidino-2-phenylindole (DAPI). The stained cells were observed using a Leica confocal microscopy.

### siRNA transfection

siRNA oligonucleotide duplexes were purchased from SIGMA Genosys for targeting the human SUV39H2 transcripts. siEGFP, siFFLuc and siNegative control (siNC), which is a mixture of three different oligonucleotide duplexes, were used as control siRNAs. The siRNA sequences are described in Table 2. siRNA duplexes (100 nM final concentration) were transfected into lung and bladder cancer cell lines with Lipofectamine 2000 (Invitrogen) for 72 hours, and cell viability was examined using the Cell Counting Kit-8 (Dojindo, Kumamoto, Japan) and colony formation assay as described previously [Sato N et al, Cancer Res 2010; 70: 5326-5336].

**[Table 2]**

| **siRNA sequences** | | | |
|---|---|---|---|
| **siRNA name** | **Sequence** | | **SEQ ID NO** |
| siEGFP | | Sense: 5' GCAGCACGACUUCUUCAAGTT 3' | 7 |
| | | Antisense: 5' CUUGAAGAAGUCGUGCUGCTT 3' | 8 |
| siNegative control (Cocktail) | Target#1 | Sense: 5' AUCCGCGCGAUAGUACGUA 3' | 9 |
| | | Antisense: 5' UACGUACUAUCGCGCGGAU 3' | 10 |
| | Target#2 | Sense: 5' UUACGCGUAGCGUAAUACG 3' | 11 |
| | | Antisense: 5' CGUAUUACGCUACGCGUAA 3' | 12 |
| | Target#3 | Sense: 5' UAUUCGCGCGUAUAGCGGU 3' | 13 |
| | | Antisense: 5'ACCGCUAUACGCGCGAAUA 3' | 14 |
| siSUV39H2#1 | | Target: 5' CUUUGGUUGUUCAUGCACA 3' | 19 |
| | | Sense: 5' CUUUGGUUGUUCAUGCACATT 3' | 15 |
| | | Antisense: 5' UGUGCAUGAACAACCAAAGTT 3' | 16 |
| siSUV39H2#2 | | Target: 5' CUGGAAUCAGCUUAGUCAA 3' | 20 |
| | | Sense: 5' CUGGAAUCAGCUUAGUCAATT 3' | 17 |
| | | Antisense: 5' UUGACUAAGCUGAUUCCAGTT 3' | 18 |

### Flow cytometry assay (FACS)

SW780 and A549 cells were treated with siSUV39H2 (siSUV39H2#1 and siSUV39H2#2) or control siRNAs (siEGFP and siNC), and cultured in a CO₂ incubator at 37 degrees C for 72 hours. Aliquots of 1 X 10⁵ cells were collected by trypsinization, and stained with propidium iodide (PI) following the manufacturer's instructions (Cayman Chemical, Ann Arbor, MI). Cells were analyzed by FACScan (BECKMAN COULTER, Brea, CA) with MultiCycle for Windows software (BECKMAN COULTER) for detailed cell cycle status. The percentages of cells in G₀ / G₁, S and G₂/M phases of the cell cycle were determined from at least 20,000 ungated cells.

### Coupled cell cycle and cell proliferation assay

A 5'-bromo-2'-deoxyuridine (BrdU) flow kit (BD Pharmingen, San Diego, CA) was used to determine the cell cycle kinetics and to measure the incorporation of BrdU into DNA of proliferating cells. The assay was performed according to the manufacturer's protocol. Briefly, MEFs (1 x 10⁵ per well) were seeded in 6-well tissue culture plates for 72 hours, followed by addition of 10 micromolar BrdU, and incubations continued for an additional 30 min. Both floating and adherent cells were pooled from triplicate wells per treatment point, fixed in a solution containing paraformaldehyde and the detergent saponin, and incubated for 1 hour with DNase at 37 degrees C (30 microgram per sample). FITC-conjugated anti-BrdU antibody (1:50 dilution in Wash buffer; BD Pharmingen, San Diego, CA) was added and incubation continued for 20 min at room temperature. Cells were washed in Wash buffer and total DNA was stained with 7-amino-actinomycin D (7-AAD; 20 microliter per sample), followed by flow cytometric analysis using FACScan (BECKMAN COULTER) and total DNA content (7-AAD) was determined CXP Analysis Software Ver. 2.2 (BECKMAN COULTER).

### Microarray hybridization and statistical analysis for the clarification of down-stream genes

Microarray analysis to identify down-stream genes and Gene Ontology pathway analysis were performed as described previously [Yoshimatsu M et al, Int J Cancer 2011; 128: 562-573]. Purified total RNA was labeled and hybridized onto Affymetrix GeneChip U133 Plus 2.0 oligonucleotide arrays (Affymetrix, Santa Clara, CA) according to the manufacturer's instructions. Probe signal intensities were normalized by RNA and Quantile (using R and Bioconductor). A pathway analysis was performed using the using the hypergeometric distribution test, which calculates the probability of overlap between the up/down-regulated gene set and each GO category compared against another gene list that is randomly sampled. The test to the identified up/ down-regulated genes was applied to test whether or not they are significantly enriched (FDR <= 0.05) in each category of 'biological processes' (857 categories) as defined by Gene Ontology database.

### Example 2: Aberrant expression of SUV39H2 in clinical lung and bladder cancer tissues

To analyze expression levels of SUV39H2 in clinical samples, qRT-PCR was performed using 16 normal and 14 lung cancer tissues (9 NSCLC cases and 5 SCLC cases), and found that SUV39H2 is significantly up-regulated in lung cancer tissues relative to normal tissues (Figures 1A and 1B). Among normal tissues, only testis showed high expression levels of SUV39H2 comparable to those of lung cancer tissues. Immunohistochemical analysis was conducted to evaluate protein expression levels of SUV39H2 and observed no significant staining in several normal tissues except testis (Figure 1C). Subsequent tissue microarray analysis indicated that SUV39H2 was stained positively in 34 out of 64 lung cancer cases (53.1%; Figure 2 and Table 3) and observed no significant staining in normal vital organs (Figure 2C). These results imply that SUV39H2 is frequently over-expressed in lung cancer tissues, whereas its expression in normal tissues including vital organs appears vanishingly low. To analyze the association of SUV39H2 expression with clinical outcomes, the present inventors performed tumor tissue microarray on arrays containing 328 archival NSCLC cases (Figure 10). SUV39H2 stained positively in 217 cases (66.16%) and negatively in 111 cases (33.84%). Meanwhile, no significant statistical significance was observed between SUV39H2-positivity and any patients' characteristics (Table 11).

**[Table 3]**

| **Clinicopathologic characteristics of lung tissues on the tissue microarray*** | | | | | | |
|---|---|---|---|---|---|---|
| **Case No.** | **Age** | **Gender** | **Histology** | **Differentiation** | **Stage (TNM)** | **SUV39H2 expression** ^{±±} |
| A1 | 29 | F | Human Normal Placenta | | | - |
| A2 | | | | | | - |
| A3 | 60 | M | Pulmonary metastases renal cell carcinoma | Moderately | T2NxM1 | + |
| A4 | N/A | N/A | Adenocarcinoma | | T0NxMx | + |
| A5 | N/A | N/A | Squamous cell carcinoma | | T0NxMx | - |
| A6 | 60 | M | Squamous cell carcinoma | Poorly | T2N0M0 | - |
| A7 | 47 | F | Adenocarcinoma | Poorly | T2N0M0 | - |
| A8 | 53 | F | Squamous cell carcinoma | Moderately | T0N0M0 | - |
| A9 | 40 | M | Squamous cell carcinoma | Moderately | T2N0M0 | + |
| A10 | 56 | F | Adenocarcinoma | Poorly | T2N0M0 | + |
| A11 | 49 | M | Squamous cell carcinoma | Moderately | T2N0M0 | + |
| B1 | 45 | F | Bronchio alveolar carcinoma | N/A | T2N0M0 | - |
| B2 | 34 | F | Fibrosarcoma | Moderately | T0N0M0 | - |
| B3 | 50 | M | Bronchio alveolar carcinoma | N/A | T3N0M0 | - |
| B4 | 57 | M | Squamous cell carcinoma | Poorly | T2N0M0 | + |
| B5 | 65 | M | Atypical Carcinoma, (central type) | Moderately | T3N0M0 | + |
| B6 | 36 | F | Adenocarcinoma, mucous | Well | T2N0M0 | - |
| B7 | 57 | M | Squamous cell carcinoma | Moderately | T2N0M0 | + |
| B8 | 29 | M | Squamous cell carcinoma | Moderately | T2N0M0 | + |
| B9 | 52 | M | Undifferentiated small cell carcinoma | Poorly | T2N0M0 | - |
| B10 | 63 | M | Squamous cell carcinoma (cornifying) | Moderately | T3N0M0 | + |
| B11 | 68 | M | Adenocarcinoma, papillary (peripheral type) | Well | T2N1M0 | + |
| C1 | 57 | M | Squamous cell carcinoma, (center type) | Well | T2N0M0 | - |
| C2 | 56 | F | Tuberculosis | N/A | T1N0M0 | - |
| C3 | 52 | M | Squamous cell carcinoma | Moderately | T2N0M0 | + |
| C4 | 46 | M | Squamous cell carcinoma, (cornifying) | Well | T3N0M0 | + |
| C5 | 58 | M | Squamous cell carcinoma, (central type) | Moderately | T2N1M0 | + |
| C6 | 63 | M | Adenocarcinoma | Moderately | T3N0M0 | - |
| C7 | 61 | F | Bronchio alveolar carcinoma | Well | T2N0M0 | - |
| C8 | 40 | M | Squamous cell carcinoma | Well | T3N1M0 | + |
| C9 | 64 | M | Squamous cell carcinoma | Moderately | T3N0M0 | - |
| C10 | 44 | F | Adenosqumous carcinoam | Moderately | T2N1M0 | - |
| C11 | 61 | M | Squamous cell carcinoma | Well | T2N0M0 | - |
| D1 | 65 | F | Squamous cell carcinoma | Poorly | T1N0M0 | + |
| D2 | 64 | F | Adenocarcinoma, papillary (peripheral type) | Well | T2N0M0 | - |
| D3 | 70 | M | Adenosquamous carcinoma | Moderately | T2N1M0 | - |
| D4 | 68 | M | Undifferentiated small cell carcinoma | Poorly | T2N0M0 | - |
| D5 | 65 | M | Carcinoma, (peripheral type) | Moderately | T2N0M0 | - |
| D6 | 59 | F | Adenocarcinoma, papillary | Well | T2N0M0 | - |
| D7 | 67 | M | Squamous cell carcinoma | Moderately | T2N0M0 | - |
| D8 | 70 | M | Squamous cell carcinoma | Poorly | T2N0M0 | + |
| D9 | 47 | F | Adenocarcinoma | Moderately | T2N0M0 | - |
| D10 | 71 | M | Squamous cell carcinoma | Moderately | T2N0M0 | + |
| D11 | 65 | M | Squamous cell carcinoma | Moderately | T2N0M0 | + |
| E1 | 68 | M | Adenocarcinoma, squamous cell carcinoma | Moderately | T3N0M0 | + |
| E2 | 47 | F | Large cell Carcinoma | Moderately | T2N0M0 | + |
| E3 | 39 | F | Adenocarcinoma | Moderately | T2N1M0 | - |
| E4 | 67 | M | Squamous cell carcinoma | Moderately | T2N1M0 | + |
| E5 | 60 | F | Alveolus cell carcinoma | N/A | T2N0M0 | - |
| E6 | 70 | F | Carcinoma | Moderately | T1N0M0 | - |
| E7 | 27 | M | metastasis tumor Sarcoma | Moderately | T2NxM1 | - |
| E8 | 65 | M | Squamous cell carcinoma | Moderately | T3N0M0 | + |
| E9 | 68 | F | Squamous cell carcinoma | Moderately | T2N0M0 | + |
| E10 | 58 | F | Adenocarcinoma | Moderately | T2N1M0 | + |
| E11 | 68 | M | Squamous cell carcinoma | Well | T2N0M0 | + |
| F1 | 48 | M | Squamous cell carcinoma | Moderately | T3N0M0 | + |
| F2 | 59 | M | Squamous cell carcinoma | N/A | T1N0M0 | + |
| F3 | 54 | M | Adenocarcinoma, cyst | Moderately | T2N1M0 | - |
| F4 | 45 | M | Squamous cell carcinoma | Moderately | T3N0M0 | + |
| F5 | 69 | M | Squamous cell carcinoma | Poorly | T2N1M0 | + |
| F6 | 78 | F | Alveolus cell adenocarcinoma | Moderately | T1N0M0 | + |
| F7 | 60 | M | Adenocarcinoma | Moderately | T1N0M0 | - |
| F8 | 54 | F | Alveolus cell carcinoma | Moderately | T2N1M0 | + |
| F9 | 78 | M | Alveolus cell carcinoma | Moderately | T1N0M0 | + |
| F10 | 70 | M | Alveolus cell carcinoma | Well | T1N0M0 | + |
| F11 | 45 | F | Bronchio alveolar carcinoma | Moderately | T2N0M0 | - |

| | | | | | | |
|---|---|---|---|---|---|---|
| *The tissue microarray was purchased from BioChain **(-) negative expression; (+) positive expression | | | | | | |

**[Table 11]**

| **Association between SUV39H2-positivity in NSCLC tissues and patients' characteristics (n = 328)** | | | | | |
|---|---|---|---|---|---|
| | **Total** | **SUV39H2 expression** | | | ***P* value** |
| | | **Strong expression** | **Low expression** | **Absent expression** | **Strong vs Low or absent** |
| | **n = 328** | **n = 130** | **n = 87** | **n = 111** | |
| Gender | | | | | |
| Female | 97 | 40 | 26 | 31 | 0.7121 |
| Male | 231 | 90 | 61 | 80 | |
| Age(year) | | | | | |
| < 65 | 148 | 64 | 41 | 43 | 0.2569 |
| >=65 | 180 | 66 | 46 | 68 | |
| Smoking status | | | | | |
| never smoker | 89 | 37 | 19 | 33 | 0.7040 |
| current or ex-smoker | 239 | 93 | 68 | 78 | |
| Histological type | | | | | |
| ADC | 197 | 73 | 47 | 77 | 02512 |
| non-ADC | 131 | 57 | 40 | 34 | |
| T factor | | | | | |
| T1 | 131 | 58 | 33 | 40 | 0.1685 |
| T2+T3 | 197 | 72 | 54 | 71 | |
| N factor | | | | | |
| N0 | 214 | 86 | 63 | 65 | 0.8133 |
| N1+N2 | 114 | 44 | 24 | 46 | |

| | | | | | |
|---|---|---|---|---|---|
| **P* < 0.05 (Fisher's exact test) ADC, adenocarcinoma non-ADC, squamous cell carcinoma, large cell carcinoma and adenosquamous cell carcinoma | | | | | |

by qRT-PCR and found significant elevation of SUV39H2 in cancer tissues compared with nonneoplastic bladder tissues (P < 0.05, Mann-Whitney U test; Figure 3A). Sub-classification of tumors according to tumor grade, metastasis status, gender, recurrence status and smoking history identified no significant differences (Table 4). The cDNA expression analysis of Japanese bladder clinical cases also identified SUV39H2 over-expression (Figure 3B). Then, SUV39H2 protein expression levels in bladder tissues were examined by immunohistochemistry and observed strong SUV39H2 staining in the nucleus of cancer tissues but no significant staining in non-neoplastic tissues (Figure 3C). Tissue microarray analysis showed that over-expression of SUV39H2 was observed in 16 out of 29 bladder cancer cases (55.2%; Table 5). Furthermore, the microarray expression analysis using a large number of clinical samples indicated that SUV39H2 expression was significantly up-regulated in various types of cancer besides lung and bladder cancers (Table 6). The data suggest that elevated expression of SUV39H2 is frequently observed in human carcinogenesis.

**[Table 4]**

| **Statistical analysis of *SUV39H2* expression levels in clinical bladder tissues** | | | | |
|---|---|---|---|---|
| | | **SUV39H2** | | |
| **Characteristic** | **Case (n)** | **Mean** | **SD** | **95%CI** |
| **Normal (Control)** | 28 | 0.226 | 0.141 | 0.174 - 0.278 |
| **Tumor (Total)** | 124 | 0.509 | 0.648 | 0.395 - 0.623 |
| Tumor stage | | | | |
| pTa, pT1 | 88 | 0.540 | 0.723 | 0.388 - 0.691 |
| pT2 | 25 | 0.373 | 0.346 | 0.237 - 0.509 |
| pT3, pT4 | 7 | 0.556 | 0.488 | 0.194 - 0.917 |
| Tumor grade | | | | |
| G1 | 12 | 0.302 | 0.274 | 0.147 - 0.457 |
| G2 | 62 | 0.512 | 0.644 | 0.352 - 0.673 |
| G3 | 49 | 0.559 | 0.721 | 0.357 - 0.761 |
| Metastasis | | | | |
| Negative | 97 | 0.523 | 0.710 | 0.381 - 0.664 |
| Positive | 27 | 0.459 | 0.350 | 0.327 - 0.591 |
| Gender | | | | |
| Male | 91 | 0.573 | 0.729 | 0.423 - 0.722 |
| Female | 31 | 0.326 | 0.270 | 0.231 - 0.421 |
| Recurrence | | | | |
| No | 27 | 0.443 | 0.335 | 0.317 - 0.570 |
| Yes | 51 | 0.495 | 0.588 | 0.334 - 0.657 |
| Died | 8 | 0.729 | 0.896 | 0.108 - 1.350 |
| Smoke | | | | |
| No | 27 | 0.494 | 0.566 | 0.281 - 0.708 |
| Yes | 49 | 0.449 | 0.513 | 0.305 - 0.593 |

**[Table 5]**

| **Clinicopathologic characteristics of bladder tissues on the tissue microarray*** | | | | | | |
|---|---|---|---|---|---|---|
| **Case No.** | **Age** | **Gender** | **Histology** | **Grade** | **Stage (TNM)** | **SUV39H2 expression**** |
| **1** | 71 | M | Squamous cell carcinoma | I | T1N0M0 | + |
| 2 | 60 | M | Squamous cell carcinoma | I | T2N0M0 | + |
| 3 | 76 | M | Adenocarcinoma | II | T2N0M0 | + |
| 4 | 50 | M | Adenocarcinoma | II | T2N0M0 | + |
| 5 | 68 | M | Adenocarcinoma | III | T2N0M0 | - |
| 6 | 74 | F | Adenocarcinoma | III | T2N0M0 | + |
| 7 | 27 | M | Transitional cell carcinoma | I | TisN0M0 | + |
| 8 | 50 | M | Transitional cell carcinoma | I | T1N0M0 | - |
| 9 | 49 | F | Transitional cell carcinoma | I | T1N0M0 | + |
| 10 | 67 | M | Transitional cell carcinoma | I | T1N0M0 | - |
| 11 | 51 | F | Transitional cell carcinoma | I | T1N0M0 | + |
| 12 | 57 | M | Transitional cell carcinoma | I | T1N0M0 | + |
| 13 | 47 | M | Transitional cell carcinoma | II | T2N0M0 | + |
| 14 | 54 | M | Transitional cell carcinoma | II | T2N0M0 | + |
| 15 | 45 | M | Transitional cell carcinoma | II | T1N0M0 | + |
| 16 | 74 | M | Transitional cell carcinoma | II | T2N0M0 | - |
| 17 | 51 | M | Transitional cell carcinoma | II | T1N0M0 | - |
| 18 | 80 | M | Transitional cell carcinoma | II | T2N0M0 | - |
| 19 | 53 | F | Transitional cell carcinoma | II | T1N0M0 | - |
| 20 | 37 | M | Transitional cell carcinoma | II | T2N0M0 | - |
| 21 | 55 | M | Transitional cell carcinoma | II | T4N2MX | - |
| 22 | 52 | M | Transitional cell carcinoma | II | T1N0M0 | + |
| 23 | 78 | M | Transitional cell carcinoma | III | T1N0M0 | + |
| 24 | 64 | M | Transitional cell carcinoma | III | T3N2M1 | + |
| 25 | 70 | M | Transitional cell carcinoma | III | T2N0M0 | - |
| 26 | 61 | M | Transitional cell carcinoma | III | T2N0M0 | + |
| 27 | 61 | M | Transitional cell carcinoma | III | T1N0M0 | - |
| 28 | 39 | F | Transitional cell carcinoma | III | T2N0M0 | - |
| 29 | 30 | M | Sarcoma | - | T2N0M0 | - |

| | | | | | | |
|---|---|---|---|---|---|---|
| *The tissue microarray was purchased from BioChain **(-) negative expression; (+) positive expression | | | | | | |

**[Table 6]**

| **Gene expression profile of *SUV39H2* in cancer tissues analyzed by cDNA microarray*** | | | | |
|---|---|---|---|---|
| | | **Ratio (tumor/normal)** | | |
| **Tissue type** | **Case (n)** | **Count > 2 (T/N)** | **Count > 3 (T/N)** | **Count > 5 (T/N)** |
| NSCLC | 3 | 3 (100 %) | 3 (100 %) | 2 (66.7 %) |
| SCLC | 3 | 3 (100 %) | 3 (100 %) | 3 (100 %) |
| Cervical cancer | 12 | 12 (100 %) | 12 (100 %) | 11 (91.7 %) |
| Bladder cancer | 13 | 6 (46.2 %) | 4 (30.8 %) | 4 (30.8 %) |
| Esophageal cancer | 10 | 7 (70 %) | 4 (40 %) | 4 (40 %) |
| Osteosarcoma | 5 | 5 (100 %) | 3 (60 %) | 1 (20 %) |
| Prostate cancer | 18 | 12 (66.7 %) | 8 (44.4 %) | 7 (38.9 %) |
| Soft tissue tumor | 7 | 3 (42.9 %) | 2 (28.6 %) | 2 (28.6 %) |

| | | | | |
|---|---|---|---|---|
| Abbreviations: NSCLC, non-small cell lung cancer; SCLC, small cell lung cancer * We compared the signal intensity of *SUV39H2* in tumourtissues with corresponding non-neoplastic tissues derived from the same patient. | | | | |

### Example 3: SUV39H2 is crucial for cancer cell proliferation

The role of SUV39H2 in cancer cell proliferation was examined. qRT-PCR was performed to measure expression levels of SUV39H2 in various cell lines at the RNA level, and confirmed elevated SUV39H2 expression in lung and bladder cancer cells compared with non-cancerous cells (Figure 4A and Figure 6). Over-expression of SUV39H2 protein in cancer cells was confirmed by Western blotting (Figure 4B). In order to examine the effects of SUV39H2 knockdown on the growth of cancer cells, two independent siRNAs targeting SUV39H2 were prepared and were transfected each of them into cancer cells. Then, the present inventors confirmed significant suppression of SUV39H2 expression after treatment with SUV39H2 siRNAs compared with control siRNAs (siEGFP and siNC; Figure 4C). Cell growth assays performed on cancer cells transfected with these siRNAs, revealed significant growth suppression in the lung cancer cell line A549 and the bladder cancer cell line SW780 (Figure 4D). When these siRNAs were transfected into the normal cell line CCD-18Co cells, expressing undetectable levels of SUV39H2 (Figure 4A), they had no effect on growth (Figure 4D). This implies that suppression of cancer cell growth by treatment with these specific siRNAs does not deploy a mechanism used by normal cells, and thus directly acts on a growth mechanism specific to cancer cells. The growth suppression of cancer cells by siRNAs targeting SUV39H2 was confirmed by colony formation assay (Figure 4E and Figure 7A). To examine whether SUV39H2 possesses oncogenic activity, the present inventors conducted a clonogenicity assay. A wild-type SUV39H2 (SUV39H2 Wt) vector and an enzyme-dead SUV39H2 (SUV39H2 delta-SET) vector were transfected into COS-7 cells together with a mock vector as a control. A clonogenicity assay was performed on each culture (Figure 4F). Cells transfected with a wild-type SUV39H2 vector formed more colonies than those transfected with an enzyme-dead SUV39H2 vector or a mock control vector, therefore it is the methylation activity of SUV39H2 that promotes oncogenesis in cells. Because SUV39H2 is over-expressed at an early stage in cancer progression, SUV39H2 may play a crucial role in human carcinogenesis. To further assess the mechanism of growth suppression induced by the siRNA, FACS analysis was performed to examine the detailed cell cycle status of cancer cells. The proportion of cancer cells at G₁ phase was significantly higher in the cells treated with siSUV39H2 compared to those treated with control siRNAs, while the proportion of cells at S phase was markedly decreased (Figure 5). These data indicate that SUV39H2 plays a critical role in the growth regulation of cancer cells, especially at the G₁/S transition.

### Example 4: Identification of downstream genes of SUV39H2 by microarray expression analysis

In order to identify how SUV39H2 might contribute to human carcinogenesis, the present inventors looked for downstream genes and pathways associated with SUV39H2. Twenty-four hours after SUV39H2 siRNA treatment in SW780 and A549 cells, microarray hybridization analysis was performed as described previously [Yoshimatsu M,et al, Int J Cancer 2011; 128: 562-573]. Expression profiles were analyzed by Affymetrix's HG-U133 Plus 2.0 Array and compared with profiles from cells treated with control siRNAs (siEGFP and siFFLuc). The data showed 146 genes to be downregulated whereas 64 genes were upregulated after knockdown of SUV39H2: these 210 genes were considered to be downstream genes regulated by SUV39H2 inhibition (Figure 8, Table 7 and Table 8). Signal pathway analysis of the downstream candidates by the Gene Ontology database suggested that SUV39H2 modulates the pathways for cell cycle regulation and chromatin modification (Table 9). Importantly, mass spectrometric analysis identified proteins involved in cell cycle regulation and chromatin functions as interacting partners of SUV39H2 (Table 10). Thus, deregulation of SUV39H2 is likely to contribute to human carcinogenesis, in part through regulating histone and chromatin functions.

**[Table 7]**

| **Downstream genes up-regulated by the inhibition of SUV39H2 expression** | | | |
|---|---|---|---|
| | Ratio | Gene | Symbol |
| 1 | 5.097 | IFI6(IFI6) | 204415_at |
| 2 | 3.427 | SP110(SP110) | 209761_s_at |
| 3 | 2.893 | PTX3(PTX3) | 206157_at |
| 4 | 2.668 | NHLRC3(NHLRC3) | 227040_at |
| 5 | 2.602 | IL11(IL11) | 206924_at |
| 6 | 2.423 | RGS2(RGS2) | 202388_at |
| 7 | 2.353 | RAB39B(RAB39B) | 230075_at |
| 8 | 2.137 | SLFN5(SLFN5) | 238430_x_at |
| 9 | 1.885 | CYBRD1(CYBRD1) | 222453_at |
| 10 | 1.857 | FBXW7(FBXW7) | 229419_at |
| 11 | 1.837 | SKP2(SKP2) | 203626_s_at |
| 12 | 1.824 | FST(FST) | 207345_at |
| 13 | 1.804 | DDAH1(DDAH1) | 209094_at |
| 14 | 1.758 | PLAU(PLAU) | 211668_s_at |
| 15 | 1.699 | CCNG2(CCNG2) | 202769_at |
| 16 | 1.688 | APOL6(APOL6) | 219716_at |
| 17 | 1.671 | RFX7(RFX7) | 222630_at |
| 18 | 1.669 | PHF15(PHF15) | 212660_at |
| 19 | 1.598 | CD83(CD83) | 204440_at |
| 20 | 1.595 | FOXN2(FOXN2) | 226711_at |
| 21 | 1.574 | SLC35A1(SLC35A1) | 203306_s_at |
| 22 | 1.569 | MED30(MED30) | 227787_s_at |
| 23 | 1.552 | HSPA1A(HSPA1A) | 200800s_at |
| 24 | 1.552 | HSPA1B(HSPA1B) | 200800_s_at |
| 25 | 1.548 | CTDSP2(CTDSP2) | 203445_s_at |
| 26 | 1.544 | PRR3(PRR3) | 204795_at |
| 27 | 1.531 | ANKRD46(ANKRD46) | 212731_at |
| 28 | 1.506 | FUT4(FUT4) | 209892_at |
| 29 | 1.479 | ACTR2(ACTR2) | 200727_s_at |
| 30 | 1.47 | ARHGAP29(ARHGAP29) | 203910_at |
| 31 | 1.47 | XIAP(XIAP) | 228363_at |
| 32 | 1.468 | PIK3C2B(PIK3C2B) | 204484_at |
| 33 | 1.466 | STK16[STK16) | 209622_at |
| 34 | 1.461 | CDC5L(CDC5L) | 209057_x_at |
| 35 | 1.459 | PTGS2(PTGS2) | 204748_at |
| 36 | 1.449 | 5TK38L(5TK38L) | 212572_at |
| 37 | 1.446 | PPFIBP1(PPFIBP1) | 203735_x_at |
| 38 | 1.438 | RILPL2(RILPL2) | 227983_at |
| 39 | 1.418 | ETS1(ETS1) | 224833_at |
| 40 | 1.418 | SDC4(SDC4) | 202071_at |
| 41 | 1.413 | MGC5370(MDM2) | 225160_x_at |
| 42 | 1.412 | DUSP6(DUSP6) | 208893_s_at |
| 43 | 1.412 | ITGA2(ITGA2) | 227314_at |
| 44 | 1.411 | TOB2(TOB2) | 222243_s_at |
| 45 | 1.407 | ELK3(ELK3) | 221773_at |
| 46 | 1.403 | IP6K2(IP6K2) | 223165_s_at |
| 47 | 1.402 | SLC5A3(SLC5A3) | 212944_at |
| 48 | 1.395 | MCAM(MCAM) | 211042_x_at |
| 49 | 1.394 | MAPK1IP1L(MAPK1IP1L) | 225643_at |
| 50 | 1.392 | ASB13(ASB13) | 218862_at |
| 51 | 1.381 | RHPN2(RHPN2) | 227196_at |
| 52 | 1.371 | RFFL(RFFL) | 228980_at |
| 53 | 1.35 | NET1(NET1) | 201830_s_at |
| 54 | 1.34 | PTP4A1(PTP4A1) | 200730_s_at |
| 55 | 1.332 | NUCKS1(NUCKS1) | 222424s_at |
| 56 | 1.315 | LSM6(LSM6) | 205036_at |
| 57 | 1.299 | EIF2S1(EIF2S1) | 201142_at |
| 58 | 1.283 | TWFRSF21(TNFRSF21) | 218856_at |
| 59 | 1.26 | PRKAR1A(PRKAR1A) | 200603_at |
| 60 | 1.241 | PPP2R2A(PPP2R2A) | 202313_at |
| 61 | 1.233 | ID3(ID3) | 207826_s_at |
| 62 | 1.227 | G3BP1(G3BP1) | 201503_at |
| 63 | 1.212 | SLC38A2(SLC38A2) | 222982_x_at |
| 64 | 1.205 | LSM14A(LSM14A) | 212132_at |

**[Table 8]**

| **Downstream genes down-regulated by the inhibition of SUV39H2 expression** | | | | | | | |
|---|---|---|---|---|---|---|---|
| NO | Ratio | Gene | Symbol | NO | Ratio | Gene | Symbol |
| 1 | 0.807 | EIF4G2(EIF4G2) | 200004_at | 74 | 0.566 | NANOS1(NANOS1) ) | 228523_at |
| 2 | 0.778 | NQO1(NQO1) | 201468_s_at | 75 | 0.564 | GPR125(GPR125) | 210473_s_at |
| 3 | 0.778 | SEPT10(SEPT10) | 212698_s_at | 76 | 0.563 | LMLN(LMLN) | 244881_at |
| 4 | 0.762 | NPC1(NPC1) | 202679_at | 77 | 0.562 | GOLGA1(GOLGA1) | 203384_ s_at |
| 5 | 0.758 | KLHDC2(KLHDC2) | 217906_at | 78 | 0.562 | NFIA(NFIA) | 224970_at |
| 6 | 0.754 | TNPO3(TNPO3) | 212318_at | 79 | 0.561 | POLR2C(POLR2C) | 214263_x_at |
| 7 | 0.753 | CDCA4(CDCA4) | 218399_s_at | 80 | 0.555 | SMYD2(SMYD2) | 212922_sat |
| 8 | 0.747 | PTP4A2(PTP4A2) | 208617_s_at | 81 | 0.554 | IGF2BP3(IGF2BP3) | 203819_s_at |
| 9 | 0.729 | SUMO2(SUMO2) | 208739_x_at | 82 | 0.549 | ATP11A(ATP11A) | 213582_at |
| 10 | 0.727 | RAB31(RAB31) | 217763_s_at | 83 | 0.549 | PPAP2B(PPAP2B) | 212230_at |
| 11 | 0.723 | RAB15(RAB15) | 59697_at | 64 | 0.549 | SRGAP2P1(SRGAP2P1) | 1568955_at |
| 12 | 0.723 | TMEM77(DRAM2) | 225228_at | 35 | 0.549 | TTL(TTL) | 224896_s_at |
| 13 | 0.718 | PRKCI(PRKCI) | 209678_s_at | 86 | 0.547 | FRMD6(FRMD6) | 225464_at |
| 14 | 0.717 | NICN1(NICN1) | 223442_at | 87 | 0.547 | SLC9A6(SLC9A6) | 203909_at |
| 15 | 0.717 | PGRMC2(PGRMC2) | 213227_at | 88 | 0.547 | USP46(USP46) | 203870_at |
| 16 | 0.714 | ENDOD1(ENDOD1) | 212573_at | 89 | 0.544 | CNOT6(CNOT6) | 222476_at |
| 17 | 0.708 | DNAJB12(DNAJB12) | 202867_s_at | 90 | 0.54 | AADACL1(NCEH1) | 225847_at |
| 18 | 0.696 | LEPROT(LEPROT) | 202378_s_at | 91 | 0.54 | NUP54(NUP54) | 218256_s_at |
| 19 | 0.694 | ABTB1 (ABTB1 ) | 229164_s_at | 92 | 0.538 | TMEM87B(TMEM87B) | 225412at |
| 20 | 0.694 | MGRN1(MGRN1) | 212576_at | 93 | 0.536 | XPR1(XPP1) | 226615_at |
| 21 | 0694 | SMCR7L(SMCR7L) | 204593_s_at | 94 | 0.532 | PURB(PURB) | 225120_at |
| 22 | 0.692 | SLCO3A1(SLCO3A1) | 219229_at | 95 | 0.531 | REEP4(REEP4) | 218777_at |
| 23 | 0.686 | TGOLN2(TGOLN2) | 212040_at | 96 | 0.524 | LDLRAP1(LDLRAP1) | 57082_at |
| 24 | 0.68 | LRRC8A(LRRC8A) | 224624at | 97 | 0.523 | UBE2N(UBE2N) | 212751_at |
| 25 | 0.68 | MAP3K5(MAP3K5) | 203836_s_at | 98 | 0522 | AKIRIN1 (AKIRIN1) | 217893_s_at |
| 26 | 0.679 | ACTR5(ACTR5) | 219623_at | 99 | 0.519 | JDP2(JDP2) | 226267_at |
| 27 | 0.673 | GDE1(GDE1) | 226214_at | 100 | 0.514 | NACC2(NACC2) | 212993_at |
| 28 | 0.671 | KLHL36(KLHL36) | 238523_at | 101 | 0.51 | PGM2L1(PGM2L1) | 229256_at |
| 29 | 0.671 | NSMAF(NSMAF) | 232149_s_at | 102 | 0.497 | NEDD4(NEDD4) | 213012_at |
| 30 | 0.668 | ABCC2(ABCC2) | 206155_at | 103 | 0.491 | KLRAQ1(KLRAQ1) | 1553955_at |
| 31 | 0.668 | PBX1(PBX1) | 212148_at | 104 | 0.49 | RNF11(RNF11) ) | 208924_at |
| 32 | 0.666 | SNX27(SNX27) | 221498_at | 105 | 0.489 | ARL13B(ARL13B) | 228201_at |
| 33 | 0.664 | TPM1 (TPM1) ) | 210986_s_at | 106 | 0.484 | ARHGAP1 9(ARHGAP19) | 37577_at |
| 34 | 0.662 | HIF1 AN(HIF1AN) | 226648_at | 107 | 0.484 | PNMA1 (PNMA1) | 218224_at |
| 35 | 0.662 | UBQLN1(UBQLN1) ) | 222990_at | 108 | 0.478 | CPEB4(CPEB4) | 224828_at |
| 36 | 0.66 | GPR64(GPR64) | 206002_at | 109 | 0.477 | CAV2(CAV2) | 203323_at |
| 37 | 0.639 | PTHLH(PTHLH) | 206300_s_at | 110 | 0.472 | MAP3K7IP3(TAB3) | 1552928_s_at |
| 38 | 0.638 | LLGL2(LLGL2) | 203713_s_at | 111 | 0.468 | PTGFRN(PTGFRN) | 224937_at |
| 39 | 0.637 | TARSL2(TARSL2) | 227611_at | 112 | 0.467 | SCOC(SCOC) | 224786_at |
| 40 | 0.635 | ANTXR1(ANTXR1) | 224694_at | 113 | 0.462 | MBTPS1(MBTPS1) | 201620_at |
| 41 | 0.631 | DYNLRB1(DYNLRB1) | 217917_s_at | 114 | 0.461 | TRAK2(TRAK2) | 202124_s_at |
| 42 | 0.631 | WDR42A(DCAF8) | 202250_s_at | 115 | 0.452 | CSNK2A2(CSNK2A2) | 224922_at |
| 43 | 0.624 | GSPT1(GSPT1) | 234975_at | 116 | 0.437 | FAM1 8B(FAM18B1) | 218446_s_at |
| 44 | 0.619 | BTBD7(BTBD7) | 224945_at | 117 | 0.436 | CDC42(CDC42) | 226400_at |
| 45 | 0.617 | CTGF(CTGF) | 209101_at | 118 | 0.425 | ABCC1(ABCC1) | 202804_at |
| 46 | 0.616 | LRP8(LRP8) | 208433s_at | 119 | 0.421 | CMTM4(CMTM4) | 225009_at |
| 47 | 0.612 | BZW1 (BZW1) | 200777_s_at | 120 | 0.417 | FGFRL1(FGFRL1) | 223321_s_at |
| 48 | 0.612 | CALM1 (CALM1) ) | 211985_s_at | 121 | 0.41 | OSBPL10(OSBPL10) | 219073_s_at |
| 49 | 0.612 | CALM2(CALM2) | 211985_s_at | 122 | 0.409 | MFSD6(MFSD6) | 219658_s_at |
| 50 | 0.612 | CALM3(CALM3) | 211985_s_at | 123 | 0.405 | ALS2CR4(ALS2CR4) | 228255_at |
| 51 | 0.612 | NR2F6(NR2F6) | 209262_s_at | 124 | 0.405 | C2CD2(C2CD2) | 212875_s_at |
| 52 | 0.612 | TPCN1 (TPCN1 ) | 217914_at | 125 | 0.405 | GLS(GLS) | 203159_at |
| 53 | 0.611 | SDC1(SDC1) | 201286_at | 126 | 0.397 | RP5-1022P62(GPCPD1) ) | 224826_at |
| 54 | 0.61 | PTPLB(PTPLB) | 212640_at | 127 | 0.396 | PI4K2A(PI4K2A) | 215134_at |
| 55 | 0.603 | AGTRAP(AGTRAP) | 225059_at | 128 | 0.394 | HPS5(HPS5) | 204544_at |
| 56 | 0.599 | AKT3(AKT3) | 222880_at | 129 | 0.384 | CDC2L6(CDK19) | 212899_at |
| 57 | 0.598 | TNFRSF10D(TNFRSF10D) | 227345_at | 130 | 0.381 | MTMR9(MTMR9) | 213278_at |
| 58 | 0.596 | ATP6V1 C1 (ATP6V1 C1) | 226463_at | 131 | 0.381 | UHMK1(UHMK1) | 227740_at |
| 59 | 0.596 | RAB4A(RAB4A) | 203581_at | 132 | 0.37 | SCARA3(SCARA3) | 219416_at |
| 60 | 0.595 | ZDHHC9(ZDHHC9) | 222451_s_at | 133 | 0.362 | DYNC1LI2(DYNC1LI2) | 224616_at |
| 61 | 0.594 | TMF1(TMF1) | 214948_s_at | 134 | 0.362 | RAB18(RAB18) | 224377_s_at |
| 62 | 0.593 | GABARAPL1(GABARAPL1) | 211458_s_at | 135 | 0.35 | TWF1(TWF1) | 201745_at |
| 63 | 0.593 | GABARAPL3(GABARAPL3) | 211458_s_at | 136 | 0.339 | AGPAT3(AGPAT3) | 223184_s_at |
| 64 | 0.584 | ABHD2(ABHD2) | 228490_at | 137 | 0.336 | TPRG1 L(TPRG1L) | 224871_at |
| 65 | 0.584 | KREMEN1(KREMEN1) | 227250_at | 138 | 0.326 | PTK2(PTK2) | 241453_at |
| 66 | 0.582 | CACNB3(CACNB3) | 34726_at | 139 | 0.315 | ENTPD4(ENTPD4) | 204076_at |
| 67 | 0.581 | FIGN(FIGN) | 242828_at | 140 | 0.306 | EIF2C2(EIF2C2) | 225569_at |
| 68 | 0581 | TUBGCP3(TUBGCP3) | 1554086_ at | 141 | 0.3 | RNF38(RNF38) | 218528_s_at |
| 69 | 0.578 | DUSP3(DUSP3) | 201536_at | 142 | 0.294 | TMEM167A(TMEM167A) | 224702_at |
| 70 | 0.578 | EGLN3(EGLN3) | 219232_s_at | 143 | 0.218 | EPDR1(EPDR1) | 223253_at |
| 71 | 0.568 | MTMR4(MTMR4) | 212277_at | 144 | 0.212 | STARD3NL(STARD3NL) | 223065_s_at |
| 72 | 0.567 | FAM83D(FAM83D) | 225687_at | 145 | 0.21 | RC3H2(RC3H2) | 225813_at |
| 73 | 0.567 | SUMF1(SUMF1) | 226850_at | 146 | 0.138 | SUV39H2(SUV39H2) | 1554572_ a_at |

**[Table 9]**

| **Gene Ontology pathway analysis based on the Affymetrix's microarray data** | | | |
|---|---|---|---|
| Entry ID | Name | Definition | *P*-value |
| G 00031058 | positwe regulation of histone modification | Any process that activates or increases the frequency, rate or extent of the covalent alteration of a histone. | 1.62 × 10⁻² |
| G 00046974 | histone lysine N-methyltransferase activity (H3-K9 specific) | Catalysis of the addition of a methyl group onto lysine at position 9 of the histone H3 protein. | 1.74 × 10⁻² |
| G 00045002 | double-strand break repair via single-strand annealing | Repair of a DSB made between two repeated sequences oriented in the same direction occurs primarily by the single strand annealing pathway. The ends of the break are processed by a 5' to 3' exonuclease. exposing complementary single-strand regions of the direct repeats that can anneal, resulting in a deletion of the unique DNA between the direct repeats. | 1.62 × 10⁻² |
| G 00000729 | DNA double-strand break processing | The 5' to 3' exonucleolytic resection of the DNA at the site of the break to form a 3' single-strand D NA overhang. | 1.62 × 10⁻² |
| G 00043687 | post-translational protein modification | The covalent alteration of one or more amino acids occurring in a protein after the protein has been completely translated and released from the ribosome. | 2.33 × 10⁻² |
| G 00031056 | regulation of histone modification | Any process that modulates the frequency, rate or extent of the covalent alteration of a histone. | 2.42 × 10⁻² |
| G 00031399 | regulation of protein modification process | Any process that modulates the frequency, rate or extent of the covalent alteration of one or more amino acid residues within a protein. | 4.34 × 10⁻² |

**[Table 10]**

| **Interacting proteins of SUV39H2** | |
|---|---|
| **Protein** | **Description** |
| CBX5 / HP1 alpha | Chromobox protein homolog 5/Heterochromatin protein 1 homolog alpha |
| CBX1 / HP1 beta | Chromobox protein homolog 1/Heterochromatin protein 1 homolog beta |
| CBX3/ HP1 gamma | Chromobox protein homolog 3/Heterochromatin protein 1 homolog gamma |
| RS14 | 40S ribosomal protein S14 |
| RL22 | 60S ribosomal protein L22 |
| H3L | Histone H3-like |
| RL31 | 60S ribosomal protein L31 |
| SMD | Small nuclear ribonucleoprotein Sm D2/SNRPD2 |
| H2B1A | Histone H2B type 1-A |
| H2A1A | Histone H2A type 1-A |
| H4 | Histone H4 |
| KU86 / XRCC5 | ATP-dependent DNA helicase 2 subunit 2 |
| ADT2 | ADP/ATP translocase 2 |
| ADT3 | ADP/ATP translocase 3 |
| CALX | Major histocompatibility complex class I antigen-binding protein p88 |
| CH60 | 60 kDa heat shock protein, mitochondrial |
| EF1A1 | Elongation factor 1 alpha 1 |
| GCN1L | Translational activator GCN1 |
| HSP70 | Heat shock 70kDa protein |
| HSPA5/GRP78 | Heat shock 70kDa protein 5 |
| HSPA8 | Heat shock 70kDa protein 8 |
| HSP90AA1 | Heat shock protein HSP 90-alpha |
| HSP90AB1 | Heat shock protein HSP 90-beta |
| HUWE1 | E3 ubiquitin-protein ligase HUWE1 |
| IRS4 | Insulin receptor substrate 4 |
| PCNA | Proliferating cell nuclear antigen |
| PYR1 | CAD protein, Glutamine-dependent carbamoyl-phosphate synthase |
| SACS | Sacsin |
| TBB2C | Tubulin beta-2C chain |
| TBB4 | Tubulin beta-4 chain |
| TBB5 | Tubulin beta chain |
| XPO1 | Exportin-1, Chromosome region maintenance 1 protein homolog |
| XPOT | Exportin-T, tRNA exportin |

### Example 5: Screening for inhibitors of methyltransferase activity of SUV39H2

GST-fusion SUV39H2 (SEQ ID NO: 33; GST: amino acid position 1-98, SUV39H2 fragment: amino acid position 99-410) was incubated in methyltransferase buffer (20 mM Tris-HCl, 50 mM NaCl, 10 mM MgCl₂, 0.03% Tween-80 and 10 mM DTT) along with 0.35 mM biotinylated-histone H3 peptide (SEQ ID NO: 31), 0.1 mM S-adenosyl-L-[methyl-³H]methionine and a test substance for 1 hour at room temperature in a total volume of 20 microliter. After incubation for 1 hour at room temperature, the reactions were stopped by adding 20 microliter of scintillation proximity assay (SPA) beads buffer containing 60 microgram of streptavidin-coated PVT beads, then light emitted from the beads was measured using a scintillation counter.

As a result of evaluating a number of chemically synthesized compounds by aforementioned assay, some compounds that inhibit methyltransferase activity of SUV39H2 were identified.

### Discussion

Epigenetic modifications, including DNA methylation, covalent histone modifications, nucleosome positioning and miRNAs, play important roles in normal mammalian development and regulation of gene expression [Sharma S et al, Carcinogenesis 2010; 31: 27-36]. Besides the significance of epigenetics in normal physiological functions, its deregulation is deeply involved in various types of diseases such as cancer [Sharma S et al, Carcinogenesis 2010; 31: 27-36], cardiovascular diseases [Turunen MP et al, Biochim Biophys Acta 2009; 1790: 886-891], metabolic diseases [Symonds ME et al. Nat Rev Endocrinol 2009;5:604-610], and autoimmune diseases [Javierre BM et al. Genome Res 2010;20:170-179]. Among epigenetic alterations, abnormal histone modifications are involved in cancer initiation and progression [Chi P et al. Nat Rev Cancer 2010;10:457-469, Portela A et al. Nat Biotechnol 2010;28:1057-1068]. Although the association of acetylation/deacetylation dynamics with human carcinogenesis has been most widely studied among histone modifications [Cortez CC et al, Mutat Res 2008;647:44-51], a growing body of evidences indicates that histone methyltransferases are also crucial for carcinogenesis [Schneider R et al. Trends Biochem Sci 2002;27:396-402].

With the exception of Dot1/DOT1L, all identified histone lysine methyltransferases (HKMTs) harbor a conserved methyltransferase domain termed a SET [Su(var)3-9, Enhancer-of-zeste, Trithorax] domain [Jenuwein T et al. Cell Mol Life Sci 1998;54: 80-93]. Among HKMTs, Su(var) genes were initially identified by genetic screens on centromeric position effects in Drosophila melanogaster and Shizosaccharomyces pombe [Reuter G et al. Bioessays 1992;14:605-612, Allshire RC et al. Genes Dev 1995;9:218-233] and characterized family members actually represent enzymes that can modify chromatin [Wallrath LL Curr Opin Genet Dev 1998;8:147-153]. Suv39h2, the murine homologue of human SUV39H2, was then isolated and characterized as the second methyltransferase targeting H3K9, and contributing to heterochromatin formation [O'Carroll D et al. Mol Cell Biol.2000;20:9423-9433]. Importantly, a subsequent study suggested that Suv39-depleted mice show impaired viability, chromosomal instabilities and spermatogenic failure as phenotypes [Peters AH et al. Cell 2001;107: 323-337].

Furthermore, another study showed that inhibition of Suv39 expression results in reduced di- and trimethylation of H3K9 at telomeres, concomitant with more monomethylation of H3K9 and lowered chromobox protein binding [Garcia-Cao M,et al. Nat Genet 2004; 36: 94-99]. Although these data implied an epigenetic regulation on a variety of mammalian biological functions by Suv39h2, its role in human carcinogenesis remains to be clarified.

As demonstrated herein, the present inventors found significant up-regulation of SUV39H2 in lung and bladder cancer tissues compared to normal tissues by qPCR together with microarray-based expression profiles of a large number of clinical tissues; SUV39H2 is aberrantly over-expressed in various types of cancer. Subsequently, it was clarified that SUV39H2 serves an important role in the growth regulation of cancer cells and was confirmed that inhibition of SUV39H2 expression resulted in the growth suppression of lung and bladder cancer cell lines, with the number of cells at S phase decreased and in G₁ phase increased (Figures 4 and 5). Pathway analysis of the cells treated with SUV39H2 siRNAs indicated that SUV39H2 may regulate multiple biological pathways including chromatin modifications. This result is consistent with our findings that SUV39H2 modulates expression of, and indeed interacts directly with, a number of proteins associated with histone modification and chromatin dynamics (Table 6).

The importance of SUV39H2 in cell growth and G₁ to S progression was also confirmed in Suv39hDN(SUV39H1/SUV39H2 double null) mouse embryonic fibroblasts (MEFs, Figures 7B and 7C). Again, microarray and Gene Ontology analysis also showed that SUV39H2 could be associated with multiple biological processes including the cell cycle (data not shown). Recently, it was reported that LSD1 could demethylate MYPT1, and this process could be involved in human carcinogenesis [Cho HS,et al. Cancer Res. 2010]. In addition to our study, a string of reports have indicated that a variety of non-histone proteins are subjected to a methylation/ demethylation cycle and that this process affects processes such as transcriptional activity, stability at the protein level, and binding affinity to interacting partners [Huang J,et al. Nature 2006; 444: 629-632, Huang J et al. Nature 2007; 449: 105-108, Esteve PO et al. Proc Natl Acad Sci U S A 2009; 106: 5076-5081, Guo Z et al. Nat Chem Biol 2010; 6: 766-773]. Given that SUV39H2 may interact with a number of non-histone proteins (Table 10), further study should be conducted to identify novel substrates of SUV39H2.

These expression analysis showed that expression levels of SUV39H2 in various types of cancer tissues are significantly elevated compared with normal tissues (Figures 1, 2 and 3) and siSUV39H2 resulted in the significant suppression of cancer cell proliferation (Figures 4C-4E). As expression levels of SUV39H2 in various types of normal tissues are significantly low (Figure 1C and Figure 9: data from BioGPS), this gene appears to be an ideal target for cancer therapy. The fact that inhibitors targeting DNA methyltransferases (DNMTs) and histone deacetylases (HDACs) have been approved for hematologic malignancies attracts increasing attention to the development of specific inhibitors targeting epigenetic machinery for cancer therapy [Cortez CC et al. Mutat Res 2008; 647: 44-51, Ma WW et al.CA Cancer J Clin 2009; 59: 111-137]. Importantly, structural analysis of human H3K9 methyltransferases has been conducted, providing important insights to guide the development of selective inhibitors of H3K9 methyltransferases [Wu H,et al. PLoS One 2010; 5: e8570]. Taking these findings into account, it appears feasible to develop inhibitors of SUV39H2 for cancer therapy. As the development of inhibitors targeting methyltransferases started only recently [Copeland RA et al. Nat Rev Drug Discov 2009; 8: 724-732], further validation against the functions of this enzyme may ensure the usefulness of this approach in the near future.

### Industrial Applicability

The gene-expression analysis of cancers described herein, using the combination of laser-capture dissection and genome-wide cDNA microarray, has identified a specific gene as a target for cancer prevention and therapy. Based on the expression of this differentially expressed gene, i.e., SUV39H2, the present description provides a novel molecular diagnostic marker for identifying and detecting cancers. Therefore, the present description also provides a novel diagnostic strategy using SUV39H2.

Furthermore, as described herein, SUV39H2 are involved in cancer cell survival. Therefore, the present description also provides novel molecular targets for treating and preventing cancer. They may be useful for developing novel therapeutic drugs and preventative agents without adverse effects.

The present inventors have shown that the cell growth is suppressed by a doublestranded molecule that specifically targets the SUV39H2 gene. Thus, the doublestranded molecule against the SUV39H2 gene is useful for the development of anti-cancer pharmaceuticals.

Moreover, substances that block the expression of SUV39H2 protein or inhibit its activity may find therapeutic utility as anti-cancer agents, particularly anti-cancer agents for the treatment of lung cancer, cervical cancer, bladder cancer, esophageal cancer, osteosarcoma, prostate cancer or soft tissue tumor.

The methods described herein are also useful for the identification of additional molecular targets for prevention, diagnosis, and treatment of cancers. The data provided herein add to a comprehensive understanding of cancers, facilitate development of novel diagnostic strategies, and provide clues for identification of molecular targets for therapeutic drugs and preventative agents. Such information contributes to a more profound understanding of tumorigenesis, and provides indicators for developing novel strategies for diagnosis, treatment, and ultimately prevention of cancers.

## Claims

1. A method of screening for a candidate substance for either or both of the treatment and prevention of cancer or the inhibition of cancer cell growth, said cancer is bladder cancer and said method comprising the steps of:
(a) contacting a test substance with an SUV39H2 polypeptide;
(b) detecting a methyltransferase activity or cell-proliferation promoting activity of the polypeptide of step (a); and
(c) selecting the test substance that suppresses the methyltransferase activity or cell-proliferation promoting activity of the polypeptide in comparison with the methyltransferase activity or cell-proliferation promoting activity detected in the absence of the test substance.

## Patentansprüche

1. Verfahren zum Screenen auf einen Substanzkandidaten entweder für die Behandlung und Prävention von Krebs oder die Hemmung des Krebszellwachstums oder beides, wobei der Krebs Blasenkrebs ist und das Verfahren die folgenden Schritte umfasst:
(a) Inkontaktbringen einer Testsubstanz mit einem SUV39H2-Polypeptid;
(b) Detektieren einer Methyltransferase-Aktivität oder einer die Zellproliferation fördernden Aktivität des Polypeptids aus Schritt (a); und
(c) Auswählen der Testsubstanz, die die Methyltransferase-Aktivität oder die die Zellproliferation fördernde Aktivität des Polypeptids unterdrückt, verglichen mit der Methyltransferase-Aktivität oder der die Zellproliferation fördernden Aktivität, die in Abwesenheit der Testsubstanz detektiert wurde.

## Revendications

1. Méthode de criblage d'une substance candidate destinée soit ou à la fois au traitement et à la prévention d'un cancer ou à l'inhibition de la croissance de cellules cancéreuses, ledit cancer étant un cancer de la vessie et ladite méthode comprenant les étapes consistant à :
(a) mettre en contact une substance à tester avec un polypeptide SUV39H2 ;
(b) détecter une activité méthyltransférase ou une activité favorisant la prolifération cellulaire du polypeptide de l'étape (a) ; et
(c) sélectionner la substance à tester qui supprime l'activité méthyltransférase ou l'activité favorisant la prolifération cellulaire du polypeptide par comparaison à l'activité méthyltransférase ou l'activité favorisant la prolifération cellulaire détectée en l'absence de la substance à tester.


### SEQUENCE LISTING

<110> ONCOTHERAPY SCIENCE, INC.
<120> SUV39H2 AS A TARGET GENE FOR CANCER THERAPY AND DIAGNOSIS
<130> ONC-A1106P
<150> US 61/493,235
   <151> 2011-06-03
<160> 33
<170> PatentIn version 3.5
<210> 1
   <211> 20
   <212> DNA
   <213> Artificial Sequence
<220>
   <223> An artificially synthesized primer sequence for RT-PCR
<400> 1
   gcaaattcca tggcaccgtc 20
<210> 2
   <211> 19
   <212> DNA
   <213> Artificial Sequence
<220>
   <223> An artificially synthesized primer sequence for RT-PCR
<400> 2
   tcgccccact tgattttgg 19
<210> 3
   <211> 20
   <212> DNA
   <213> Artificial Sequence
<220>
   <223> An artificially synthesized primer sequence for RT-PCR
<400> 3
   tgggaacaag agggcatctg 20
<210> 4
   <211> 22
   <212> DNA
   <213> Artificial Sequence
<220>
   <223> An artificially synthesized primer sequence for RT-PCR
<400> 4
   ccaccactgc atcaaattca tg 22
<210> 5
   <211> 21
   <212> DNA
   <213> Artificial Sequence
<220>
   <223> An artificially synthesized primer sequence by RT-PCR
<400> 5
   tggggtgtaa agacccttgt g 21
<210> 6
   <211> 21
   <212> DNA
   <213> Artificial Sequence
<220>
   <223> An artificially synthesized primer sequence by RT-PCR
<400> 6
   attcccttgt tgtcatagaa c 21
<210> 7
   <211> 21
   <212> DNA
   <213> Artificial Sequence
<220>
   <223> An artificially synthesized sense strand sequence for siRNA
<220>
   <221> misc_feature
   <222> (1)..(19)
   <223> RNA region
<400> 7
   gcagcacgac uucuucaagt t 21
<210> 8
   <211> 21
   <212> DNA
   <213> Artificial Sequence
<220>
   <223> An artificially synthesized antisense strand sequence for siRNA
<220>
   <221> misc_feature
   <222> (1)..(19)
   <223> RNA region
<400> 8
   cuugaagaag ucgugcugct t 21
<210> 9
   <211> 19
   <212> RNA
   <213> Artificial Sequence
<220>
   <223> An artificially synthesized sense strand sequence for siRNA
<400> 9
   auccgcgcga uaguacgua 19
<210> 10
   <211> 19
   <212> RNA
   <213> Artificial Sequence
<220>
   <223> An artificially synthesized antisense strand sequence for siRNA
<400> 10
   uacguacuau cgcgcggau 19
<210> 11
   <211> 19
   <212> RNA
   <213> Artificial Sequence
<220>
   <223> An artificially synthesized sense strand sequence for siRNA
<400> 11
   uuacgcguag cguaauacg 19
<210> 12
   <211> 19
   <212> RNA
   <213> Artificial Sequence
<220>
   <223> An artificially synthesized antisense strand sequence for siRNA
<400> 12
   cguauuacgc uacgcguaa 19
<210> 13
   <211> 19
   <212> RNA
   <213> Artificial Sequence
<220>
   <223> An artificially synthesized sense strand sequence for siRNA
<400> 13
   uauucgcgcg uauagcggu 19
<210> 14
   <211> 19
   <212> RNA
   <213> Artificial Sequence
<220>
   <223> An artificially synthesized antisense strand sequence for siRNA
<400> 14
   accgcuauac gcgcgaaua 19
<210> 15
   <211> 21
   <212> DNA
   <213> Artificial Sequence
<220>
   <223> An artificially synthesized sense strand sequence for siRNA
<220>
   <221> misc_feature
   <222> (1)..(19)
   <223> RNA region
<400> 15
   cuuugguugu ucaugcacat t 21
<210> 16
   <211> 21
   <212> DNA
   <213> Artificial Sequence
<220>
   <223> An artificially synthesized antisense strand sequence for siRNA
<220>
   <221> misc_feature
   <222> (1)..(19)
   <223> RNA region
<400> 16
   ugugcaugaa caaccaaagt t 21
<210> 17
   <211> 21
   <212> DNA
   <213> Artificial Sequence
<220>
   <223> An artificially synthesized sense strand sequence for siRNA
<220>
   <221> misc_feature
   <222> (1)..(19)
   <223> RNA region
<400> 17
   cuggaaucag cuuagucaat t 21
<210> 18
   <211> 21
   <212> DNA
   <213> Artificial Sequence
<220>
   <223> An artificially synthesized antisense strand sequence for siRNA
<220>
   <221> misc_feature
   <222> (1)..(19)
   <223> RNA region
<400> 18
   uugacuaagc ugauuccagt t 21
<210> 19
   <211> 19
   <212> RNA
   <213> Artificial Sequence
<220>
   <223> An artificially synthesized target sequence for siRNA
<400> 19
   cuuugguugu ucaugcaca 19
<210> 20
   <211> 19
   <212> RNA
   <213> Artificial Sequence
<220>
   <223> An artificially synthesized target sequence for siRNA
<400> 20
   cuggaaucag cuuagucaa 19
<210> 21
   <211> 3148
   <212> DNA
   <213> Homo sapiens
<220>
   <221> CDS
   <222> (107)..(1339)
<400> 21
   aacaagcccc ggcccccaag tcccgcgcgg gccggccagg ggcggggcgt cgggccagct       60
   gagctatccc gtcagaccgc gccagtttga atgaaagctc tacaag atg gcg gcg         115
                                                      Met Ala Ala
                                                      1
   gtc ggg gcc gag gcg cga gga gct tgg tgt gtg cct tgc cta gtt tca        163
   Val Gly Ala Glu Ala Arg Gly Ala Trp Cys Val Pro Cys Leu Val Ser
       5                   10                  15
   ctt gat act ctt cag gaa tta tgt aga aaa gaa aag ctc aca tgt aaa        211
   Leu Asp Thr Leu Gln Glu Leu Cys Arg Lys Glu Lys Leu Thr Cys Lys
   20                  25                  30                  35
   tcg att gga atc acc aaa agg aat cta aac aat tat gag gtg gaa tac         259
   Ser Ile Gly Ile Thr Lys Arg Asn Leu Asn Asn Tyr Glu Val Glu Tyr
                   40                  45                  50
   ttg tgt gac tac aag gta gta aag gat atg gaa tat tat ctt gta aaa         307
   Leu Cys Asp Tyr Lys Val Val Lys Asp Met Glu Tyr Tyr Leu Val Lys
               55                  60                  65
   tgg aaa gga tgg cca gat tct aca aat act tgg gaa cct ttg caa aat         355
   Trp Lys Gly Trp Pro Asp Ser Thr Asn Thr Trp Glu Pro Leu Gln Asn
           70                  75                  80
   ctg aag tgc ccg tta ctg ctt cag caa ttc tct aat gac aag cat aat         403
   Leu Lys Cys Pro Leu Leu Leu Gln Gln Phe Ser Asn Asp Lys His Asn
       85                  90                  95
   tat tta tct cag gta aag aaa ggc aaa gca ata act cca aaa gac aat         451
   Tyr Leu Ser Gln Val Lys Lys Gly Lys Ala Ile Thr Pro Lys Asp Asn
   100                 105                 110                 115
   aac aaa act ttg aaa cct gcc att gct gag tac att gtg aag aag gct         499
   Asn Lys Thr Leu Lys Pro Ala Ile Ala Glu Tyr Ile Val Lys Lys Ala
                   120                 125                 130
   aaa caa agg ata gct ctg cag aga tgg caa gat gaa ctc aac aga aga         547
   Lys Gln Arg Ile Ala Leu Gln Arg Trp Gln Asp Glu Leu Asn Arg Arg
               135                 140                 145
   aag aat cat aaa gga atg ata ttt gtt gaa aat act gtt gat tta gag         595
   Lys Asn His Lys Gly Met Ile Phe Val Glu Asn Thr Val Asp Leu Glu
           150                 155                 160
   ggc cca cct tca gac ttc tat tac att aac gaa tac aaa cca gct cct         643
   Gly Pro Pro Ser Asp Phe Tyr Tyr Ile Asn Glu Tyr Lys Pro Ala Pro
       165                 170                 175
   gga atc agc tta gtc aat gaa gct acc ttt ggt tgt tca tgc aca gat         691
   Gly Ile Ser Leu Val Asn Glu Ala Thr Phe Gly Cys Ser Cys Thr Asp
   180                 185                 190                 195
   tgc ttc ttt caa aaa tgt tgt cct gct gaa gct gga gtt ctt ttg gct         739
   Cys Phe Phe Gln Lys Cys Cys Pro Ala Glu Ala Gly Val Leu Leu Ala
                   200                 205                 210
   tat aat aaa aac caa caa att aaa atc cca cct ggt act ccc atc tat         787
   Tyr Asn Lys Asn Gln Gln Ile Lys Ile Pro Pro Gly Thr Pro Ile Tyr
               215                 220                 225
   gaa tgc aac tca agg tgt cag tgt ggt cct gat tgt ccc aat agg att         835
   Glu Cys Asn Ser Arg Cys Gln Cys Gly Pro Asp Cys Pro Asn Arg Ile
           230                 235                 240
   gta caa aaa ggc aca cag tat tcg ctt tgc atc ttt cga act agc aat         883
   Val Gln Lys Gly Thr Gln Tyr Ser Leu Cys Ile Phe Arg Thr Ser Asn
       245                 250                 255
   gga cgt ggc tgg ggt gta aag acc ctt gtg aag att aaa aga atg agt         931
   Gly Arg Gly Trp Gly Val Lys Thr Leu Val Lys Ile Lys Arg Met Ser
   260                 265                 270                 275
   ttt gtc atg gaa tat gtt gga gag gta atc aca agt gaa gaa gct gaa         979
   Phe Val Met Glu Tyr Val Gly Glu Val Ile Thr Ser Glu Glu Ala Glu
                   280                 285                 290
   aga cga gga cag ttc tat gac aac aag gga atc acg tat ctc ttt gat      1027
   Arg Arg Gly Gln Phe Tyr Asp Asn Lys Gly Ile Thr Tyr Leu Phe Asp
               295                 300                 305
   ctg gac tat gag tct gat gaa ttc aca gtg gat gcg gct cga tac ggc      1075
   Leu Asp Tyr Glu Ser Asp Glu Phe Thr Val Asp Ala Ala Arg Tyr Gly
           310                 315                 320
   aat gtg tct cat ttt gtg aat cac agc tgt gac cca aat ctt cag gtg      1123
   Asn Val Ser His Phe Val Asn His Ser Cys Asp Pro Asn Leu Gln Val
       325                 330                 335
   ttc aat gtt ttc att gat aac ctc gat act cgt ctt ccc cga ata gca      1171
   Phe Asn Val Phe Ile Asp Asn Leu Asp Thr Arg Leu Pro Arg Ile Ala
   340                 345                 350                 355
   ttg ttt tcc aca aga acc ata aat gct gga gaa gag ctg act ttt gat      1219
   Leu Phe Ser Thr Arg Thr Ile Asn Ala Gly Glu Glu Leu Thr Phe Asp
                   360                 365                 370
   tat caa atg aaa ggt tct gga gat ata tct tca gat tct att gac cac      1267
   Tyr Gln Met Lys Gly Ser Gly Asp Ile Ser Ser Asp Ser Ile Asp His
               375                 380                 385
   agc cca gcc aaa aag agg gtc aga aca gta tgt aaa tgt gga gct gtg      1315
   Ser Pro Ala Lys Lys Arg Val Arg Thr Val Cys Lys Cys Gly Ala Val
           390                 395                 400
   act tgc aga ggt tac ctc aac tga actttttcag gaaatagagc tgatgattat     1369
   Thr Cys Arg Gly Tyr Leu Asn
       405                 410
   aatatttttt tcctaatgtt aacattttta aaaatacata tttgggactc ttattatcaa    1429
   ggttctacct atgttaattt acaattcatg tttcaagaca tttgccaaat gtattaccga    1489
   tgcctctgaa aagggggtca ctgggtctca tagactgata tgaagtcgac atatttatag   1549
   tgcttagaga ccaaactaat ggaaggcaga ctatttacag cttagtatat gtgtacttaa    1609
   gtctatgtga acagagaaat gcctcccgta gtgtttgaaa gcgttaagct gataatgtaa    1669
   ttaacaactg ctgagagatc aaagattcaa cttgccatac acctcaaatt cggagaaaca    1729
   gttaatttgg gcaaatctac agttctgttt ttgctactct attgtcattc ctgtttaata    1789
   ctcactgtac ttgtatttga gacaaatagg tgatactgaa ttttatactg ttttctactt    1849
   ttccattaaa acattggcac ctcaatgata aagaaattta aggtataaaa ttaaatgtaa    1909
   aaattaattt cagcttcatt tcgtatttcg aagcaatcta gactgttgtg atgagtgtat    1969
   gtctgaacct gtaattctta aaagacttct taatcttcta gaagaaaaat ctccgaagag    2029
   ctctctctag aagtccaaaa tggctagcca ttatgcttct ttgaaaggac atgataatgg    2089
   gaccaggatg gttttttgga gtaccaagca aggggaatgg agcactttaa gggcgcctgt    2149
   tagtaacatg aattggaaat ctgtgtcgag tacctctgat ctaaacggta aaacaagctg    2209
   cctggagagc agctgtacct aacaatactg taatgtacat taacattaca gcctctcaat    2269
   ttcaggcagg tgtaacagtt cctttccacc agatttaata tttttatact tcctgcaggt 2329
   tcttcttaaa aagtaatcta tatttttgaa ctgatacttg ttttatacat aaattttttt 2389
   tagatgtgat aaagctaaac ttggccaaag tgtgtgcctg aattattaga cctttttatt 2449
   agtcaaccta cgaagactaa aatagaatat attagttttc aagggagtgg gaggcttcca 2509
   acatagtatt gaatctcagg aaaaactatt ctttcatgtc tgattctgag atttctaatt 2569
   gtgttgtgaa aatgataaat gcagcaaatc tagctttcag tattcctaat ttttacctaa 2629
   gctcattgct ccaggctttg attacctaaa ataagcttgg ataaaattga accaacttca 2689
   agaatgcagc acttcttaat ctttagctct ttcttgggag aagctagact ttattcatta 2749
   tattgctatg acaacttcac tctttcataa tatataggat aaattgttta catgattgga 2809
   ccctcagatt ctgttaacca aaattgcaga atggggggcc aggcctgtgt ggtggctcac 2869
   acctgtgatc ccagcacttt gggaggctga ggtaggagga tcacgtgagg tcgggagttc 2929
   aagaccagcc tggccatcat ggtgaaaccc tgtctctact gaaaatacaa aaattagccg 2989
   ggcgtggtgg cacacgcctg tagtcccagc tactcaggag gctgaggcag gagaatcact 3049
   tgaattcagg aggcggaggt tgcagtgagc caagatcata ccactgcact gcagcctgag 3109
   tgacacagta agactgtctc caaaaaaaaa aaaaaaaaa 3148
<210> 22
   <211> 410
   <212> PRT
   <213> Homo sapiens
<400> 22
   Met Ala Ala Val Gly Ala Glu Ala Arg Gly Ala Trp Cys Val Pro Cys
   1 5 10 15
   Leu Val Ser Leu Asp Thr Leu Gln Glu Leu Cys Arg Lys Glu Lys Leu
   20 25 30
   Thr Cys Lys Ser Ile Gly Ile Thr Lys Arg Asn Leu Asn Asn Tyr Glu
   35 40 45
   Val Glu Tyr Leu Cys Asp Tyr Lys Val Val Lys Asp Met Glu Tyr Tyr
   50 55 60
   Leu Val Lys Trp Lys Gly Trp Pro Asp Ser Thr Asn Thr Trp Glu Pro
   65 70 75 80
   Leu Gln Asn Leu Lys Cys Pro Leu Leu Leu Gln Gln Phe Ser Asn Asp
   85 90 95
   Lys His Asn Tyr Leu Ser Gln Val Lys Lys Gly Lys Ala Ile Thr Pro
               100                 105                 110
   Lys Asp Asn Asn Lys Thr Leu Lys Pro Ala Ile Ala Glu Tyr Ile Val
           115                 120                 125
   Lys Lys Ala Lys Gln Arg Ile Ala Leu Gln Arg Trp Gln Asp Glu Leu
       130                 135                 140
   Asn Arg Arg Lys Asn His Lys Gly Met Ile Phe Val Glu Asn Thr Val
   145                 150                 155                 160
   Asp Leu Glu Gly Pro Pro Ser Asp Phe Tyr Tyr Ile Asn Glu Tyr Lys
                   165                 170                 175
   Pro Ala Pro Gly Ile Ser Leu Val Asn Glu Ala Thr Phe Gly Cys Ser
               180                 185                 190
   Cys Thr Asp Cys Phe Phe Gln Lys Cys Cys Pro Ala Glu Ala Gly Val
           195                 200                 205
   Leu Leu Ala Tyr Asn Lys Asn Gln Gln Ile Lys Ile Pro Pro Gly Thr
       210                 215                 220
   Pro Ile Tyr Glu Cys Asn Ser Arg Cys Gln Cys Gly Pro Asp Cys Pro
   225                 230                 235                 240
   Asn Arg Ile Val Gln Lys Gly Thr Gln Tyr Ser Leu Cys Ile Phe Arg
                   245                 250                 255
   Thr Ser Asn Gly Arg Gly Trp Gly Val Lys Thr Leu Val Lys Ile Lys
               260                 265                 270
   Arg Met Ser Phe Val Met Glu Tyr Val Gly Glu Val Ile Thr Ser Glu
           275                 280                 285
   Glu Ala Glu Arg Arg Gly Gln Phe Tyr Asp Asn Lys Gly Ile Thr Tyr
       290                 295                 300
   Leu Phe Asp Leu Asp Tyr Glu Ser Asp Glu Phe Thr Val Asp Ala Ala
   305                 310                 315                 320
   Arg Tyr Gly Asn Val Ser His Phe Val Asn His Ser Cys Asp Pro Asn
                   325                 330                 335
   Leu Gln Val Phe Asn Val Phe Ile Asp Asn Leu Asp Thr Arg Leu Pro
               340                 345                 350
   Arg Ile Ala Leu Phe Ser Thr Arg Thr Ile Asn Ala Gly Glu Glu Leu
           355                 360                 365
   Thr Phe Asp Tyr Gln Met Lys Gly Ser Gly Asp Ile Ser Ser Asp Ser
       370                 375                 380
   Ile Asp His Ser Pro Ala Lys Lys Arg Val Arg Thr Val Cys Lys Cys
   385                 390                 395                 400
   Gly Ala Val Thr Cys Arg Gly Tyr Leu Asn
                   405                 410
<210> 23
   <211> 3106
   <212> DNA
   <213> Homo sapiens
<220>
   <221> CDS
   <222> (245)..(1297)
<400> 23
   agtttgaatg aaagctctac aagatggcgg cggtcggggc cgaggcgcga ggaggtgagg       60
   ctggagcgcg gccccctcgc cttccctgtt cccagcttgg tgtgtgcctt gcctagtttc      120
   acttgatact cttcaggaat tatgtagaaa agaaaagctc acatgtaaat cgattggaat      180
   caccaaaagg aatctaaaca attatgaggt ggaatacttg tgtgactaca aggtagtaaa      240
   ggat atg gaa tat tat ctt gta aaa tgg aaa gga tgg cca gat tct aca       289
        Met Glu Tyr Tyr Leu Val Lys Trp Lys Gly Trp Pro Asp Ser Thr
        1               5                   10                  15
   aat act tgg gaa cct ttg caa aat ctg aag tgc ccg tta ctg ctt cag        337
   Asn Thr Trp Glu Pro Leu Gln Asn Leu Lys Cys Pro Leu Leu Leu Gln
                   20                  25                  30
   caa ttc tct aat gac aag cat aat tat tta tct cag gta aag aaa ggc        385
   Gln Phe Ser Asn Asp Lys His Asn Tyr Leu Ser Gln Val Lys Lys Gly
               35                  40                  45
   aaa gca ata act cca aaa gac aat aac aaa act ttg aaa cct gcc att        433
   Lys Ala Ile Thr Pro Lys Asp Asn Asn Lys Thr Leu Lys Pro Ala Ile
           50                  55                  60
   gct gag tac att gtg aag aag gct aaa caa agg ata gct ctg cag aga        481
   Ala Glu Tyr Ile Val Lys Lys Ala Lys Gln Arg Ile Ala Leu Gln Arg
       65                  70                  75
   tgg caa gat gaa ctc aac aga aga aag aat cat aaa gga atg ata ttt        529
   Trp Gln Asp Glu Leu Asn Arg Arg Lys Asn His Lys Gly Met Ile Phe
   80                  85                  90                  95
   gtt gaa aat act gtt gat tta gag ggc cca cct tca gac ttc tat tac        577
   Val Glu Asn Thr Val Asp Leu Glu Gly Pro Pro Ser Asp Phe Tyr Tyr
                   100                 105                 110
   att aac gaa tac aaa cca gct cct gga atc agc tta gtc aat gaa gct        625
   Ile Asn Glu Tyr Lys Pro Ala Pro Gly Ile Ser Leu Val Asn Glu Ala
               115                 120                 125
   acc ttt ggt tgt tca tgc aca gat tgc ttc ttt caa aaa tgt tgt cct        673
   Thr Phe Gly Cys Ser Cys Thr Asp Cys Phe Phe Gln Lys Cys Cys Pro
           130                 135                 140
   gct gaa gct gga gtt ctt ttg gct tat aat aaa aac caa caa att aaa        721
   Ala Glu Ala Gly Val Leu Leu Ala Tyr Asn Lys Asn Gln Gln Ile Lys
       145                 150                 155
   atc cca cct ggt act ccc atc tat gaa tgc aac tca agg tgt cag tgt        769
   Ile Pro Pro Gly Thr Pro Ile Tyr Glu Cys Asn Ser Arg Cys Gln Cys
   160                 165                 170                 175
   ggt cct gat tgt ccc aat agg att gta caa aaa ggc aca cag tat tcg        817
   Gly Pro Asp Cys Pro Asn Arg Ile Val Gln Lys Gly Thr Gln Tyr Ser
                   180                 185                 190
   ctt tgc atc ttt cga act agc aat gga cgt ggc tgg ggt gta aag acc        865
   Leu Cys Ile Phe Arg Thr Ser Asn Gly Arg Gly Trp Gly Val Lys Thr
               195                 200                 205
   ctt gtg aag att aaa aga atg agt ttt gtc atg gaa tat gtt gga gag        913
   Leu Val Lys Ile Lys Arg Met Ser Phe Val Met Glu Tyr Val Gly Glu
           210                 215                 220
   gta atc aca agt gaa gaa gct gaa aga cga gga cag ttc tat gac aac        961
   Val Ile Thr Ser Glu Glu Ala Glu Arg Arg Gly Gln Phe Tyr Asp Asn
       225                 230                 235
   aag gga atc acg tat ctc ttt gat ctg gac tat gag tct gat gaa ttc       1009
   Lys Gly Ile Thr Tyr Leu Phe Asp Leu Asp Tyr Glu Ser Asp Glu Phe
   240                 245                 250                 255
   aca gtg gat gcg gct cga tac ggc aat gtg tct cat ttt gtg aat cac       1057
   Thr Val Asp Ala Ala Arg Tyr Gly Asn Val Ser His Phe Val Asn His
                   260                 265                 270
   agc tgt gac cca aat ctt cag gtg ttc aat gtt ttc att gat aac ctc       1105
   Ser Cys Asp Pro Asn Leu Gln Val Phe Asn Val Phe Ile Asp Asn Leu
               275                 280                 285
   gat act cgt ctt ccc cga ata gca ttg ttt tcc aca aga acc ata aat       1153
   Asp Thr Arg Leu Pro Arg Ile Ala Leu Phe Ser Thr Arg Thr Ile Asn
           290                 295                 300
   gct gga gaa gag ctg act ttt gat tat caa atg aaa ggt tct gga gat       1201
   Ala Gly Glu Glu Leu Thr Phe Asp Tyr Gln Met Lys Gly Ser Gly Asp
       305                 310                 315
   ata tct tca gat tct att gac cac agc cca gcc aaa aag agg gtc aga       1249
   Ile Ser Ser Asp Ser Ile Asp His Ser Pro Ala Lys Lys Arg Val Arg
   320                 325                 330                 335
   aca gta tgt aaa tgt gga gct gtg act tgc aga ggt tac ctc aac tga       1297
   Thr Val Cys Lys Cys Gly Ala Val Thr Cys Arg Gly Tyr Leu Asn
                   340                 345                 350
   actttttcag gaaatagagc tgatgattat aatatttttt tcctaatgtt aacattttta       1357
   aaaatacata tttgggactc ttattatcaa ggttctacct atgttaattt acaattcatg 1417
   tttcaagaca tttgccaaat gtattaccga tgcctctgaa aagggggtca ctgggtctca 1477
   tagactgata tgaagtcgac atatttatag tgcttagaga ccaaactaat ggaaggcaga 1537
   ctatttacag cttagtatat gtgtacttaa gtctatgtga acagagaaat gcctcccgta 1597
   gtgtttgaaa gcgttaagct gataatgtaa ttaacaactg ctgagagatc aaagattcaa 1657
   cttgccatac acctcaaatt cggagaaaca gttaatttgg gcaaatctac agttctgttt 1717
   ttgctactct attgtcattc ctgtttaata ctcactgtac ttgtatttga gacaaatagg 1777
   tgatactgaa ttttatactg ttttctactt ttccattaaa acattggcac ctcaatgata 1837
   aagaaattta aggtataaaa ttaaatgtaa aaattaattt cagcttcatt tcgtatttcg 1897
   aagcaatcta gactgttgtg atgagtgtat gtctgaacct gtaattctta aaagacttct 1957
   taatcttcta gaagaaaaat ctccgaagag ctctctctag aagtccaaaa tggctagcca 2017
   ttatgcttct ttgaaaggac atgataatgg gaccaggatg gttttttgga gtaccaagca 2077
   aggggaatgg agcactttaa gggcgcctgt tagtaacatg aattggaaat ctgtgtcgag 2137
   tacctctgat ctaaacggta aaacaagctg cctggagagc agctgtacct aacaatactg 2197
   taatgtacat taacattaca gcctctcaat ttcaggcagg tgtaacagtt cctttccacc 2257
   agatttaata tttttatact tcctgcaggt tcttcttaaa aagtaatcta tatttttgaa 2317
   ctgatacttg ttttatacat aaattttttt tagatgtgat aaagctaaac ttggccaaag 2377
   tgtgtgcctg aattattaga cctttttatt agtcaaccta cgaagactaa aatagaatat 2437
   attagttttc aagggagtgg gaggcttcca acatagtatt gaatctcagg aaaaactatt 2497
   ctttcatgtc tgattctgag atttctaatt gtgttgtgaa aatgataaat gcagcaaatc 2557
   tagctttcag tattcctaat ttttacctaa gctcattgct ccaggctttg attacctaaa 2617
   ataagcttgg ataaaattga accaacttca agaatgcagc acttcttaat ctttagctct 2677
   ttcttgggag aagctagact ttattcatta tattgctatg acaacttcac tctttcataa 2737
   tatataggat aaattgttta catgattgga ccctcagatt ctgttaacca aaattgcaga 2797
   atggggggcc aggcctgtgt ggtggctcac acctgtgatc ccagcacttt gggaggctga 2857
   ggtaggagga tcacgtgagg tcgggagttc aagaccagcc tggccatcat ggtgaaaccc 2917
   tgtctctact gaaaatacaa aaattagccg ggcgtggtgg cacacgcctg tagtcccagc 2977
   tactcaggag gctgaggcag gagaatcact tgaattcagg aggcggaggt tgcagtgagc 3037
   caagatcata ccactgcact gcagcctgag tgacacagta agactgtctc caaaaaaaaa 3097
   aaaaaaaaa 3106
<210> 24
   <211> 350
   <212> PRT
   <213> Homo sapiens
<400> 24
   Met Glu Tyr Tyr Leu Val Lys Trp Lys Gly Trp Pro Asp Ser Thr Asn
   1               5                   10                  15
   Thr Trp Glu Pro Leu Gln Asn Leu Lys Cys Pro Leu Leu Leu Gln Gln
               20                  25                  30
   Phe Ser Asn Asp Lys His Asn Tyr Leu Ser Gln Val Lys Lys Gly Lys
           35                  40                  45
   Ala Ile Thr Pro Lys Asp Asn Asn Lys Thr Leu Lys Pro Ala Ile Ala
       50                  55                  60
   Glu Tyr Ile Val Lys Lys Ala Lys Gln Arg Ile Ala Leu Gln Arg Trp
   65                  70                  75                  80
   Gln Asp Glu Leu Asn Arg Arg Lys Asn His Lys Gly Met Ile Phe Val
                   85                  90                  95
   Glu Asn Thr Val Asp Leu Glu Gly Pro Pro Ser Asp Phe Tyr Tyr Ile
               100                 105                 110
   Asn Glu Tyr Lys Pro Ala Pro Gly Ile Ser Leu Val Asn Glu Ala Thr
           115                 120                 125
   Phe Gly Cys Ser Cys Thr Asp Cys Phe Phe Gln Lys Cys Cys Pro Ala
       130                 135                 140
   Glu Ala Gly Val Leu Leu Ala Tyr Asn Lys Asn Gln Gln Ile Lys Ile
   145                 150                 155                 160
   Pro Pro Gly Thr Pro Ile Tyr Glu Cys Asn Ser Arg Cys Gln Cys Gly
                   165                 170                 175
   Pro Asp Cys Pro Asn Arg Ile Val Gln Lys Gly Thr Gln Tyr Ser Leu
               180                 185                 190
   Cys Ile Phe Arg Thr Ser Asn Gly Arg Gly Trp Gly Val Lys Thr Leu
           195                 200                 205
   Val Lys Ile Lys Arg Met Ser Phe Val Met Glu Tyr Val Gly Glu Val
       210                 215                 220
   Ile Thr Ser Glu Glu Ala Glu Arg Arg Gly Gln Phe Tyr Asp Asn Lys
   225                 230                 235                 240
   Gly Ile Thr Tyr Leu Phe Asp Leu Asp Tyr Glu Ser Asp Glu Phe Thr
                   245                 250                 255
   Val Asp Ala Ala Arg Tyr Gly Asn Val Ser His Phe Val Asn His Ser
               260                 265                 270
   Cys Asp Pro Asn Leu Gln Val Phe Asn Val Phe Ile Asp Asn Leu Asp
           275                 280                 285
   Thr Arg Leu Pro Arg Ile Ala Leu Phe Ser Thr Arg Thr Ile Asn Ala
       290                 295                 300
   Gly Glu Glu Leu Thr Phe Asp Tyr Gln Met Lys Gly Ser Gly Asp Ile
   305                 310                 315                 320
   Ser Ser Asp Ser Ile Asp His Ser Pro Ala Lys Lys Arg Val Arg Thr
                   325                 330                 335
   Val Cys Lys Cys Gly Ala Val Thr Cys Arg Gly Tyr Leu Asn
               340                 345                 350
<210> 25
   <211> 2608
   <212> DNA
   <213> Homo sapiens
<220>
   <221> CDS
   <222> (107)..(799)
<400> 25
   aacaagcccc ggcccccaag tcccgcgcgg gccggccagg ggcggggcgt cgggccagct       60
   gagctatccc gtcagaccgc gccagtttga atgaaagctc tacaag atg gcg gcg         115
                                                      Met Ala Ala
                                                      1
   gtc ggg gcc gag gcg cga gga gct tgg tgt gtg cct tgc cta gtt tca         163
   Val Gly Ala Glu Ala Arg Gly Ala Trp Cys Val Pro Cys Leu Val Ser
       5                   10                  15
   ctt gat act ctt cag gaa tta tgt aga aaa gaa aag ctc aca tgt aaa         211
   Leu Asp Thr Leu Gln Glu Leu Cys Arg Lys Glu Lys Leu Thr Cys Lys
   20                  25                  30                  35
   tcg att gga atc acc aaa agg aat cta aac aat tat gag gtg gaa tac         259
   Ser Ile Gly Ile Thr Lys Arg Asn Leu Asn Asn Tyr Glu Val Glu Tyr
                   40                  45                  50
   ttg tgt gac tac aag gta gta aag gat atg gaa tat tat ctt gta aaa         307
   Leu Cys Asp Tyr Lys Val Val Lys Asp Met Glu Tyr Tyr Leu Val Lys
               55                  60                  65
   tgg aaa gga tgg cca gat tct aca aat act tgg gaa cct ttg caa aat         355
   Trp Lys Gly Trp Pro Asp Ser Thr Asn Thr Trp Glu Pro Leu Gln Asn
           70                  75                  80
   ctg aag tgc ccg tta ctg ctt cag caa ttc tct aat gac aag cat aat         403
   Leu Lys Cys Pro Leu Leu Leu Gln Gln Phe Ser Asn Asp Lys His Asn
       85                  90                  95
   tat tta tct cag gta atc aca agt gaa gaa gct gaa aga cga gga cag         451
   Tyr Leu Ser Gln Val Ile Thr Ser Glu Glu Ala Glu Arg Arg Gly Gln
   100                 105                 110                 115
   ttc tat gac aac aag gga atc acg tat ctc ttt gat ctg gac tat gag         499
   Phe Tyr Asp Asn Lys Gly Ile Thr Tyr Leu Phe Asp Leu Asp Tyr Glu
                   120                 125                 130
   tct gat gaa ttc aca gtg gat gcg gct cga tac ggc aat gtg tct cat         547
   Ser Asp Glu Phe Thr Val Asp Ala Ala Arg Tyr Gly Asn Val Ser His
               135                 140                 145
   ttt gtg aat cac agc tgt gac cca aat ctt cag gtg ttc aat gtt ttc         595
   Phe Val Asn His Ser Cys Asp Pro Asn Leu Gln Val Phe Asn Val Phe
           150                 155                 160
   att gat aac ctc gat act cgt ctt ccc cga ata gca ttg ttt tcc aca         643
   Ile Asp Asn Leu Asp Thr Arg Leu Pro Arg Ile Ala Leu Phe Ser Thr
       165                 170                 175
   aga acc ata aat gct gga gaa gag ctg act ttt gat tat caa atg aaa         691
   Arg Thr Ile Asn Ala Gly Glu Glu Leu Thr Phe Asp Tyr Gln Met Lys
   180                 185                 190                 195
   ggt tct gga gat ata tct tca gat tct att gac cac agc cca gcc aaa         739
   Gly Ser Gly Asp Ile Ser Ser Asp Ser Ile Asp His Ser Pro Ala Lys
                   200                 205                 210
   aag agg gtc aga aca gta tgt aaa tgt gga gct gtg act tgc aga ggt         787
   Lys Arg Val Arg Thr Val Cys Lys Cys Gly Ala Val Thr Cys Arg Gly
               215                 220                 225
   tac ctc aac tga actttttcag gaaatagagc tgatgattat aatatttttt             839
   Tyr Leu Asn
           230
   tcctaatgtt aacattttta aaaatacata tttgggactc ttattatcaa ggttctacct      899
   atgttaattt acaattcatg tttcaagaca tttgccaaat gtattaccga tgcctctgaa      959
   aagggggtca ctgggtctca tagactgata tgaagtcgac atatttatag tgcttagaga     1019
   ccaaactaat ggaaggcaga ctatttacag cttagtatat gtgtacttaa gtctatgtga     1079
   acagagaaat gcctcccgta gtgtttgaaa gcgttaagct gataatgtaa ttaacaactg     1139
   ctgagagatc aaagattcaa cttgccatac acctcaaatt cggagaaaca gttaatttgg     1199
   gcaaatctac agttctgttt ttgctactct attgtcattc ctgtttaata ctcactgtac     1259
   ttgtatttga gacaaatagg tgatactgaa ttttatactg ttttctactt ttccattaaa     1319
   acattggcac ctcaatgata aagaaattta aggtataaaa ttaaatgtaa aaattaattt     1379
   cagcttcatt tcgtatttcg aagcaatcta gactgttgtg atgagtgtat gtctgaacct     1439
   gtaattctta aaagacttct taatcttcta gaagaaaaat ctccgaagag ctctctctag 1499
   aagtccaaaa tggctagcca ttatgcttct ttgaaaggac atgataatgg gaccaggatg 1559
   gttttttgga gtaccaagca aggggaatgg agcactttaa gggcgcctgt tagtaacatg 1619
   aattggaaat ctgtgtcgag tacctctgat ctaaacggta aaacaagctg cctggagagc 1679
   agctgtacct aacaatactg taatgtacat taacattaca gcctctcaat ttcaggcagg 1739
   tgtaacagtt cctttccacc agatttaata tttttatact tcctgcaggt tcttcttaaa 1799
   aagtaatcta tatttttgaa ctgatacttg ttttatacat aaattttttt tagatgtgat 1859
   aaagctaaac ttggccaaag tgtgtgcctg aattattaga cctttttatt agtcaaccta 1919
   cgaagactaa aatagaatat attagttttc aagggagtgg gaggcttcca acatagtatt 1979
   gaatctcagg aaaaactatt ctttcatgtc tgattctgag atttctaatt gtgttgtgaa 2039
   aatgataaat gcagcaaatc tagctttcag tattcctaat ttttacctaa gctcattgct 2099
   ccaggctttg attacctaaa ataagcttgg ataaaattga accaacttca agaatgcagc 2159
   acttcttaat ctttagctct ttcttgggag aagctagact ttattcatta tattgctatg 2219
   acaacttcac tctttcataa tatataggat aaattgttta catgattgga ccctcagatt 2279
   ctgttaacca aaattgcaga atggggggcc aggcctgtgt ggtggctcac acctgtgatc 2339
   ccagcacttt gggaggctga ggtaggagga tcacgtgagg tcgggagttc aagaccagcc 2399
   tggccatcat ggtgaaaccc tgtctctact gaaaatacaa aaattagccg ggcgtggtgg 2459
   cacacgcctg tagtcccagc tactcaggag gctgaggcag gagaatcact tgaattcagg 2519
   aggcggaggt tgcagtgagc caagatcata ccactgcact gcagcctgag tgacacagta 2579
   agactgtctc caaaaaaaaa aaaaaaaaa 2608
<210> 26
   <211> 230
   <212> PRT
   <213> Homo sapiens
<400> 26
   Met Ala Ala Val Gly Ala Glu Ala Arg Gly Ala Trp Cys Val Pro Cys
   1               5                   10                  15
   Leu Val Ser Leu Asp Thr Leu Gln Glu Leu Cys Arg Lys Glu Lys Leu
               20                  25                  30
   Thr Cys Lys Ser Ile Gly Ile Thr Lys Arg Asn Leu Asn Asn Tyr Glu
           35                  40                  45
   Val Glu Tyr Leu Cys Asp Tyr Lys Val Val Lys Asp Met Glu Tyr Tyr
       50                  55                  60
   Leu Val Lys Trp Lys Gly Trp Pro Asp Ser Thr Asn Thr Trp Glu Pro
   65                  70                  75                  80
   Leu Gln Asn Leu Lys Cys Pro Leu Leu Leu Gln Gln Phe Ser Asn Asp
                   85                  90                  95
   Lys His Asn Tyr Leu Ser Gln Val Ile Thr Ser Glu Glu Ala Glu Arg
               100                 105                 110
   Arg Gly Gln Phe Tyr Asp Asn Lys Gly Ile Thr Tyr Leu Phe Asp Leu
           115                 120                 125
   Asp Tyr Glu Ser Asp Glu Phe Thr Val Asp Ala Ala Arg Tyr Gly Asn
       130                 135                 140
   Val Ser His Phe Val Asn His Ser Cys Asp Pro Asn Leu Gln Val Phe
   145                 150                 155                 160
   Asn Val Phe Ile Asp Asn Leu Asp Thr Arg Leu Pro Arg Ile Ala Leu
                   165                 170                 175
   Phe Ser Thr Arg Thr Ile Asn Ala Gly Glu Glu Leu Thr Phe Asp Tyr
               180                 185                 190
   Gln Met Lys Gly Ser Gly Asp Ile Ser Ser Asp Ser Ile Asp His Ser
           195                 200                 205
   Pro Ala Lys Lys Arg Val Arg Thr Val Cys Lys Cys Gly Ala Val Thr
       210                 215                 220
   Cys Arg Gly Tyr Leu Asn
   225                 230
<210> 27
   <211> 2566
   <212> DNA
   <213> Homo sapiens
<220>
   <221> CDS
   <222> (245)..(757)
<400> 27
   agtttgaatg aaagctctac aagatggcgg cggtcggggc cgaggcgcga ggaggtgagg      60
   ctggagcgcg gccccctcgc cttccctgtt cccagcttgg tgtgtgcctt gcctagtttc     120
   acttgatact cttcaggaat tatgtagaaa agaaaagctc acatgtaaat cgattggaat     180
   caccaaaagg aatctaaaca attatgaggt ggaatacttg tgtgactaca aggtagtaaa      240
   ggat atg gaa tat tat ctt gta aaa tgg aaa gga tgg cca gat tct aca       289
        Met Glu Tyr Tyr Leu Val Lys Trp Lys Gly Trp Pro Asp Ser Thr
        1               5                   10                  15
   aat act tgg gaa cct ttg caa aat ctg aag tgc ccg tta ctg ctt cag       337
   Asn Thr Trp Glu Pro Leu Gln Asn Leu Lys Cys Pro Leu Leu Leu Gln
                   20                  25                  30
   caa ttc tct aat gac aag cat aat tat tta tct cag gta atc aca agt       385
   Gln Phe Ser Asn Asp Lys His Asn Tyr Leu Ser Gln Val Ile Thr Ser
               35                  40                  45
   gaa gaa gct gaa aga cga gga cag ttc tat gac aac aag gga atc acg       433
   Glu Glu Ala Glu Arg Arg Gly Gln Phe Tyr Asp Asn Lys Gly Ile Thr
           50                  55                  60
   tat ctc ttt gat ctg gac tat gag tct gat gaa ttc aca gtg gat gcg       481
   Tyr Leu Phe Asp Leu Asp Tyr Glu Ser Asp Glu Phe Thr Val Asp Ala
       65                  70                  75
   gct cga tac ggc aat gtg tct cat ttt gtg aat cac agc tgt gac cca       529
   Ala Arg Tyr Gly Asn Val Ser His Phe Val Asn His Ser Cys Asp Pro
   80                  85                  90                  95
   aat ctt cag gtg ttc aat gtt ttc att gat aac ctc gat act cgt ctt       577
   Asn Leu Gln Val Phe Asn Val Phe Ile Asp Asn Leu Asp Thr Arg Leu
                   100                 105                 110
   ccc cga ata gca ttg ttt tcc aca aga acc ata aat gct gga gaa gag       625
   Pro Arg Ile Ala Leu Phe Ser Thr Arg Thr Ile Asn Ala Gly Glu Glu
               115                 120                 125
   ctg act ttt gat tat caa atg aaa ggt tct gga gat ata tct tca gat       673
   Leu Thr Phe Asp Tyr Gln Met Lys Gly Ser Gly Asp Ile Ser Ser Asp
           130                 135                 140
   tct att gac cac agc cca gcc aaa aag agg gtc aga aca gta tgt aaa       721
   Ser Ile Asp His Ser Pro Ala Lys Lys Arg Val Arg Thr Val Cys Lys
       145                 150                 155
   tgt gga gct gtg act tgc aga ggt tac ctc aac tga actttttcag            767
   Cys Gly Ala Val Thr Cys Arg Gly Tyr Leu Asn
   160                 165                 170
   gaaatagagc tgatgattat aatatttttt tcctaatgtt aacattttta aaaatacata      827
   tttgggactc ttattatcaa ggttctacct atgttaattt acaattcatg tttcaagaca      887
   tttgccaaat gtattaccga tgcctctgaa aagggggtca ctgggtctca tagactgata       947
   tgaagtcgac atatttatag tgcttagaga ccaaactaat ggaaggcaga ctatttacag     1007
   cttagtatat gtgtacttaa gtctatgtga acagagaaat gcctcccgta gtgtttgaaa     1067
   gcgttaagct gataatgtaa ttaacaactg ctgagagatc aaagattcaa cttgccatac     1127
   acctcaaatt cggagaaaca gttaatttgg gcaaatctac agttctgttt ttgctactct     1187
   attgtcattc ctgtttaata ctcactgtac ttgtatttga gacaaatagg tgatactgaa     1247
   ttttatactg ttttctactt ttccattaaa acattggcac ctcaatgata aagaaattta     1307
   aggtataaaa ttaaatgtaa aaattaattt cagcttcatt tcgtatttcg aagcaatcta 1367
   gactgttgtg atgagtgtat gtctgaacct gtaattctta aaagacttct taatcttcta 1427
   gaagaaaaat ctccgaagag ctctctctag aagtccaaaa tggctagcca ttatgcttct 1487
   ttgaaaggac atgataatgg gaccaggatg gttttttgga gtaccaagca aggggaatgg 1547
   agcactttaa gggcgcctgt tagtaacatg aattggaaat ctgtgtcgag tacctctgat 1607
   ctaaacggta aaacaagctg cctggagagc agctgtacct aacaatactg taatgtacat 1667
   taacattaca gcctctcaat ttcaggcagg tgtaacagtt cctttccacc agatttaata 1727
   tttttatact tcctgcaggt tcttcttaaa aagtaatcta tatttttgaa ctgatacttg 1787
   ttttatacat aaattttttt tagatgtgat aaagctaaac ttggccaaag tgtgtgcctg 1847
   aattattaga cctttttatt agtcaaccta cgaagactaa aatagaatat attagttttc 1907
   aagggagtgg gaggcttcca acatagtatt gaatctcagg aaaaactatt ctttcatgtc 1967
   tgattctgag atttctaatt gtgttgtgaa aatgataaat gcagcaaatc tagctttcag 2027
   tattcctaat ttttacctaa gctcattgct ccaggctttg attacctaaa ataagcttgg 2087
   ataaaattga accaacttca agaatgcagc acttcttaat ctttagctct ttcttgggag 2147
   aagctagact ttattcatta tattgctatg acaacttcac tctttcataa tatataggat 2207
   aaattgttta catgattgga ccctcagatt ctgttaacca aaattgcaga atggggggcc 2267
   aggcctgtgt ggtggctcac acctgtgatc ccagcacttt gggaggctga ggtaggagga 2327
   tcacgtgagg tcgggagttc aagaccagcc tggccatcat ggtgaaaccc tgtctctact 2387
   gaaaatacaa aaattagccg ggcgtggtgg cacacgcctg tagtcccagc tactcaggag 2447
   gctgaggcag gagaatcact tgaattcagg aggcggaggt tgcagtgagc caagatcata 2507
   ccactgcact gcagcctgag tgacacagta agactgtctc caaaaaaaaa aaaaaaaaa 2566
<210> 28
   <211> 170
   <212> PRT
   <213> Homo sapiens
<400> 28
   Met Glu Tyr Tyr Leu Val Lys Trp Lys Gly Trp Pro Asp Ser Thr Asn
   1 5 10 15
   Thr Trp Glu Pro Leu Gln Asn Leu Lys Cys Pro Leu Leu Leu Gln Gln
   20 25 30
   Phe Ser Asn Asp Lys His Asn Tyr Leu Ser Gln Val Ile Thr Ser Glu
   35 40 45
   Glu Ala Glu Arg Arg Gly Gln Phe Tyr Asp Asn Lys Gly Ile Thr Tyr
       50                  55                  60
   Leu Phe Asp Leu Asp Tyr Glu Ser Asp Glu Phe Thr Val Asp Ala Ala
   65                  70                  75                  80
   Arg Tyr Gly Asn Val Ser His Phe Val Asn His Ser Cys Asp Pro Asn
                   85                  90                  95
   Leu Gln Val Phe Asn Val Phe Ile Asp Asn Leu Asp Thr Arg Leu Pro
               100                 105                 110
   Arg Ile Ala Leu Phe Ser Thr Arg Thr Ile Asn Ala Gly Glu Glu Leu
           115                 120                 125
   Thr Phe Asp Tyr Gln Met Lys Gly Ser Gly Asp Ile Ser Ser Asp Ser
       130                 135                 140
   Ile Asp His Ser Pro Ala Lys Lys Arg Val Arg Thr Val Cys Lys Cys
   145                 150                 155                 160
   Gly Ala Val Thr Cys Arg Gly Tyr Leu Asn
                   165                 170
<210> 29
   <211> 3093
   <212> DNA
   <213> Homo sapiens
<220>
   <221> CDS
   <222> (232)..(1284)
<400> 29
   cggggccgag gcgcgaggag gtgaggctgg agcgcggccc cctcgccttc cctgttccca      60
   ggcaagctcc caaggcccgg gcggcggggc cgtcccgcgg gccagccaga tggcgacgtg     120
   gcggttcccc gcccgccgcg accccaactc cgggacgcac gctgcggacg cctatcctcc     180
   cccaggccgc tgacccgcct ccctgcccgg ccggctcccg ccgcggagga t atg gaa      237
                                                           Met Glu
                                                           1
   tat tat ctt gta aaa tgg aaa gga tgg cca gat tct aca aat act tgg       285
   Tyr Tyr Leu Val Lys Trp Lys Gly Trp Pro Asp Ser Thr Asn Thr Trp
           5                   10                  15
   gaa cct ttg caa aat ctg aag tgc ccg tta ctg ctt cag caa ttc tct       333
   Glu Pro Leu Gln Asn Leu Lys Cys Pro Leu Leu Leu Gln Gln Phe Ser
       20                  25                  30
   aat gac aag cat aat tat tta tct cag gta aag aaa ggc aaa gca ata       381
   Asn Asp Lys His Asn Tyr Leu Ser Gln Val Lys Lys Gly Lys Ala Ile
   35                  40                  45                  50
   act cca aaa gac aat aac aaa act ttg aaa cct gcc att gct gag tac          429
   Thr Pro Lys Asp Asn Asn Lys Thr Leu Lys Pro Ala Ile Ala Glu Tyr
                   55                  60                  65
   att gtg aag aag gct aaa caa agg ata gct ctg cag aga tgg caa gat          477
   Ile Val Lys Lys Ala Lys Gln Arg Ile Ala Leu Gln Arg Trp Gln Asp
               70                  75                  80
   gaa ctc aac aga aga aag aat cat aaa gga atg ata ttt gtt gaa aat          525
   Glu Leu Asn Arg Arg Lys Asn His Lys Gly Met Ile Phe Val Glu Asn
           85                  90                  95
   act gtt gat tta gag ggc cca cct tca gac ttc tat tac att aac gaa          573
   Thr Val Asp Leu Glu Gly Pro Pro Ser Asp Phe Tyr Tyr Ile Asn Glu
       100                 105                 110
   tac aaa cca gct cct gga atc agc tta gtc aat gaa gct acc ttt ggt          621
   Tyr Lys Pro Ala Pro Gly Ile Ser Leu Val Asn Glu Ala Thr Phe Gly
   115                 120                 125                 130
   tgt tca tgc aca gat tgc ttc ttt caa aaa tgt tgt cct gct gaa gct          669
   Cys Ser Cys Thr Asp Cys Phe Phe Gln Lys Cys Cys Pro Ala Glu Ala
                   135                 140                 145
   gga gtt ctt ttg gct tat aat aaa aac caa caa att aaa atc cca cct          717
   Gly Val Leu Leu Ala Tyr Asn Lys Asn Gln Gln Ile Lys Ile Pro Pro
               150                 155                 160
   ggt act ccc atc tat gaa tgc aac tca agg tgt cag tgt ggt cct gat          765
   Gly Thr Pro Ile Tyr Glu Cys Asn Ser Arg Cys Gln Cys Gly Pro Asp
           165                 170                 175
   tgt ccc aat agg att gta caa aaa ggc aca cag tat tcg ctt tgc atc          813
   Cys Pro Asn Arg Ile Val Gln Lys Gly Thr Gln Tyr Ser Leu Cys Ile
       180                 185                 190
   ttt cga act agc aat gga cgt ggc tgg ggt gta aag acc ctt gtg aag          861
   Phe Arg Thr Ser Asn Gly Arg Gly Trp Gly Val Lys Thr Leu Val Lys
   195                 200                 205                 210
   att aaa aga atg agt ttt gtc atg gaa tat gtt gga gag gta atc aca          909
   Ile Lys Arg Met Ser Phe Val Met Glu Tyr Val Gly Glu Val Ile Thr
                   215                 220                 225
   agt gaa gaa gct gaa aga cga gga cag ttc tat gac aac aag gga atc          957
   Ser Glu Glu Ala Glu Arg Arg Gly Gln Phe Tyr Asp Asn Lys Gly Ile
               230                 235                 240
   acg tat ctc ttt gat ctg gac tat gag tct gat gaa ttc aca gtg gat         1005
   Thr Tyr Leu Phe Asp Leu Asp Tyr Glu Ser Asp Glu Phe Thr Val Asp
           245                 250                 255
   gcg gct cga tac ggc aat gtg tct cat ttt gtg aat cac agc tgt gac         1053
   Ala Ala Arg Tyr Gly Asn Val Ser His Phe Val Asn His Ser Cys Asp
       260                 265                 270
   cca aat ctt cag gtg ttc aat gtt ttc att gat aac ctc gat act cgt         1101
   Pro Asn Leu Gln Val Phe Asn Val Phe Ile Asp Asn Leu Asp Thr Arg
   275                 280                 285                 290
   ctt ccc cga ata gca ttg ttt tcc aca aga acc ata aat gct gga gaa         1149
   Leu Pro Arg Ile Ala Leu Phe Ser Thr Arg Thr Ile Asn Ala Gly Glu
                   295                 300                 305
   gag ctg act ttt gat tat caa atg aaa ggt tct gga gat ata tct tca        1197
   Glu Leu Thr Phe Asp Tyr Gln Met Lys Gly Ser Gly Asp Ile Ser Ser
               310                 315                 320
   gat tct att gac cac agc cca gcc aaa aag agg gtc aga aca gta tgt        1245
   Asp Ser Ile Asp His Ser Pro Ala Lys Lys Arg Val Arg Thr Val Cys
           325                 330                 335
   aaa tgt gga gct gtg act tgc aga ggt tac ctc aac tga actttttcag         1294
   Lys Cys Gly Ala Val Thr Cys Arg Gly Tyr Leu Asn
       340                 345                 350
   gaaatagagc tgatgattat aatatttttt tcctaatgtt aacattttta aaaatacata      1354
   tttgggactc ttattatcaa ggttctacct atgttaattt acaattcatg tttcaagaca      1414
   tttgccaaat gtattaccga tgcctctgaa aagggggtca ctgggtctca tagactgata      1474
   tgaagtcgac atatttatag tgcttagaga ccaaactaat ggaaggcaga ctatttacag      1534
   cttagtatat gtgtacttaa gtctatgtga acagagaaat gcctcccgta gtgtttgaaa      1594
   gcgttaagct gataatgtaa ttaacaactg ctgagagatc aaagattcaa cttgccatac      1654
   acctcaaatt cggagaaaca gttaatttgg gcaaatctac agttctgttt ttgctactct      1714
   attgtcattc ctgtttaata ctcactgtac ttgtatttga gacaaatagg tgatactgaa      1774
   ttttatactg ttttctactt ttccattaaa acattggcac ctcaatgata aagaaattta      1834
   aggtataaaa ttaaatgtaa aaattaattt cagcttcatt tcgtatttcg aagcaatcta      1894
   gactgttgtg atgagtgtat gtctgaacct gtaattctta aaagacttct taatcttcta      1954
   gaagaaaaat ctccgaagag ctctctctag aagtccaaaa tggctagcca ttatgcttct      2014
   ttgaaaggac atgataatgg gaccaggatg gtttttggga gtaccaagca aggggaatgg    2074
   agcactttaa gggcgcctgt tagtaacatg aattggaaat ctgtgtcgag tacctctgat      2134
   ctaaacggta aaacaagctg cctggagagc agctgtacct aacaatactg taatgtacat      2194
   taacattaca gcctctcaat ttcaggcagg tgtaacagtt cctttccacc agatttaata      2254
   tttttatact tcctgcaggt tcttcttaaa aagtaatcta tatttttgaa ctgatacttg      2314
   ttttatacat aaattttttt tagatgtgat aaagctaaac ttggccaaag tgtgtgcctg      2374
   aattattaga cctttttatt agtcaaccta cgaagactaa aatagaatat attagttttc      2434
   aagggagtgg gaggcttcca acatagtatt gaatctcagg aaaaactatt ctttcatgtc      2494
   tgattctgag atttctaatt gtgttgtgaa aatgataaat gcagcaaatc tagctttcag      2554
   tattcctaat ttttacctaa gctcattgct ccaggctttg attacctaaa ataagcttgg      2614
   ataaaattga accaacttca agaatgcagc acttcttaat ctttagctct ttcttgggag      2674
   aagctagact ttattcatta tattgctatg acaacttcac tctttcataa tatataggat      2734
   aaattgttta catgattgga ccctcagatt ctgttaacca aaattgcaga atggggggcc      2794
   aggcctgtgt ggtggctcac acctgtgatc ccagcacttt gggaggctga ggtaggagga      2854
   tcacgtgagg tcgggagttc aagaccagcc tggccatcat ggtgaaaccc tgtctctact      2914
   gaaaatacaa aaattagccg ggcgtggtgg cacacgcctg tagtcccagc tactcaggag      2974
   gctgaggcag gagaatcact tgaattcagg aggcggaggt tgcagtgagc caagatcata      3034
   ccactgcact gcagcctgag tgacacagta agactgtctc caaaaaaaaa aaaaaaaaa       3093
<210> 30
   <211> 350
   <212> PRT
   <213> Homo sapiens
<400> 30
   Met Glu Tyr Tyr Leu Val Lys Trp Lys Gly Trp Pro Asp Ser Thr Asn
   1               5                   10                  15
   Thr Trp Glu Pro Leu Gln Asn Leu Lys Cys Pro Leu Leu Leu Gln Gln
               20                  25                  30
   Phe Ser Asn Asp Lys His Asn Tyr Leu Ser Gln Val Lys Lys Gly Lys
           35                  40                  45
   Ala Ile Thr Pro Lys Asp Asn Asn Lys Thr Leu Lys Pro Ala Ile Ala
       50                  55                  60
   Glu Tyr Ile Val Lys Lys Ala Lys Gln Arg Ile Ala Leu Gln Arg Trp
   65                  70                  75                  80
   Gln Asp Glu Leu Asn Arg Arg Lys Asn His Lys Gly Met Ile Phe Val
                   85                  90                  95
   Glu Asn Thr Val Asp Leu Glu Gly Pro Pro Ser Asp Phe Tyr Tyr Ile
               100                 105                 110
   Asn Glu Tyr Lys Pro Ala Pro Gly Ile Ser Leu Val Asn Glu Ala Thr
           115                 120                 125
   Phe Gly Cys Ser Cys Thr Asp Cys Phe Phe Gln Lys Cys Cys Pro Ala
       130                 135                 140
   Glu Ala Gly Val Leu Leu Ala Tyr Asn Lys Asn Gln Gln Ile Lys Ile
   145                 150                 155                 160
   Pro Pro Gly Thr Pro Ile Tyr Glu Cys Asn Ser Arg Cys Gln Cys Gly
                   165                 170                 175
   Pro Asp Cys Pro Asn Arg Ile Val Gln Lys Gly Thr Gln Tyr Ser Leu
               180                 185                 190
   Cys Ile Phe Arg Thr Ser Asn Gly Arg Gly Trp Gly Val Lys Thr Leu
           195                 200                 205
   Val Lys Ile Lys Arg Met Ser Phe Val Met Glu Tyr Val Gly Glu Val
       210                 215                 220
   Ile Thr Ser Glu Glu Ala Glu Arg Arg Gly Gln Phe Tyr Asp Asn Lys
   225                 230                 235                 240
   Gly Ile Thr Tyr Leu Phe Asp Leu Asp Tyr Glu Ser Asp Glu Phe Thr
                   245                 250                 255
   Val Asp Ala Ala Arg Tyr Gly Asn Val Ser His Phe Val Asn His Ser
               260                 265                 270
   Cys Asp Pro Asn Leu Gln Val Phe Asn Val Phe Ile Asp Asn Leu Asp
           275                 280                 285
   Thr Arg Leu Pro Arg Ile Ala Leu Phe Ser Thr Arg Thr Ile Asn Ala
       290                 295                 300
   Gly Glu Glu Leu Thr Phe Asp Tyr Gln Met Lys Gly Ser Gly Asp Ile
   305                 310                 315                 320
   Ser Ser Asp Ser Ile Asp His Ser Pro Ala Lys Lys Arg Val Arg Thr
                   325                 330                 335
   Val Cys Lys Cys Gly Ala Val Thr Cys Arg Gly Tyr Leu Asn
               340                 345                 350
<210> 31
   <211> 21
   <212> PRT
   <213> Artificial Sequence
<220>
   <223> An artificially synthesized peptide
<400> 31
   Ala Arg Thr Lys Gln Thr Ala Arg Lys Ser Thr Gly Gly Lys Ala Pro
   1               5                   10                  15
   Arg Lys Gln Leu Ala
               20
<210> 32
   <211> 312
   <212> PRT
   <213> Homo sapiens
<400> 32
   Asn Tyr Leu Ser Gln Val Lys Lys Gly Lys Ala Ile Thr Pro Lys Asp
   1               5                   10                  15
   Asn Asn Lys Thr Leu Lys Pro Ala Ile Ala Glu Tyr Ile Val Lys Lys
               20                  25                  30
   Ala Lys Gln Arg Ile Ala Leu Gln Arg Trp Gln Asp Glu Leu Asn Arg
           35                  40                  45
   Arg Lys Asn His Lys Gly Met Ile Phe Val Glu Asn Thr Val Asp Leu
       50                  55                  60
   Glu Gly Pro Pro Ser Asp Phe Tyr Tyr Ile Asn Glu Tyr Lys Pro Ala
   65                  70                  75                  80
   Pro Gly Ile Ser Leu Val Asn Glu Ala Thr Phe Gly Cys Ser Cys Thr
                   85                  90                  95
   Asp Cys Phe Phe Gln Lys Cys Cys Pro Ala Glu Ala Gly Val Leu Leu
               100                 105                 110
   Ala Tyr Asn Lys Asn Gln Gln Ile Lys Ile Pro Pro Gly Thr Pro Ile
           115                 120                 125
   Tyr Glu Cys Asn Ser Arg Cys Gln Cys Gly Pro Asp Cys Pro Asn Arg
       130                 135                 140
   Ile Val Gln Lys Gly Thr Gln Tyr Ser Leu Cys Ile Phe Arg Thr Ser
   145                 150                 155                 160
   Asn Gly Arg Gly Trp Gly Val Lys Thr Leu Val Lys Ile Lys Arg Met
                   165                 170                 175
   Ser Phe Val Met Glu Tyr Val Gly Glu Val Ile Thr Ser Glu Glu Ala
               180                 185                 190
   Glu Arg Arg Gly Gln Phe Tyr Asp Asn Lys Gly Ile Thr Tyr Leu Phe
           195                 200                 205
   Asp Leu Asp Tyr Glu Ser Asp Glu Phe Thr Val Asp Ala Ala Arg Tyr
       210                 215                 220
   Gly Asn Val Ser His Phe Val Asn His Ser Cys Asp Pro Asn Leu Gln
   225                 230                 235                 240
   Val Phe Asn Val Phe Ile Asp Asn Leu Asp Thr Arg Leu Pro Arg Ile
                   245                 250                 255
   Ala Leu Phe Ser Thr Arg Thr Ile Asn Ala Gly Glu Glu Leu Thr Phe
               260                 265                 270
   Asp Tyr Gln Met Lys Gly Ser Gly Asp Ile Ser Ser Asp Ser Ile Asp
           275                 280                 285
   His Ser Pro Ala Lys Lys Arg Val Arg Thr Val Cys Lys Cys Gly Ala
       290                 295                 300
   Val Thr Cys Arg Gly Tyr Leu Asn
   305                 310
<210> 33
   <211> 410
   <212> PRT
   <213> Artificial Sequence
<220>
   <223> GST-fusion SUV39H2
<400> 33
   Met Ala Ala Val Gly Ala Glu Ala Arg Gly Ala Trp Cys Val Pro Cys
   1               5                   10                  15
   Leu Val Ser Leu Asp Thr Leu Gln Glu Leu Cys Arg Lys Glu Lys Leu
               20                  25                  30
   Thr Cys Lys Ser Ile Gly Ile Thr Lys Arg Asn Leu Asn Asn Tyr Glu
           35                  40                  45
   Val Glu Tyr Leu Cys Asp Tyr Lys Val Val Lys Asp Met Glu Tyr Tyr
       50                  55                  60
   Leu Val Lys Trp Lys Gly Trp Pro Asp Ser Thr Asn Thr Trp Glu Pro
   65                  70                  75                  80
   Leu Gln Asn Leu Lys Cys Pro Leu Leu Leu Gln Gln Phe Ser Asn Asp
                   85                  90                  95
   Lys His Asn Tyr Leu Ser Gln Val Lys Lys Gly Lys Ala Ile Thr Pro
               100                 105                 110
   Lys Asp Asn Asn Lys Thr Leu Lys Pro Ala Ile Ala Glu Tyr Ile Val
   115 120 125
   Lys Lys Ala Lys Gln Arg Ile Ala Leu Gln Arg Trp Gln Asp Glu Leu
   130 135 140
   Asn Arg Arg Lys Asn His Lys Gly Met Ile Phe Val Glu Asn Thr Val
   145 150 155 160
   Asp Leu Glu Gly Pro Pro Ser Asp Phe Tyr Tyr Ile Asn Glu Tyr Lys
   165 170 175
   Pro Ala Pro Gly Ile Ser Leu Val Asn Glu Ala Thr Phe Gly Cys Ser
   180 185 190
   Cys Thr Asp Cys Phe Phe Gln Lys Cys Cys Pro Ala Glu Ala Gly Val
   195 200 205
   Leu Leu Ala Tyr Asn Lys Asn Gln Gln Ile Lys Ile Pro Pro Gly Thr
   210 215 220
   Pro Ile Tyr Glu Cys Asn Ser Arg Cys Gln Cys Gly Pro Asp Cys Pro
   225 230 235 240
   Asn Arg Ile Val Gln Lys Gly Thr Gln Tyr Ser Leu Cys Ile Phe Arg
   245 250 255
   Thr Ser Asn Gly Arg Gly Trp Gly Val Lys Thr Leu Val Lys Ile Lys
   260 265 270
   Arg Met Ser Phe Val Met Glu Tyr Val Gly Glu Val Ile Thr Ser Glu
   275 280 285
   Glu Ala Glu Arg Arg Gly Gln Phe Tyr Asp Asn Lys Gly Ile Thr Tyr
   290 295 300
   Leu Phe Asp Leu Asp Tyr Glu Ser Asp Glu Phe Thr Val Asp Ala Ala
   305 310 315 320
   Arg Tyr Gly Asn Val Ser His Phe Val Asn His Ser Cys Asp Pro Asn
   325 330 335
   Leu Gln Val Phe Asn Val Phe Ile Asp Asn Leu Asp Thr Arg Leu Pro
   340 345 350
   Arg Ile Ala Leu Phe Ser Thr Arg Thr Ile Asn Ala Gly Glu Glu Leu
   355 360 365
   Thr Phe Asp Tyr Gln Met Lys Gly Ser Gly Asp Ile Ser Ser Asp Ser
       370                 375                 380
   Ile Asp His Ser Pro Ala Lys Lys Arg Val Arg Thr Val Cys Lys Cys
   385                 390                 395                 400
   Gly Ala Val Thr Cys Arg Gly Tyr Leu Asn
                   405                 410